# EUROPEAN PATENT SPECIFICATION

(11) **EP 3 134 390 B1**
(45) Date of publication and mention of the grant of the patent: **26.02.2020**
(21) Application number: 15782804.7
(22) Date of filing: 21.04.2015
(51) Int. Cl.: C07D 249/12, C07D 403/10, A61K 31/4196, A61P 35/00

(54) **SMALL MOLECULE INHIBITORS OF G PROTEIN COUPLED RECEPTOR 6 KINASE POLYPEPTIDES**
KLEINMOLEKULARE HEMMER VON G-PROTEIN-GEKOPPELTEN REZEPTOR-6-KINASEPOLYPEPTIDEN
INHIBITEURS À PETITES MOLÉCULES DE POLYPEPTIDES DE RÉCEPTEURS KINASES 6 COUPLÉS AUX PROTÉINES G

(30) Priority: 21.04.2014 US 201461982141 P; 22.07.2014 US 201462027633 P
(43) Date of publication of application: 01.03.2017
(73) Proprietor: Mayo Foundation for Medical Education and Research, Rochester, MN 55905 (US)
(72) Inventor: STEWART, Alexander Keith, Scottsdale, Arizona 85259 (US); RICH, Brian, San Jose, California 95112 (US); THOMAS, William, San Jose, California 95124 (US); SEPETOV, Nikolai, Los Gatos, California 95033 (US); GREENHOUSE, Robert, Newark, California 94560 (US)
(74) Representative: Kunz, Herbert
(86) International application number: PCT/US2015/026931
(87) International publication number: WO 2015/164415

(56) References cited:
- WO-A2-2005/092873
- WO-A2-2013/063458
- WO-A2-2013/063458

## Description

### CROSS REFERENCE TO RELATED APPLICATIONS

This application claims the benefit of U.S. Provisional Application Serial Nos. 61/982,141, filed April 21, 2014, and 62/027,633, filed July 22, 2014.

### TECHNICAL FIELD

This document relates to inhibitors of G protein coupled receptor 6 kinase (GRK6) polypeptides as well as methods and materials for using such inhibitors to treat hematological malignancies, inflammation diseases, and autoimmune disorders.

### BACKGROUND

GRK6 is a member of the enzyme group of kinases. Kinases regulate many different cell proliferation, differentiation, and signaling processes by adding phosphate groups to proteins. The kinases comprise the largest known protein group, a superfamily of enzymes with widely varied functions and specificities. They are usually named after their substrate, their regulatory molecules, or some aspect of a mutant phenotype. With regard to substrates, the protein kinases may be roughly divided into two groups; those that phosphorylate tyrosine residues (protein tyrosine kinases, PTK) and those that phosphorylate serine or threonine residues (serine/threonine kinases, STK). A few protein kinases have dual specificity and phosphorylate threonine and tyrosine residues. Almost all kinases contain a similar 250-300 amino acid catalytic domain. The N-terminal domain, which contains subdomains I-IV, generally folds into a two-lobed structure, which binds and orients the ATP (or GTP) donor molecule. The larger C terminal lobe, which contains subdomains VI-XI, binds the protein substrate and carries out the transfer of the gamma phosphate from ATP to the hydroxyl group of a serine, threonine, or tyrosine residue. Subdomain V spans the two lobes.

WO 2013/063458 A2 describes inhibitors of GRK6 polypeptides as well as methods and materials for using such inhibitors to treat hematological malignancies, inflammation diseases, and autoimmune disorders.

### SUMMARY

The present invention is defined by the independent claims. The dependent claims depict additional embodiments of the invention.

Provided herein are compounds of Formula I: 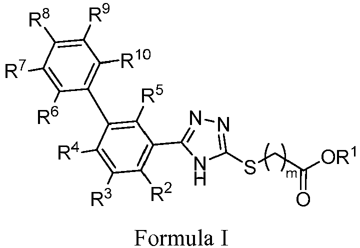 or a pharmaceutically acceptable salt thereof,
wherein:
R¹ is selected from the group consisting of H and (C₁-C₆)alkyl;
each of R², R⁴, and R⁵ are independently selected from the group consisting of: H, halo, substituted or unsubstituted (C₁-C₆)alkyl, (C₁-C₆)haloalkyl, OR^{A}, CN, NO₂, C(O)R^{A}, NR^{A}R^{B}, -S(O)₂R^{A}, -S(O)₂NR^{A}R^{B}, -S(O)NR^{A}R^{B}, -NR^{A}S(O)₂R^{B}, - C(NR^{A})R^{B}, -C(O)NR^{A}R^{B}, a substituted or unsubstituted aryl, a substituted or unsubstituted heteroaryl, a substituted or unsubstituted cycloalkyl, and a substituted or unsubstituted heterocycloalkyl;
R³ is NO₂;
each of R⁶, R⁷, R⁸, R⁹, and R¹⁰ are independently selected from the group consisting of: H, substituted or unsubstituted (C₁-C₆)alkyl, (C₁-C₆)haloalkyl, OR^{A}, NO₂, C(O)R^{A}, NR^{A}R^{B}, -S(O)₂NR^{A}R^{B}, -S(O)NR^{A}R^{B}, -C(NR^{A})R^{B}, -C(O)NR^{A}R^{B}, - NR^{A}S(O)₂R^{B}, -NR^{A}C(O)R^{B}, -S(O)₂R^{A}, a substituted or unsubstituted aryl, a substituted or unsubstituted heteroaryl, a substituted or unsubstituted cycloalkyl, and a substituted or unsubstituted heterocycloalkyl; or
R⁶ and R⁷, R⁷ and R⁸, R⁸ and R⁹, or R⁹ and R¹⁰ can come together to form a substituted or unsubstituted fused cycloalkyl ring, a substituted or unsubstituted fused heterocycloalkyl ring, or a substituted or unsubstituted fused heteroaryl ring;
each R^{A} and R^{B} is independently selected from the group consisting of H and (Ci-C₆)alkyl; and
m is an integer from 1 to 2.

In some embodiments, R¹ is H. In some such embodiments, R², R⁴, and R⁵ are independently selected from the group consisting of: H, NO₂, and a substituted or unsubstituted (C₁-C₆)alkyl. In some such embodiments, one of R⁶ and R⁷, R⁷ and R⁸, R⁸ and R⁹, or R⁹ and R¹⁰ come together to form a substituted or unsubstituted fused cycloalkyl ring. For example, one of R⁶ and R⁷, R⁷ and R⁸, R⁸ and R⁹, or R⁹ and R¹⁰ come together to form a substituted or unsubstituted fused heterocycloalkyl ring. In other such embodiments, one of R⁶ and R⁷, R⁷ and R⁸, R⁸ and R⁹, or R⁹ and R¹⁰ come together to form a substituted or unsubstituted fused heteroaryl ring. In further embodiments, each of R⁶, R⁷, R⁸, R⁹, and R¹⁰ are independently selected from the group consisting of: H, substituted or unsubstituted (C₁-C₆)alkyl, OR^{A}, C(O)R^{A}, NR^{A}R^{B}, -S(O)₂NR^{A}R^{B}, -S(O)NR^{A}R^{B}, -C(NR^{A})R^{B}, -C(O)NR^{A}R^{B}, -NR^{A}S(O)₂R^{B}, - NR^{A}C(O)R^{B}, -S(O)₂R^{A}, and a substituted or unsubstituted heteroaryl.

In some embodiments, m is 1.

Non-limiting examples of a compound of Formula I includes: 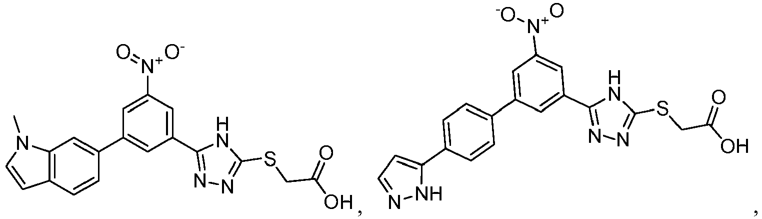 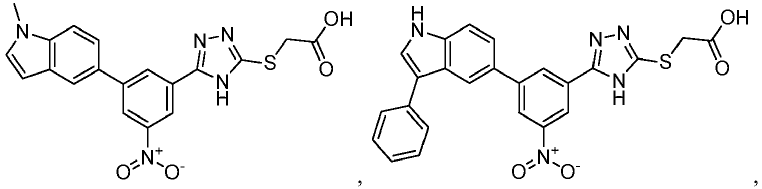 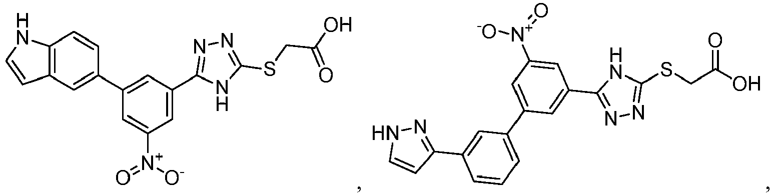 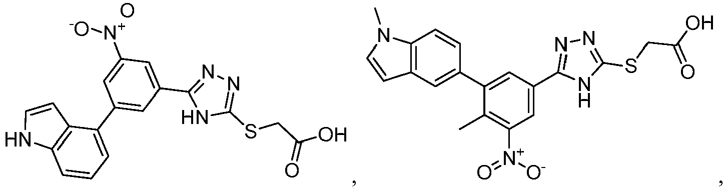 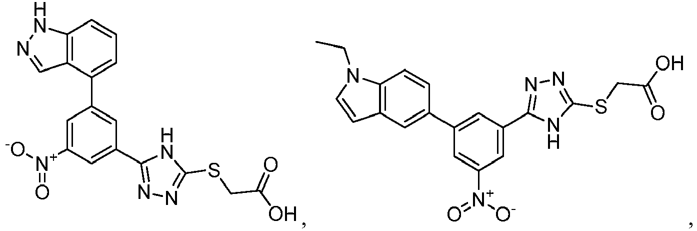 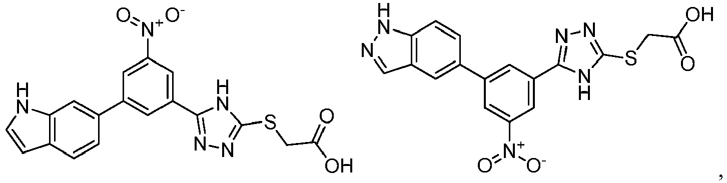 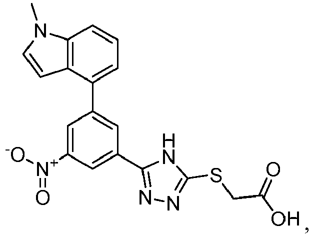 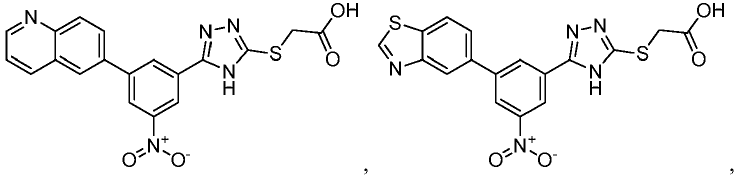 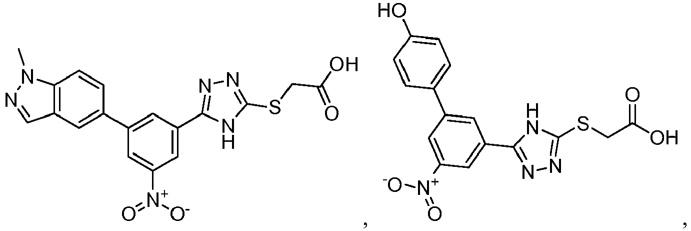 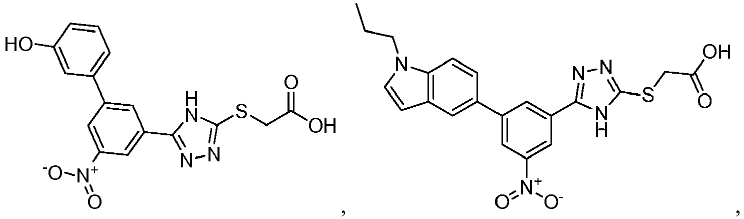 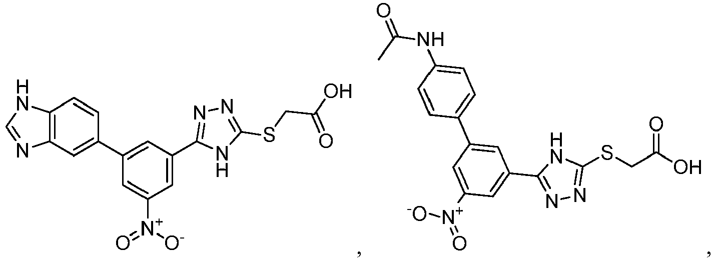 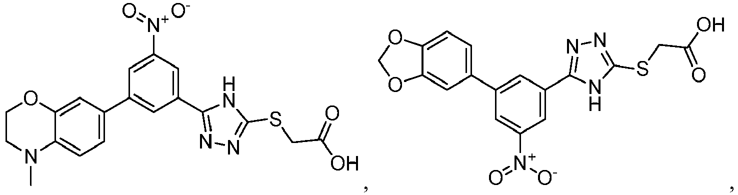 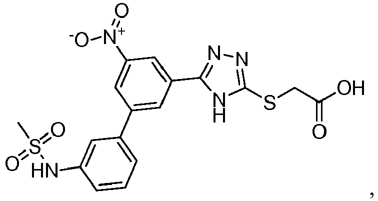 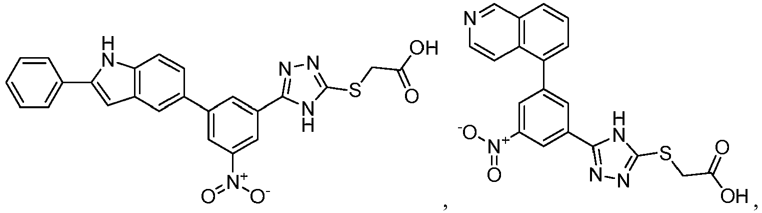 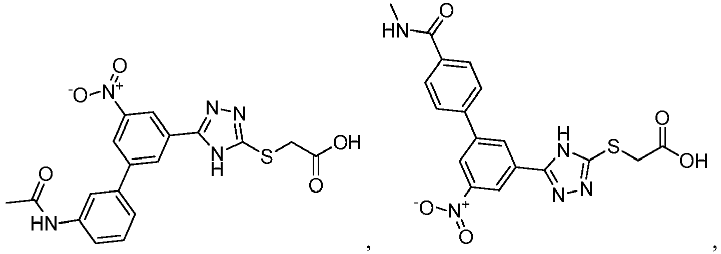 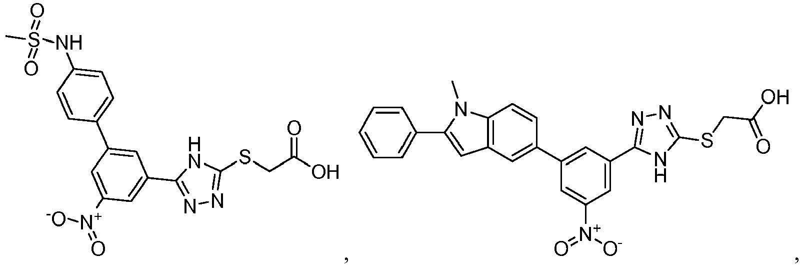 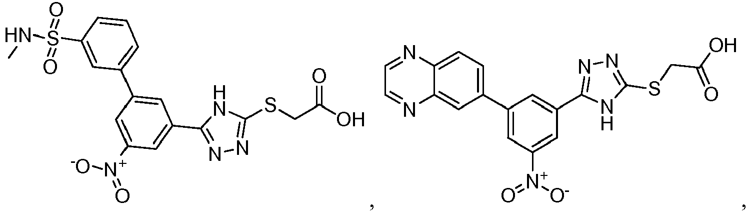 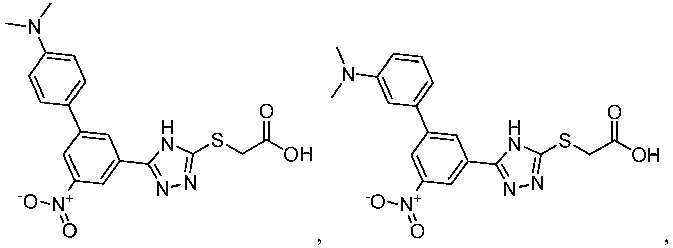 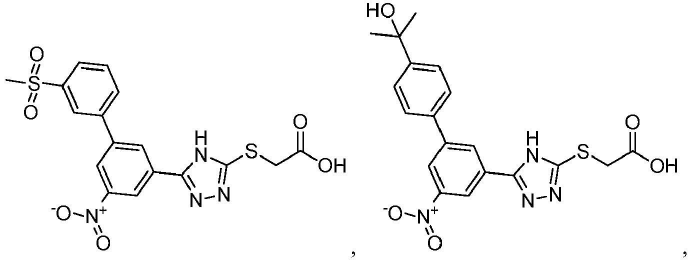 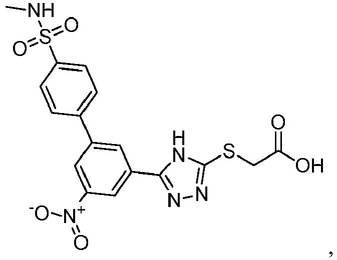 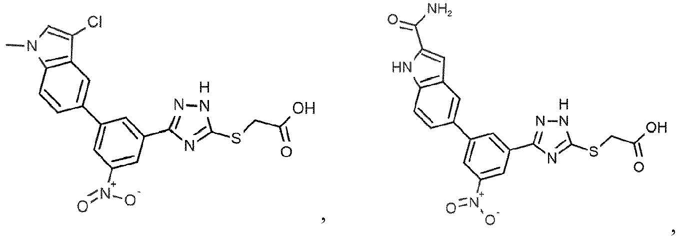 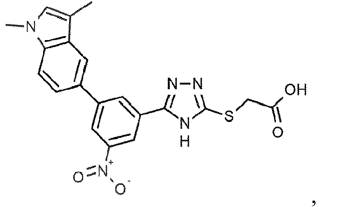 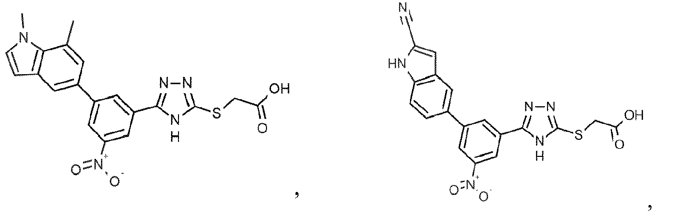 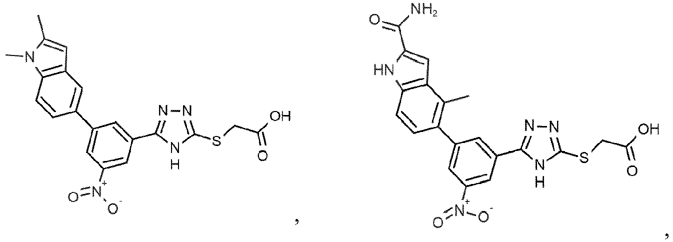 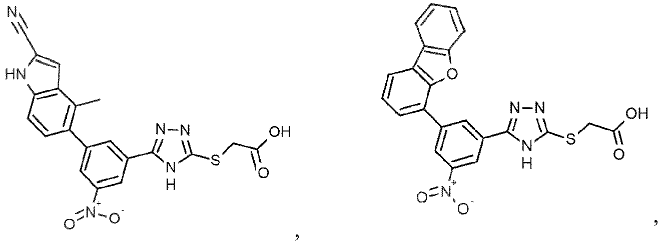 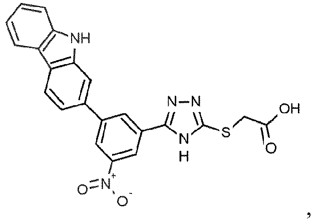 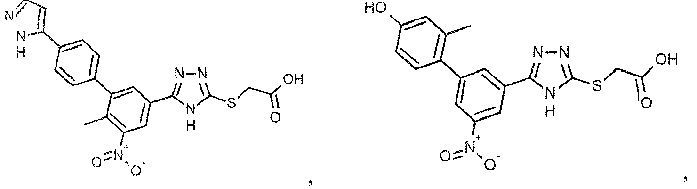 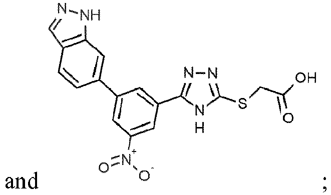 or a pharmaceutically acceptable salt thereof.

The compounds provided herein can also be present in a pharmaceutical composition comprising a compound provided herein, or a pharmaceutically acceptable salt thereof, and a pharmaceutically acceptable carrier.

The compounds provided herein are also useful in in vitro methods for inhibiting a G protein coupled receptor 6 kinase polypeptide in a patient. In some embodiments, the method can include administering to the patient a therapeutically effective amount of a compound provided herein, or a pharmaceutically acceptable salt thereof. The compounds provided herein are also useful for inhibiting a G protein coupled receptor 6 kinase polypeptide in a cell. In some embodiments, such methods include contacting the cell with an effective amount of a compound provided herein, or a pharmaceutically acceptable salt thereof. In some such embodiments, the cell is a cancerous cell. For example, a B cell cancerous cell.

Also provided herein are compositions for use in methods for treating a hematological malignancy in a patient, the method comprising administering to the patient a therapeutically effective amount of a compound provided herein, or a pharmaceutically acceptable salt thereof. In some embodiments, the hematological malignancy is a B cell cancer. For example, the B cell cancer can be selected from the group consisting of: a small lymphocytic lymphoma (SLL), a mantle cell lymphoma, a Burkitt's lymphoma, a follicle centre cell lymphoma, a follicular lymphoma, a Burkitt-like lymphoma, a marginal zone B-cell lymphoma (MZBCL), a nodal marginal zone B cell lymphoma, an extra-nodal marginal zone B cell lymphoma, a splenic marginal zone B cell lymphoma, a lymphoplasmacytic lymphoma, and a diffuse large B cell lymphoma. In some embodiments, the B cell cancer is selected from the group consisting of: a B cell acute lymphocytic leukemia (B-ALL), a precursor B cell acute lymphocytic leukemia (B-ALL), a B cell chronic lymphocytic leukemia (B-CLL), a precursor B-lymphoblastic leukaemia, a precursor B-lymphoblastic lymphoma, a small lymphocytic lymphoma, a B cell prolymphocytic leukemia, an undifferentiated B cell lymphoma, a hairy cell leukemia, a mediastinal large B-cell lymphoma, a plasma cell myeloma, a plasmacytoma, a primary effusive lymphoma, a Burkitt's cell leukemia, and a B cell diffuse mixed lymphoma.

Further provided herein are compositions for use in methods for treating an inflammation disease in a patient, the method comprising administering to the patient a therapeutically effective amount of a compound provided herein, or a pharmaceutically acceptable salt thereof. In some embodiments, the inflammatory disease can be selected from the group consisting of: encephalitis, inflammatory eye disease, otitis, pharyngitis, pneumonia, gastritis, enteritis, hepatitis, pancreatitis, nephritis, cystitis, urethritis, endometritis, vaginitis, arthritis, peripheral neuritis, malignant tumor, infectious diseases, autoimmune diseases, ischemic diseases, metabolic diseases, injury, scald, chemical corrosion, and neurodegenerative diseases.

In some embodiments, an autoimmune disease can be selected from the group consisting of: rheumatism, systemic lupus erythematosus, and sarcoidosis.

In some embodiments, an ischemic disease can be selected from the group consisting of: myocardial infarction and cerebral infarction.

In some embodiments, a metabolic disease can be selected from the group consisting of: diabetes and gout.

In some embodiments, a neurodegenerative can be Alzheimer's disease.

The compounds provided herein are also useful for suppressing an immune response in a patient, the method comprising administering to the patient a therapeutically effective amount of a compound provided herein, or a pharmaceutically acceptable salt thereof.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. In case of conflict between references mentioned herein and the present specification, the present specification, including definitions, will control.

Other features and advantages of the invention will be apparent from the following detailed description and figures, and from the claims.

### DETAILED DESCRIPTION

### Definitions

For the terms "for example" and "such as," and grammatical equivalences thereof, the phrase "and without limitation" is understood to follow unless explicitly stated otherwise. All measurements reported herein are understood to be modified by the term "about", whether or not the term is explicitly used, unless explicitly stated otherwise. As used herein, the singular forms "a," "an," and "the" include plural referents unless the context clearly dictates otherwise.

A "patient," as used herein, includes both humans and other animals, particularly mammals. Thus, the methods are applicable to both human therapy and veterinary applications. In some embodiments, the patient is a mammal, for example, a primate. In some embodiments, the patient is a human.

The terms "treating" and "treatment" mean causing a therapeutically beneficial effect, such as ameliorating one or more existing symptoms and/or reducing the severity of symptoms that will or are expected to develop.

A "therapeutically effective" amount of the inhibitors described herein is typically one which is sufficient to achieve the desired effect and may vary according to the nature and severity of the disease condition, and the potency of the inhibitor. It will be appreciated that different concentrations may be employed for prophylaxis than for treatment of an active disease.

The term "contacting" means bringing at least two moieties together, whether in an *in vitro* system or an *in vivo* system.

The term "inhibition" with respect to a GRK6 polypeptide refers to inhibition of a GRK6 polypeptide and its biological activities associated with a GRK6 polypeptide pathway. Inhibition of GRK6 polypeptide can include antagonizing or inactivation. The mode of action of a GRK6 polypeptide inhibitor can be direct, e.g., through binding to a GRK6 polypeptide as a ligand. The mode of action of an inhibitor can be indirect, e.g., through binding to and/or modifying another molecule that otherwise binds to and activates a GRK6 polypeptide.

As used herein, "administration" refers to delivery of an inhibitor or composition comprising an inhibitor provided herein by any external route, including, without limitation, IV, intramuscular, SC, intranasal, inhalation, transdermal, oral, buccal, rectal, sublingual, and parenteral administration.

The term "cancerous B cell" is used herein to refer to a B cell that is cancerous. By "cancerous cell" or "cancer cell" is meant a cell that shows aberrant cell growth, such as increased cell growth. A cancerous cell may be a hyperplastic cell, a cell that shows a lack of contact inhibition of growth *in vitro,* a tumor cell that is incapable of metastasis *in vivo,* or a metastatic cell that is capable of metastasis *in vivo.*

An inhibitor provided herein can also incorporate one or more isotopes of the atoms occurring in the inhibitor. Isotopes include, for example, those atoms having the same atomic number but different mass numbers. For example, carbon atoms can include carbon-12, carbon-13, and/or carbon-14 and hydrogen atoms can include hydrogen, deuterium, and/or tritium.

The term, "inhibitor," as used herein is meant to include all stereoisomers, geometric isomers, and tautomers of the structures depicted. Inhibitors herein identified by name or structure as one particular tautomeric form are intended to include other tautomeric forms unless otherwise specified.

For example, triazole inhibitors provided herein are intended to include the appropriate equivalent tautomeric forms shown below:

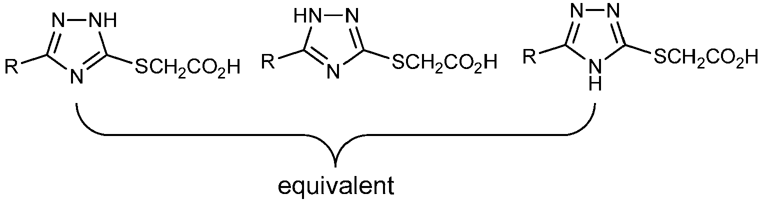

In some embodiments, an inhibitor provided herein, or salt thereof, is substantially isolated. By "substantially isolated" is meant that the inhibitor is at least partially or substantially separated from the environment in which it was formed or detected. Partial separation can include, for example, a composition enriched in the inhibitor provided herein. Substantial separation can include compositions containing at least about 50%, at least about 60%, at least about 70%, at least about 80%, at least about 90%, at least about 95%, at least about 97%, or at least about 99% by weight of the inhibitors provided herein, or salt thereof. Methods for isolating inhibitors and their salts are routine in the art.

The phrase "pharmaceutically acceptable" is used herein to refer to those inhibitors, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term "alkyl" includes a substituted or unsubstituted straight-chain alkyl groups (e.g., methyl, ethyl, propyl, butyl, pentyl, hexyl, heptyl, octyl, nonyl, decyl, *etc.*) and branched-chain alkyl groups (isopropyl, tert-butyl, isobutyl, *etc.*), cycloalkyl (alicyclic) groups (cyclopropyl, cyclopentyl, cyclohexyl, cycloheptyl, cyclooctyl), alkyl substituted cycloalkyl groups, and cycloalkyl substituted alkyl groups. In certain embodiments, a straight chain or branched chain alkyl has six or fewer carbon atoms in its backbone (e.g., C₁₋₆ for straight chain; C₃₋₆ for branched chain). The term C₁₋₆ includes alkyl groups containing 1 to 6 carbon atoms.

The term "alkenyl" includes a substituted or unsubstituted aliphatic groups that may or may not be substituted, as described above for alkyls, containing at least one double bond and at least two carbon atoms. For example, the term "alkenyl" hexenyl, heptenyl, octenyl, nonenyl, and decenyl) and branched-chain alkenyl groups. In certain embodiments, a straight chain or branched chain alkenyl group has six or fewer carbon atoms in its backbone (e.g., C₂₋₆ for straight chain; C₃₋₆ for branched chain). The term C₂₋₆ includes alkenyl groups containing 2 to 6 carbon atoms.

The term "alkynyl" includes a substituted or unsubstituted unsaturated aliphatic group analogous in length and possible substitution to the alkyls described above, but which contain at least one triple bond and two carbon atoms. For example, the term "alkynyl" includes straight-chain alkynyl groups (e.g., ethynyl, propynyl, butynyl, pentynyl, hexynyl, heptynyl, octynyl, nonynyl, and decynyl) and branched-chain alkynyl groups. In certain embodiments, a straight chain or branched chain alkynyl group has six or fewer carbon atoms in its backbone (e.g., C₂₋₆ for straight chain; C₃₋₆ for branched chain). The term C₂₋₆ includes alkynyl groups containing 2 to 6 carbon atoms.

As used herein, "haloalkyl" means a hydrocarbon substituent, which is a linear or branched or cyclic alkyl, alkenyl or alkynyl substituted with one or more chloro, bromo, fluoro, or iodo atom(s). In some embodiments, a haloalkyl is a fluoroalkyl, wherein one or more of the hydrogen atoms have been substituted by fluoro. In some embodiments, haloalkyls are of 1 to about 3 carbons in length (e.g., 1 to about 2 carbons in length or 1 carbon in length).

The term "cycloalkyl" includes a substituted or unsubstituted cyclic aliphatic group which may be saturated or unsaturated. For example, cycloalkyl groups include cyclopropyl, cyclopentyl, cyclohexyl, cycloheptyl, and cyclooctyl. In some embodiments, cycloalkyls can have from 3-8 carbon atoms in their ring structure, for example, they can have 3, 4, 5, or 6 carbons in the ring structure.

In general, the term "aryl" includes substituted or unsubstituted aromatic rings, including 5- and 6-membered single-ring aromatic groups, such as benzene and phenyl. Furthermore, the term "aryl" includes multicyclic aryl groups, e.g., tricyclic, bicyclic, such as naphthalene and anthracene. In some embodiments, the aryl is a C₆-C₁₄ aryl group. In some embodiments, the aryl is a C₆-C₁₀ aryl group. In some embodiments, the aryl is a C₅-C₆ aryl group. In some embodiments, the aryl is a substituted or unsubstituted phenyl. In some embodiments, the aryl is substituted phenyl. In some embodiments, the aryl is unsubstituted phenyl.

The term "heteroaryl" means a substituted or unsubstituted mono-, bi-, tri- or polycyclic group having 5 to 14 ring atoms, alternatively 5, 6, 9, or 10 ring atoms; having 6, 10, or 14 pi electrons shared in a cyclic array; wherein at least one ring in the system is aromatic, and at least one ring in the system contains one or more heteroatoms independently selected from the group consisting of N, O, and S. Exemplary heteroaryl groups include, for example, pyrrole, furan, thiophene, thiazole, isothiaozole, imidazole, triazole, tetrazole, pyrazole, oxazole, isooxazole, pyridine, pyrazine, pyridazine, and pyrimidine, and the like. Furthermore, the term "heteroaryl" includes multicyclic heteroaryl groups, e.g., tricyclic, bicyclic, such as benzoxazole, benzodioxazole, benzothiazole, benzoimidazole, benzothiophene, methylenedioxyphenyl, quinoline, isoquinoline, napthyridine, indole, benzofuran, purine, benzofuran, quinazoline, deazapurine, indazole, indolizine, carbazole, or dibenzo[b,d]furan.In some embodiments, the heteroaryl group is unsubstituted. In some embodiments, the heteroaryl group is substituted. In some embodiments, the heteroaryl group is a 5-14 membered heteroaryl group. In some embodiments, the heteroaryl group is a 5-10 membered heteroaryl group. In some embodiments, the heteroaryl is a 5-6 membered heteroaryl group.

The term "heterocycloalkyl" includes substituted or unsubstituted groups, including but not limited to, 3- to 10-membered single or multiple rings having one to five heteroatoms, for example, piperazine, pyrrolidine, piperidine, or homopiperazine. In some embodiments, the heterocycloalkyl is a 3-10 membered heterocycloalkyl group. In some embodiments, the heterocycloalkyl is a 5-10 membered heterocycloalkyl group. In some embodiments, the heterocycloalkyl is a 5-7 membered heterocycloalkyl group. In some embodiments, the heterocycloalkyl is a 5-6 membered heterocycloalkyl group.

The term "substituted" means that an atom or group of atoms replaces hydrogen as a "substituent" attached to another group. For aryl and heteroaryl groups, the term "substituted", unless otherwise indicated, refers to any level of substitution, namely mono, di, tri, tetra, or penta substitution, where such substitution is permitted. The substituents are independently selected, and substitution may be at any chemically accessible position. In some cases, two sites of substitution may come together to form a 3-10 membered cycloalkyl or heterocycloalkyl ring. In some embodiments, two sites of substitution mav come together to form a 3-10 membered cycloalkyl group, a 5-14 membered aryl group, a 5-14 membered heteroaryl group, or a 3-10 membered heterocycloalkyl group. Non-limiting examples of substituents include: (C₁-C₆)alkyl, halo, (C₁-C₆)haloalkyl, -CN, -NR⁸R⁹, -NO₂, -O(C₁-C₆)haloalkyl, -OR⁸, -OC(O)R⁸, -C(O)R⁸, -C(O)OR⁸, -C(O)NR⁸R⁹, -SR⁸, -S(O)R⁸, -SO₂R⁸, -SO₂NR⁸R⁹, (C₃-C₇) cycloalkyl, (C₃-C₇)heterocycloalkyl, (C₅-C₁₄)aryl, and (C₅-C₁₄)heteroaryl, wherein R⁸ and R⁹ are independently selected from H and (Ci-C₆)alkyl.

### Inhibitors

This document provides inhibitors of GRK6 polypeptides as well as methods and materials for using such inhibitors to treat hematological malignancies, inflammation diseases, and autoimmune disorders.

Disclosed herein is a compound of Formula I: 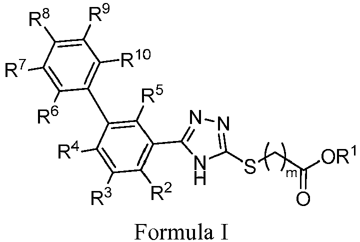 or a pharmaceutically acceptable salt thereof,
wherein:
R¹ is selected from the group consisting of H and (C₁-C₆)alkyl;
each of R², R³, R⁴, and R⁵ are independently selected from the group consisting of: H, halo, substituted or unsubstituted (C₁-C₆)alkyl, (C₁-C₆)haloalkyl, OR^{A}, CN, NO₂, C(O)R^{A}, NR^{A}R^{B}, -S(O)₂R^{A}, -S(O)₂NR^{A}R^{B}, -S(O)NR^{A}R^{B}, -NR^{A}S(O)₂R^{B}, - C(NR^{A})R^{B}, -C(O)NR^{A}R^{B}, a substituted or unsubstituted aryl, a substituted or unsubstituted heteroaryl, a substituted or unsubstituted cycloalkyl, and a substituted or unsubstituted heterocycloalkyl;
each of R⁶, R⁷, R⁸, R⁹, and R¹⁰ are independently selected from the group consisting of: H, substituted or unsubstituted (C₁-C₆)alkyl, (C₁-C₆)haloalkyl, OR^{A}, NO₂, C(O)R^{A}, NR^{A}R^{B}, -S(O)₂NR^{A}R^{B}, -S(O)NR^{A}R^{B}, -C(NR^{A})R^{B}, -C(O)NR^{A}R^{B}, - NR^{A}S(O)₂R^{B}, -NR^{A}C(O)R^{B}, -S(O)₂R^{A}, a substituted or unsubstituted aryl, a substituted or unsubstituted heteroaryl, a substituted or unsubstituted cycloalkyl, and a substituted or unsubstituted heterocycloalkyl; or
R⁶ and R⁷, R⁷ and R⁸, R⁸ and R⁹, or R⁹ and R¹⁰ can come together to form a substituted or unsubstituted fused cycloalkyl ring, a substituted or unsubstituted fused heterocycloalkyl ring, or a substituted or unsubstituted fused heteroaryl ring;
each R^{A} and R^{B} is independently selected from the group consisting of H and (Ci-C₆)alkyl; and
m is an integer from 1 to 2;
wherein if R², R³, R⁴, and R⁵ are H, then R⁶ and R⁸ are not halo, CN, or O(C₁-C₆ alkyl); and
wherein if R⁶, R⁷, R⁸, R⁹, and R¹⁰ are H, at least one of R², R³, R⁴, and R⁵ is not H.

In some embodiments, R¹ is H. In some such embodiments, each of R², R³, R⁴, and R⁵ are independently selected from the group consisting of: H, NO₂, and a substituted or unsubstituted (C₁-C₆)alkyl. In certain of these embodiments, one of R⁶ and R⁷, R⁷and R⁸, R⁸ and R⁹, or R⁹ and R¹⁰ come together to form a substituted or unsubstituted fused cycloalkyl ring. Alternatively, one of R⁶ and R⁷, R⁷ and R⁸, R⁸ and R⁹, or R⁹ and R¹⁰ come together to form a substituted or unsubstituted fused heterocycloalkyl ring. In other embodiments, one of R⁶ and R⁷, R⁷ and R⁸, R⁸ and R⁹, or R⁹ and R¹⁰ come together to form a substituted or unsubstituted fused heteroaryl ring. In certain other embodiments, each of R⁶, R⁷, R⁸, R⁹, and R¹⁰ are independently selected from the group consisting of: H, substituted or unsubstituted (C₁-C₆)alkyl, OR^{A}, C(O)R^{A}, NR^{A}R^{B}, -S(O)₂NR^{A}R^{B}, -S(O)NR^{A}R^{B}, -C(NR^{A})R^{B}, -C(O)NR^{A}R^{B}, - NR^{A}S(O)₂R^{B}, -NR^{A}C(O)R^{B}, -S(O)₂R^{A}, and a substituted or unsubstituted heteroaryl.

In some embodiments, m is 1.

Non-limiting examples of a compound of Formula I include: 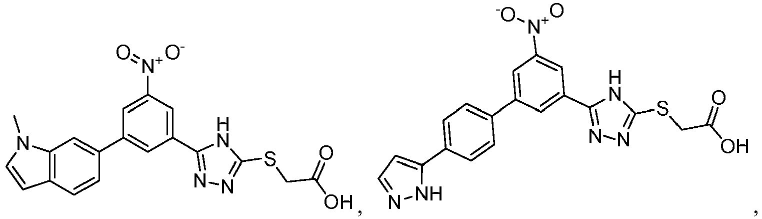 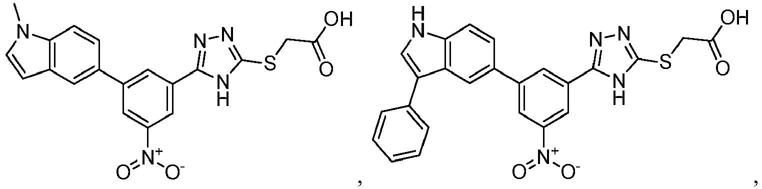 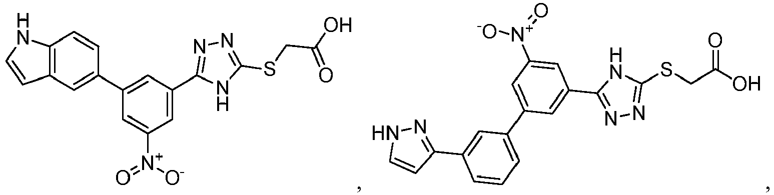 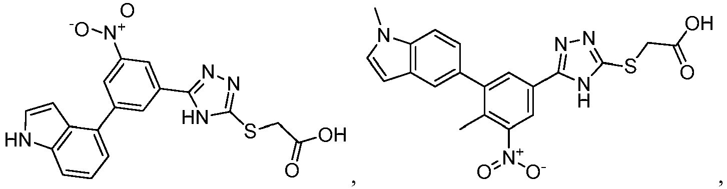 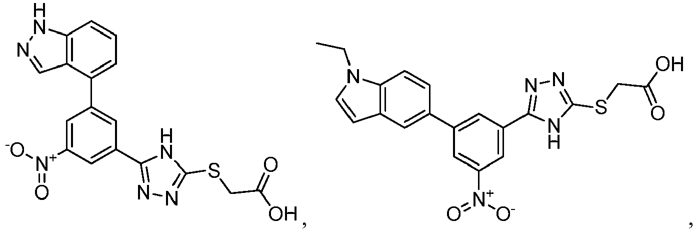 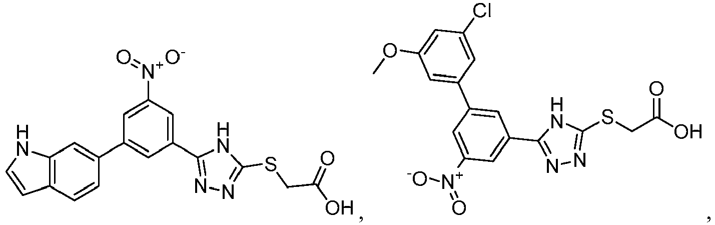 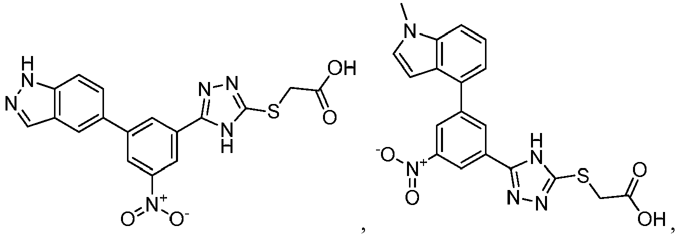 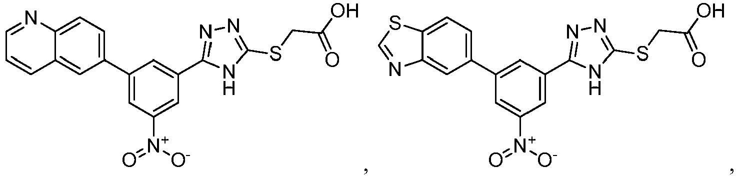 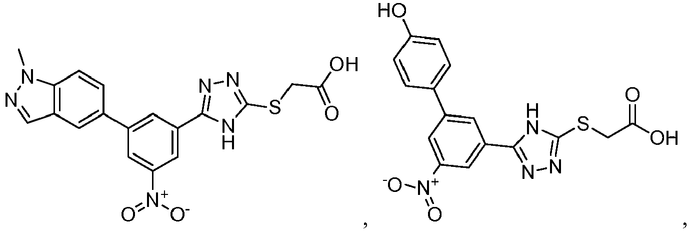 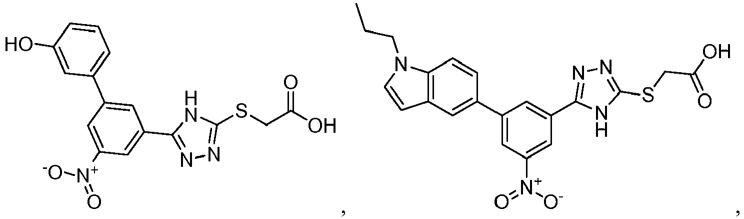 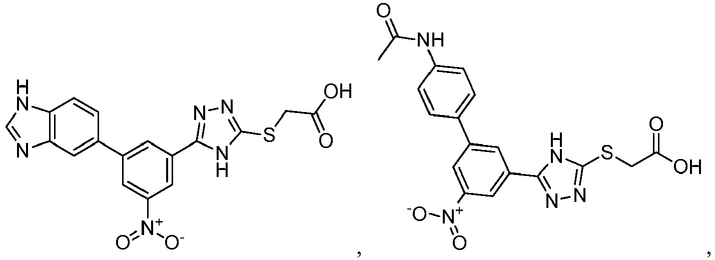 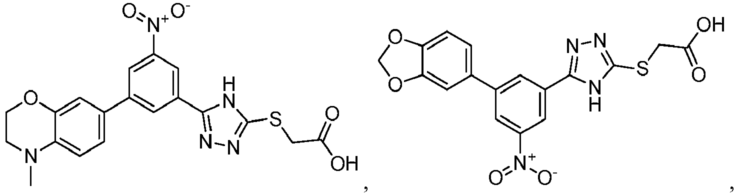 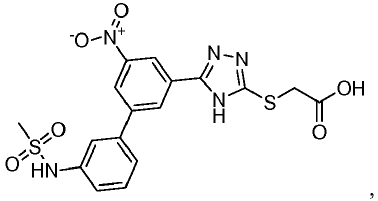 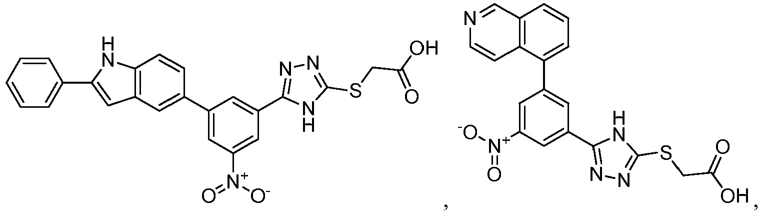 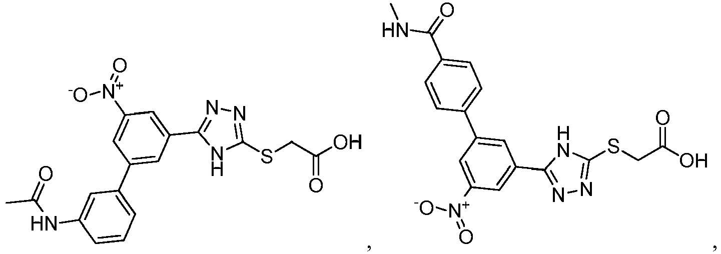 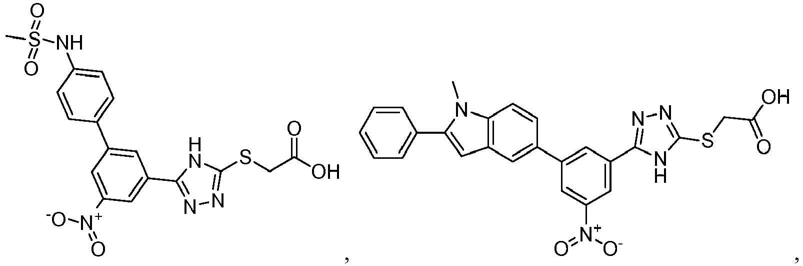 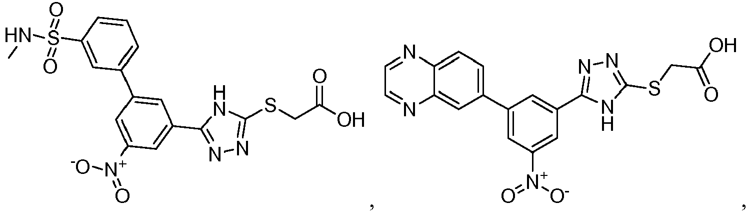 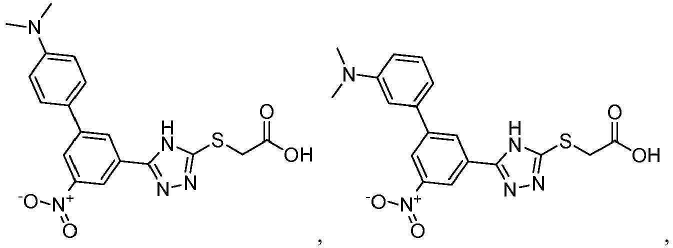 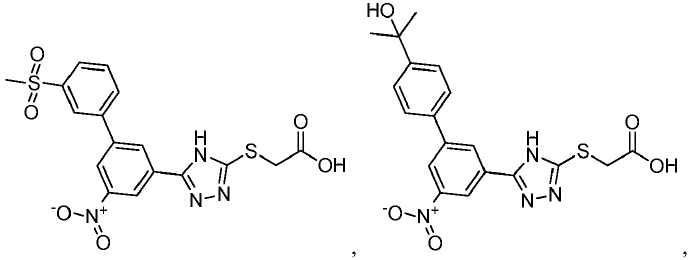 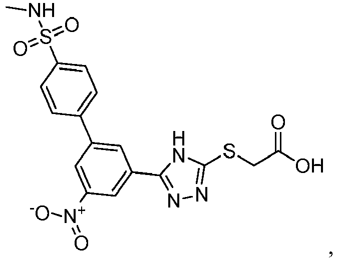 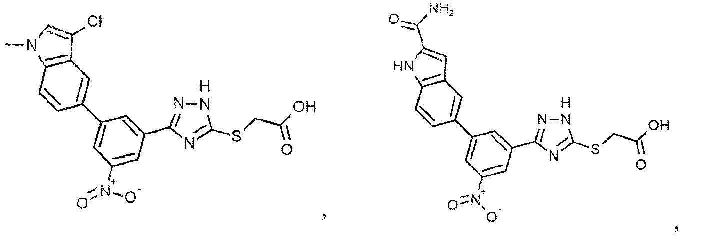 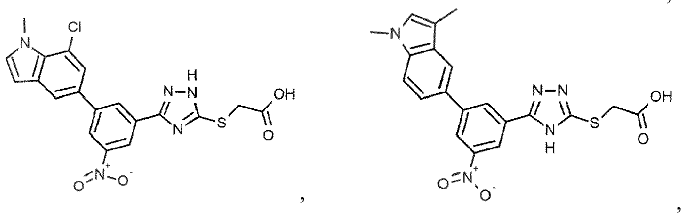 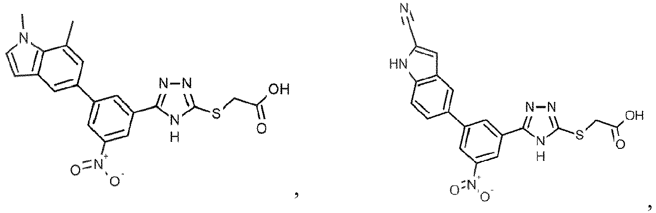 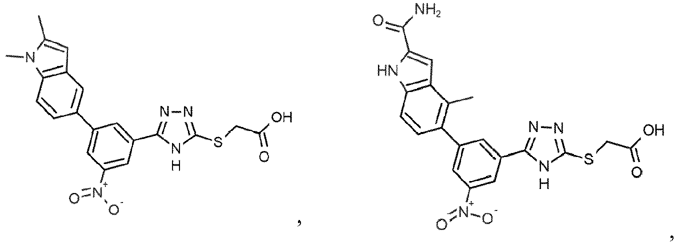 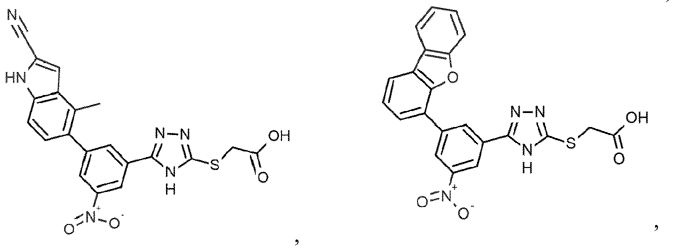 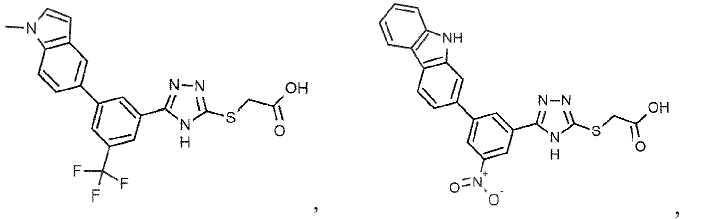 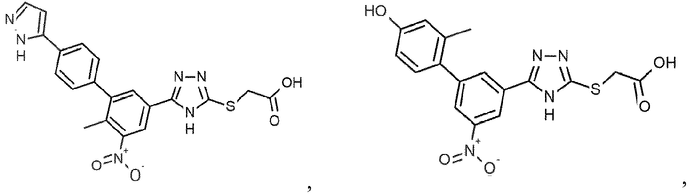 and 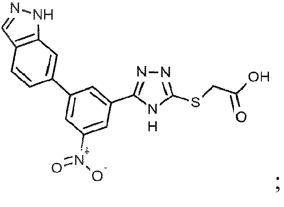 or a pharmaceutically acceptable salt thereof.

Further disclosed herein is a compound of Formula II: 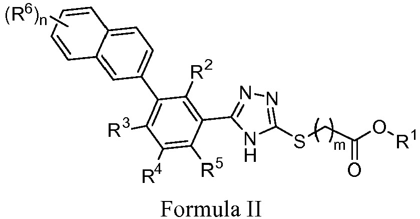 or a pharmaceutically acceptable salt thereof,
wherein:
R¹ is selected from the group consisting of H and (C₁-C₆)alkyl;
each of R², R³, R⁴, and R⁵ are independently selected from the group consisting of: H, halo, substituted or unsubstituted (C₁-C₆)alkyl, (C₁-C₆)haloalkyl, OR^{A}, CN, NO₂, C(O)R^{A}, NR^{A}R^{B}, -S(O)₂R^{A}, -S(O)₂NR^{A}R^{B}, -S(O)NR^{A}R^{B}, -NR^{A}S(O)₂R^{B}, - C(NR^{A})R^{B}, -C(O)NR^{A}R^{B}, -NR^{A}C(O)R^{B}, a substituted or unsubstituted aryl, a substituted or unsubstituted heteroaryl, a substituted or unsubstituted cycloalkyl, and a substituted or unsubstituted heterocycloalkyl;
each R⁶ is independently selected from the group consisting of: halo, substituted or unsubstituted (C₁-C₆)alkyl, (C₁-C₆)haloalkyl, OR^{A}, CN, NO₂, C(O)R^{A}, NR^{A}R^{B}, - S(O)₂NR^{A}R^{B}, -S(O)NR^{A}R^{B}, -C(NR^{A})R^{B}, -C(O)NR^{A}R^{B}, a substituted or unsubstituted aryl, a substituted or unsubstituted heteroaryl, a substituted or unsubstituted cycloalkyl, and a substituted or unsubstituted heterocycloalkyl; or
each R^{A} and R^{B} is independently selected from the group consisting of H, (Ci-C₆)alkyl, a substituted or unsubstituted heterocycloalkyl, and a substituted or unsubstituted heteroaryl;
m is an integer from 1 to 2; and
n is an integer from 0 to 7;
wherein if n is 0, then at least one of R², R³, R⁴, and R⁵ is not H; and
wherein if R², R³, R⁴, and R⁵ are H, then when n is 1, R⁶ is not O(C₁-C₆ alkyl).

In some embodiments, R¹ is H. In some such embodiments, each of R², R³, R⁴, and R⁵ are independently selected from the group consisting of: H, halo, a substituted or unsubstituted (C₁-C₆)alkyl, NO₂, CN, NR^{A}R^{B}, -NR^{A}S(O)₂R^{B}, - C(NR^{A})R^{B}, -C(O)NR^{A}R^{B}, and a substituted or unsubstituted heteroaryl. In certain of these embodiments, n is 1 and R⁶ is selected from H and OR^{A}.

Non-limiting examples of a compound of Formula II include: 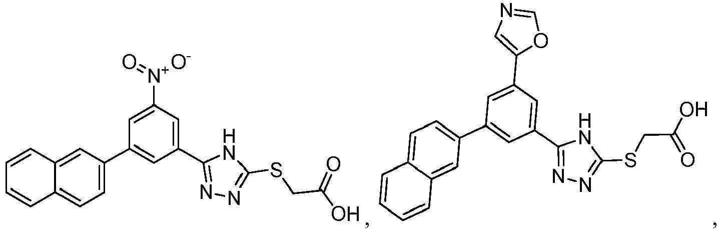 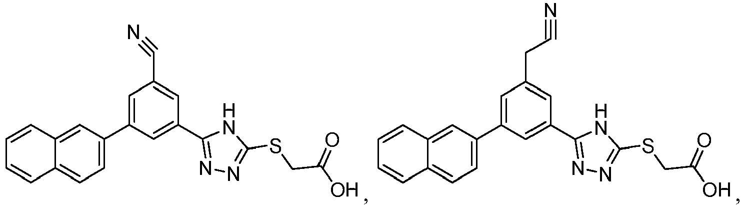 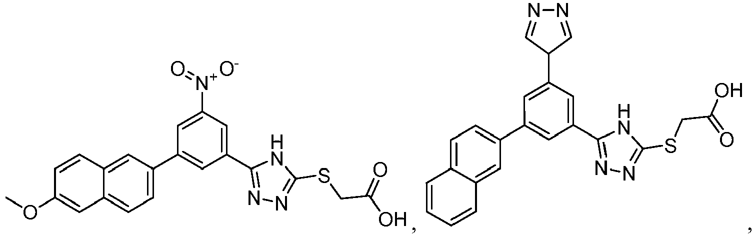 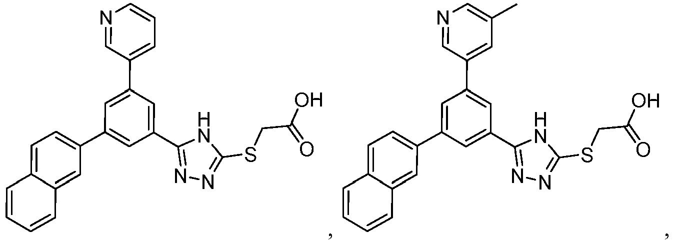 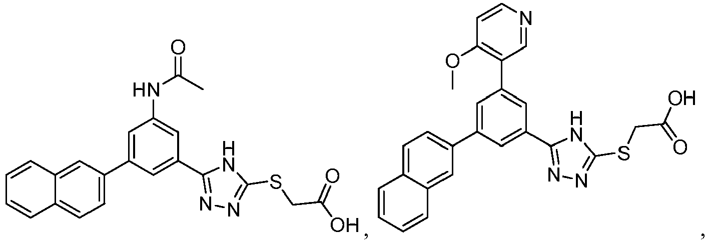 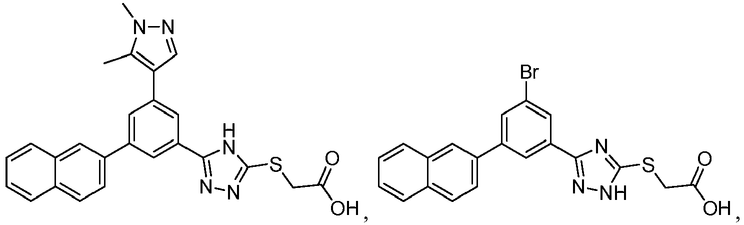 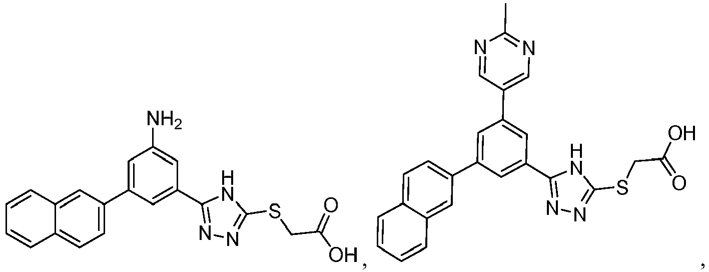 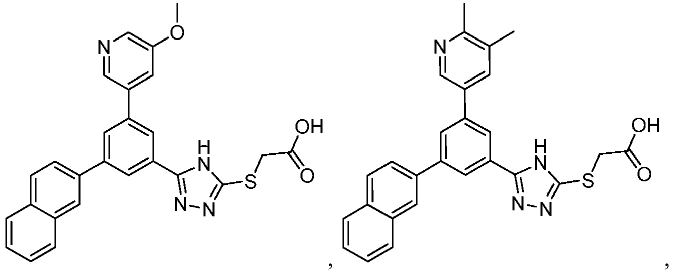 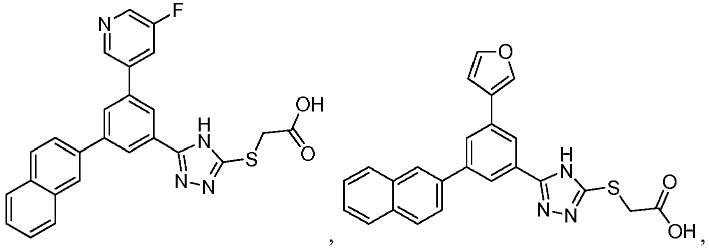 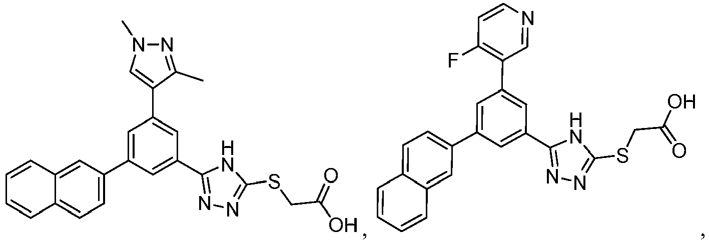 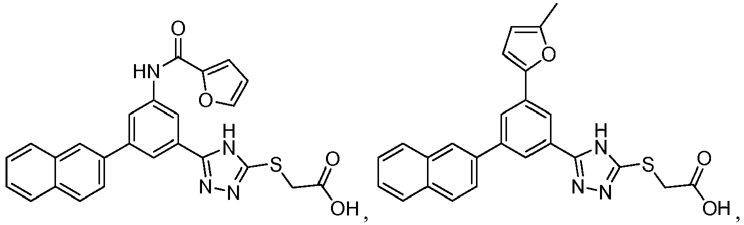 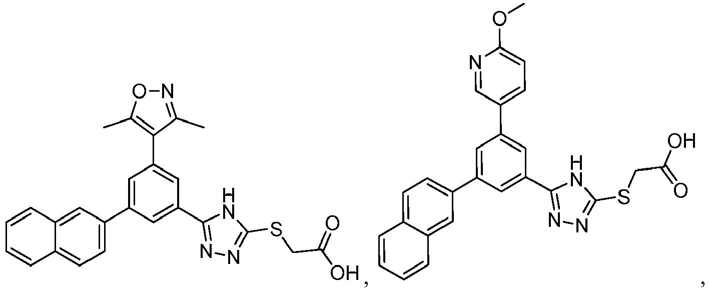 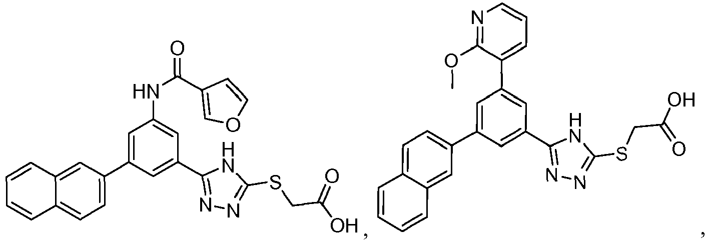 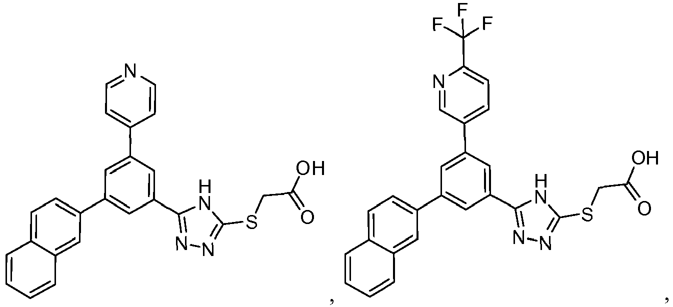 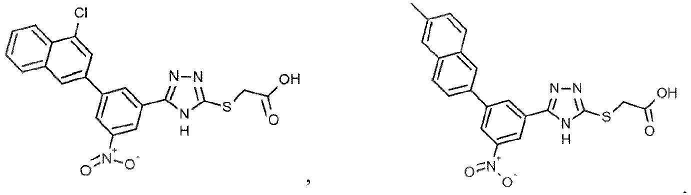 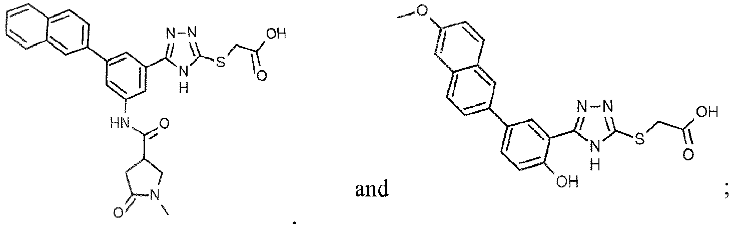 or a pharmaceutically acceptable salt thereof.

Further disclosed herein is a compound of Formula III: 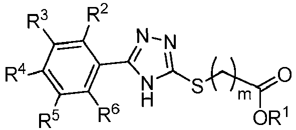 or a pharmaceutically acceptable salt thereof,
wherein:
R¹ is selected from the group consisting of H and (C₁-C₆)alkyl;
each of R², R⁴, R⁵, and R⁶ are independently selected from the group consisting of: H, halo, substituted or unsubstituted (C₁-C₆)alkyl, (C₁-C₆)haloalkyl, OR^{A}, CN, NO₂, C(O)R^{A}, NR^{A}R^{B}, -S(O)₂R^{A}, -S(O)₂NR^{A}R^{B}, -S(O)NR^{A}R^{B}, -NR^{A}S(O)₂R^{B}, -C(NR^{A})R^{B}, -C(O)NR^{A}R^{B}, a substituted or unsubstituted aryl, a substituted or unsubstituted heteroaryl, a substituted or unsubstituted cycloalkyl, and a substituted or unsubstituted heterocycloalkyl;
R³ is selected from the group consisting of: a substituted or unsubstituted pyrazole and a substituted or unsubstituted isoxazole;
each R^{A} and R^{B} is independently selected from the group consisting of H and (Ci-C₆)alkyl; and
m is an integer from 1 to 2.

In some embodiments, R¹ is H. In some such embodiments, each of R², R⁴, R⁵, and R⁶ are independently selected from the group consisting of H and NO₂. In certain of these embodiments, R³ is a substituted or unsubstituted pyrazole. For example, R³ can be a substituted pyrazole, including, for example, wherein the pyrazole is substituted with one or more of: a substituted or unsubstituted (Ci-C₆)alkyl, (C₁-C₆)haloalkyl, a substituted or unsubstituted aryl, and a substituted or unsubstituted aralkyl. In some embodiments, R³ is a substituted or unsubstituted isoxazole.

Non-limiting examples of a compound of Formula III include: 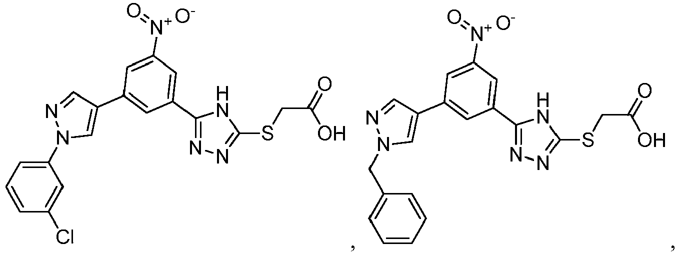 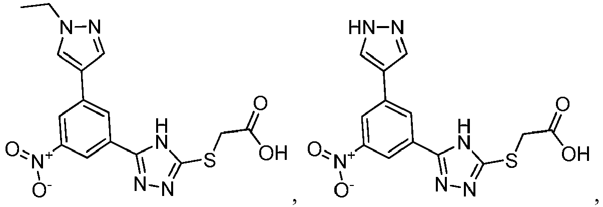 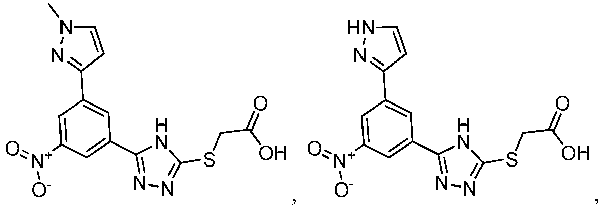 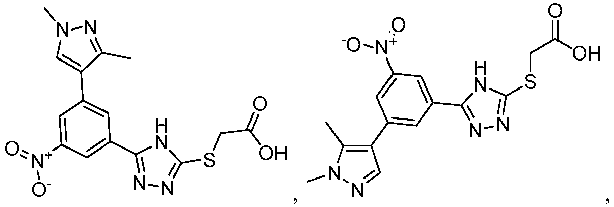 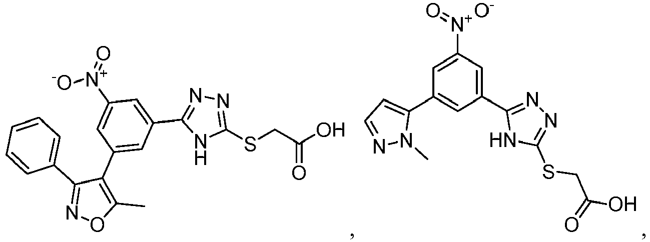 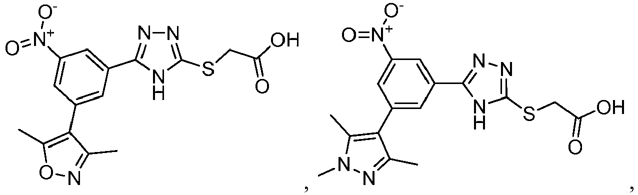 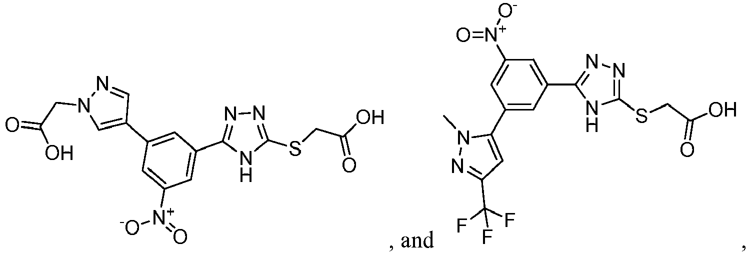 or a pharmaceutically acceptable salt thereof.

Inhibitors as disclosed herein also include, for example, the following compounds: 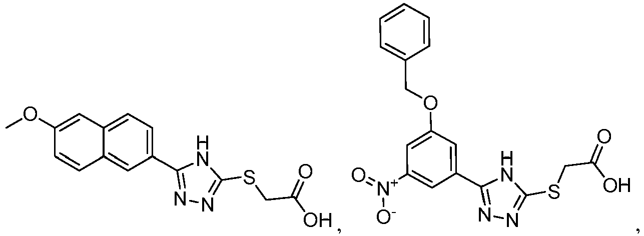 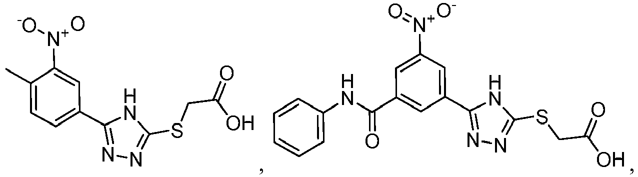 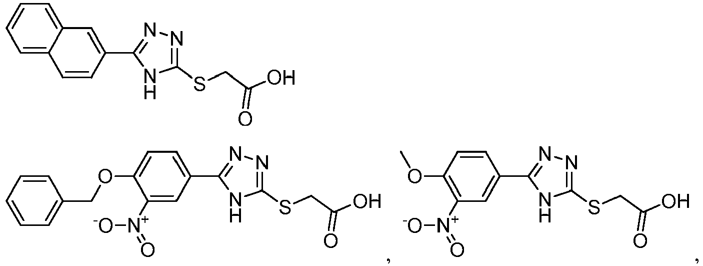 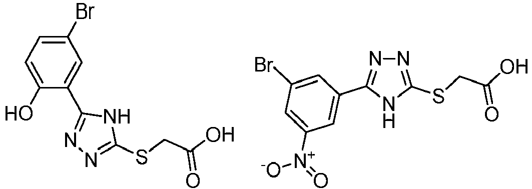 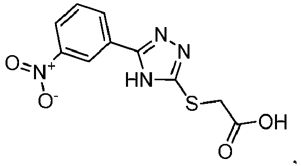 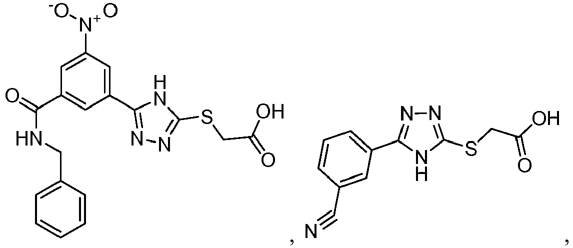 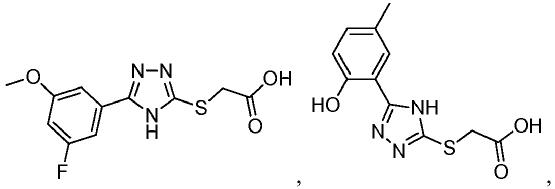 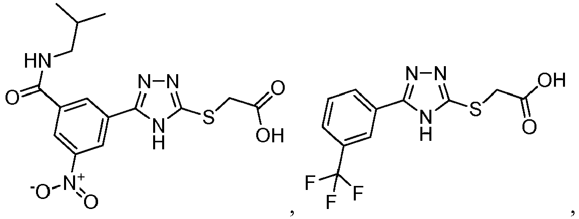 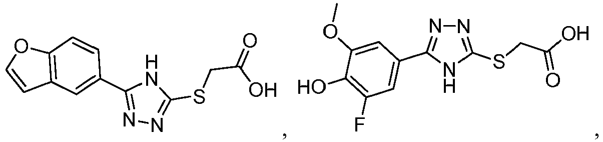 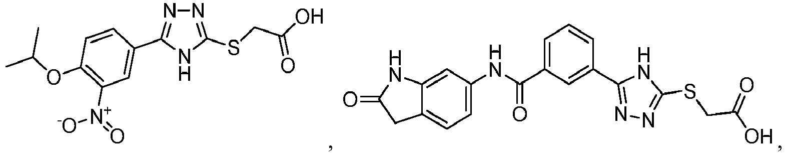 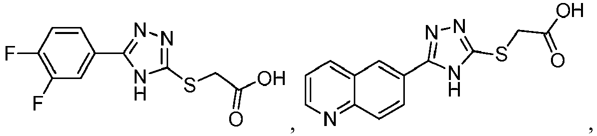 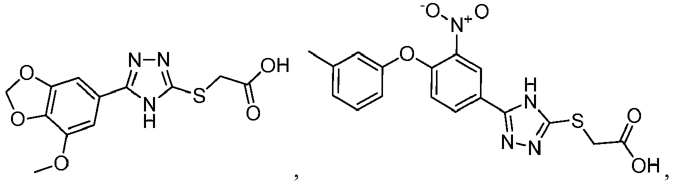 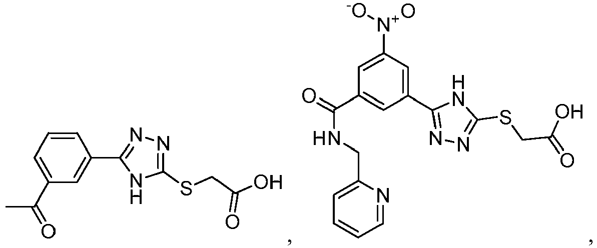 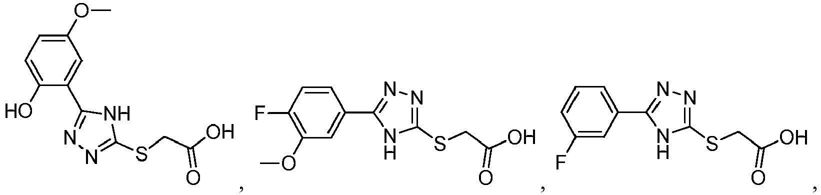 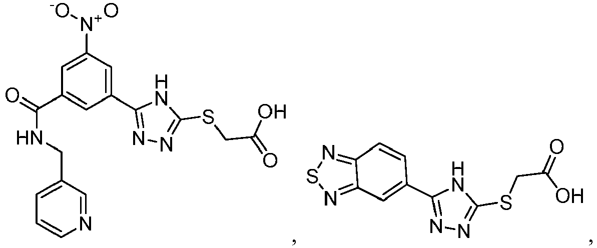 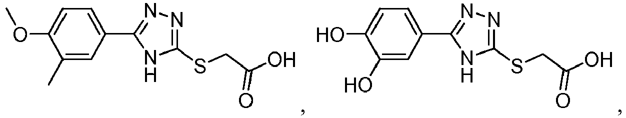 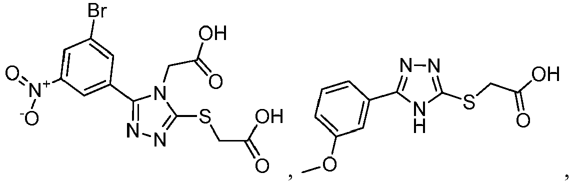 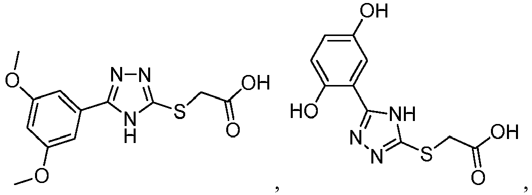 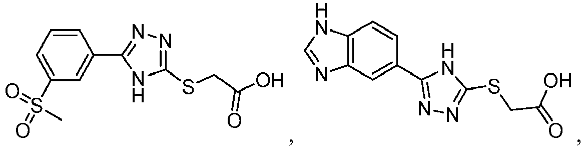 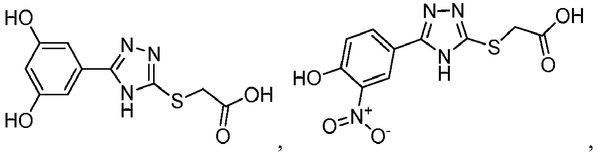 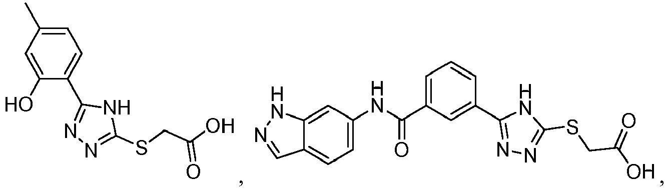 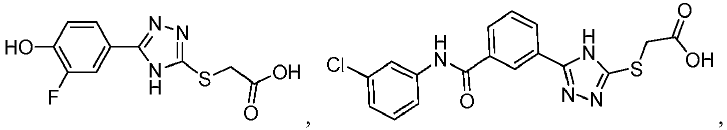 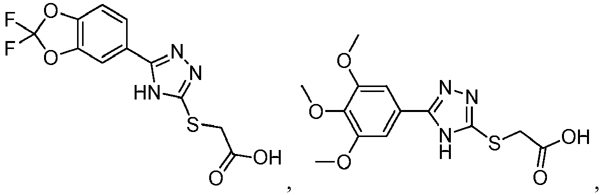 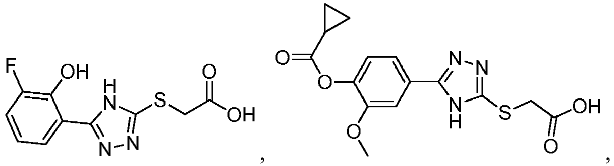 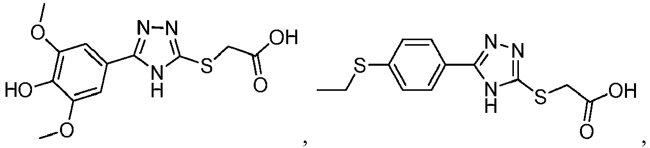 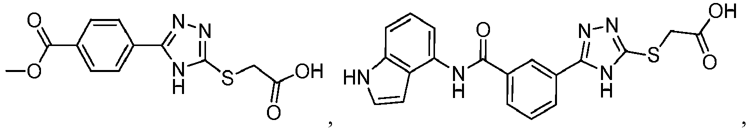 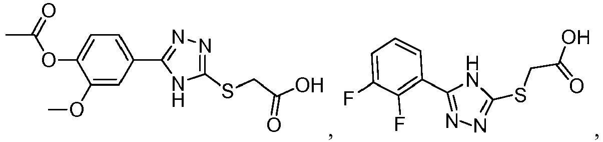 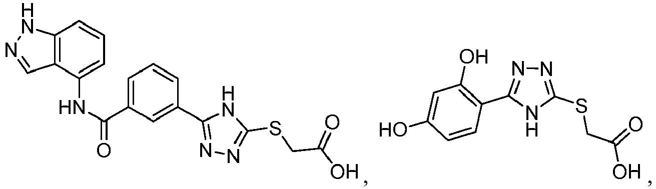 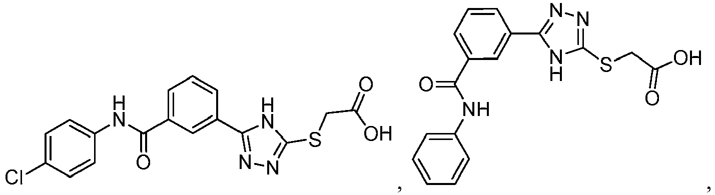 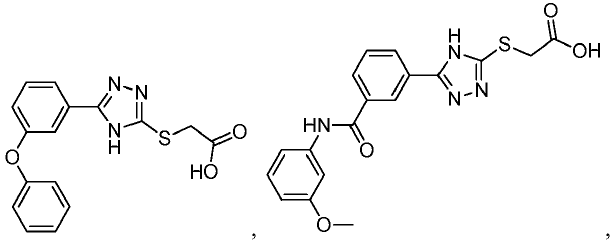 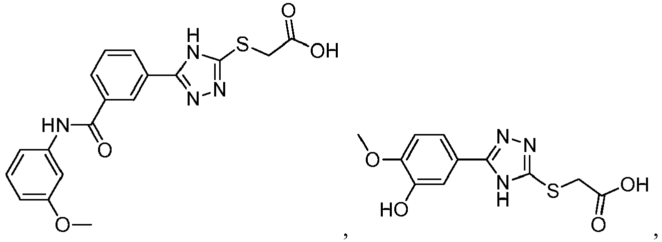 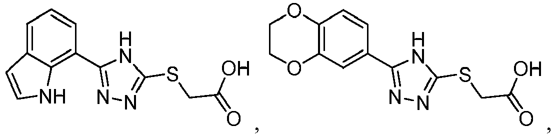 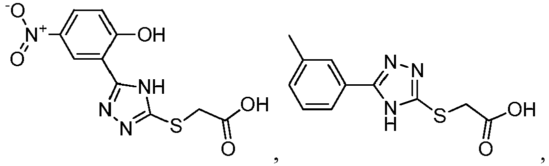 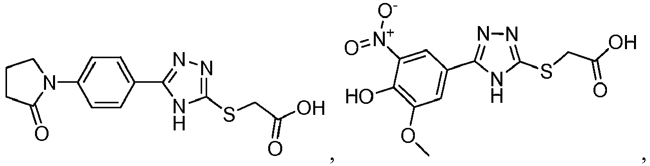 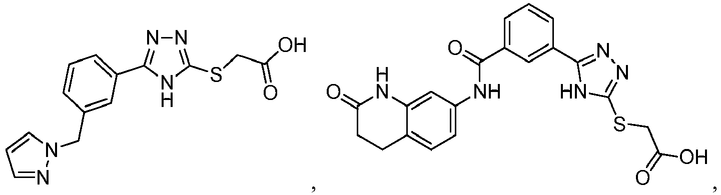 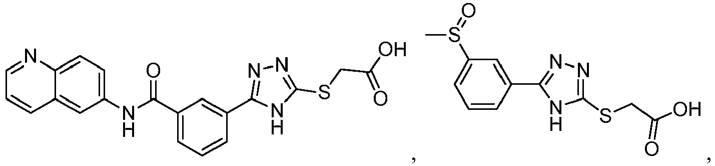 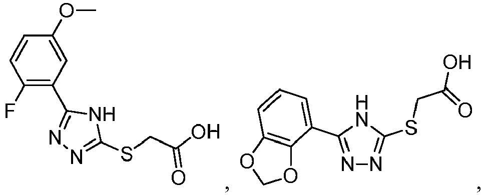 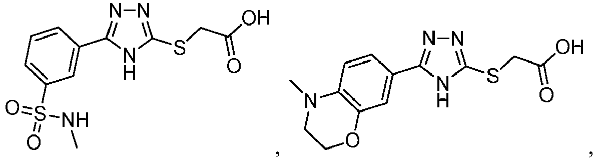 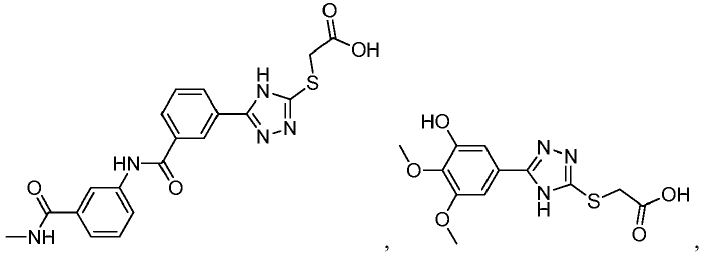 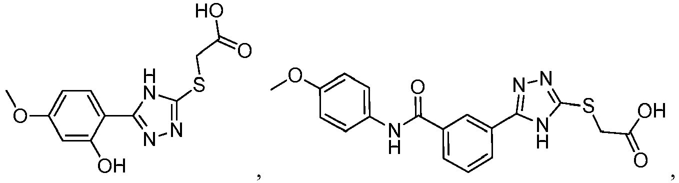 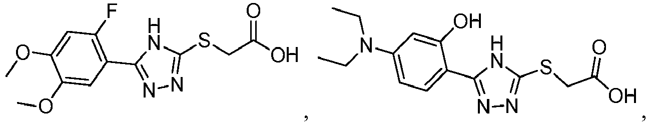 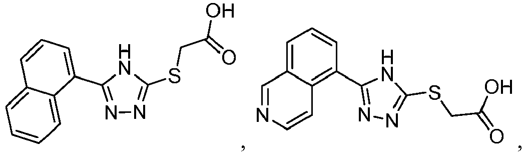 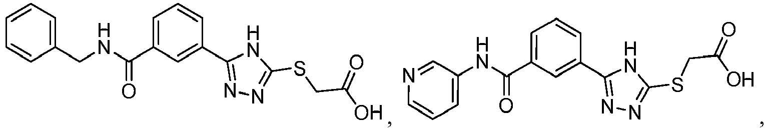 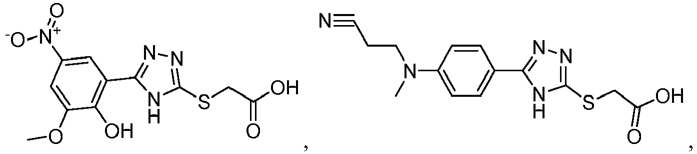 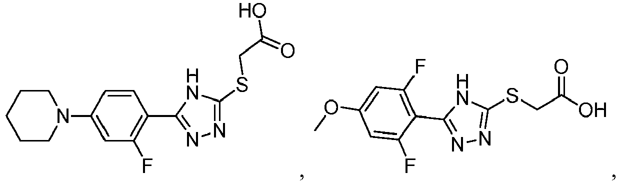 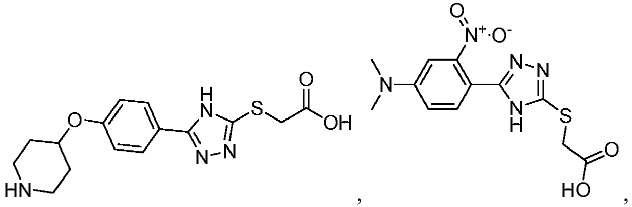 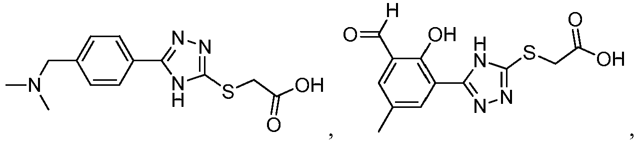 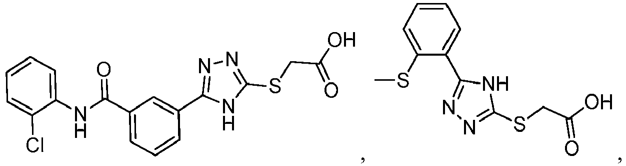 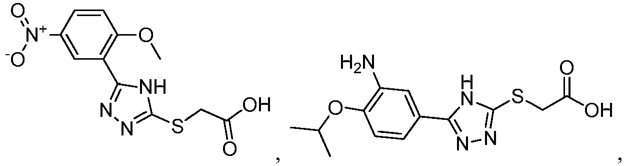 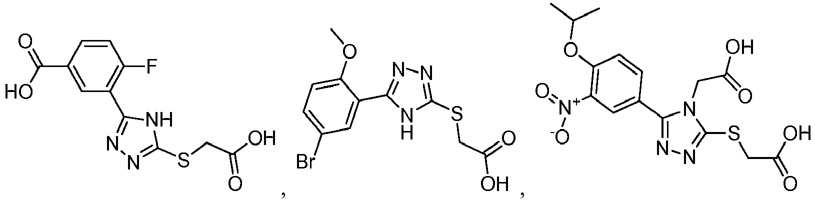 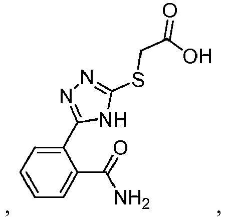 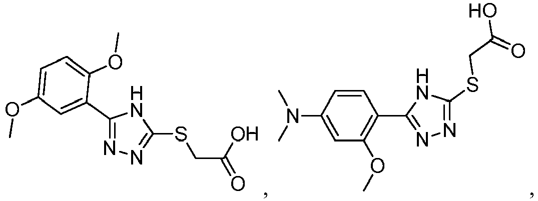 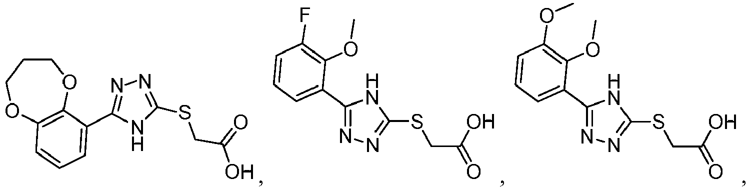 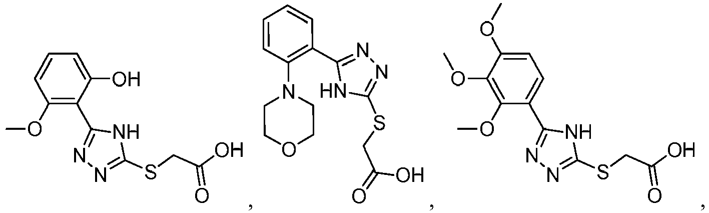 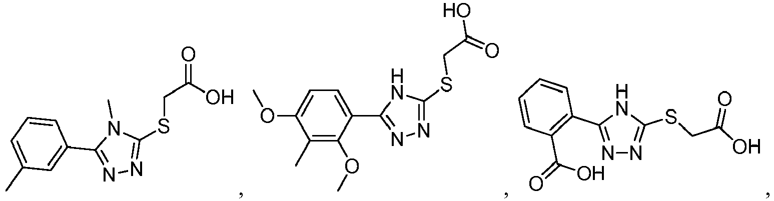 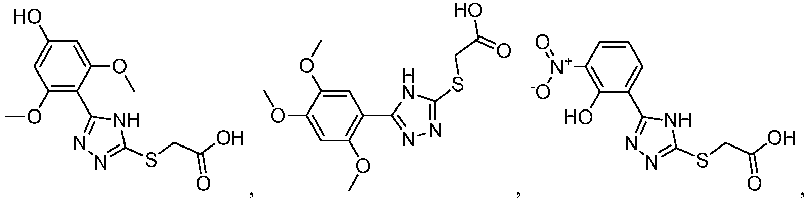 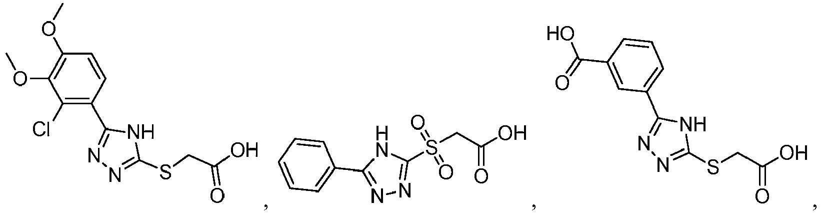 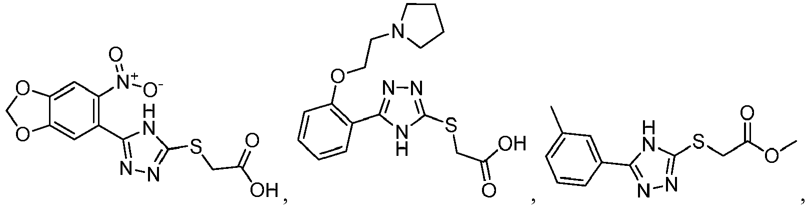 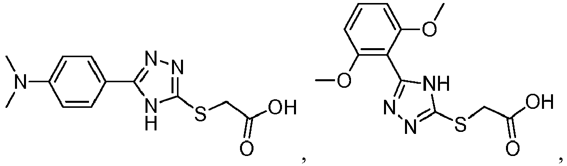 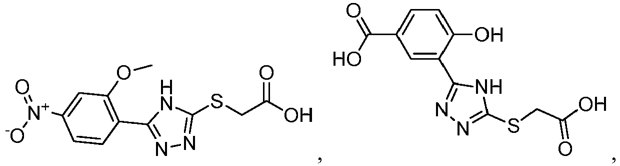 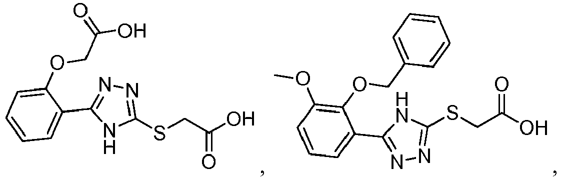 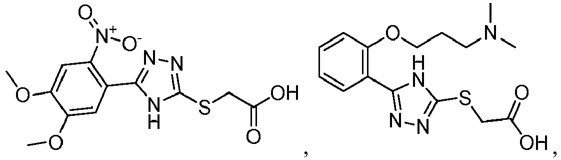 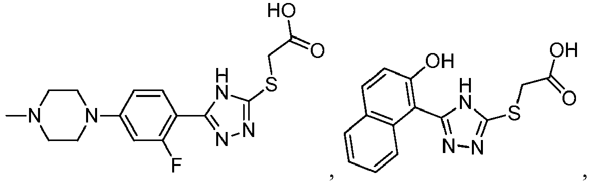 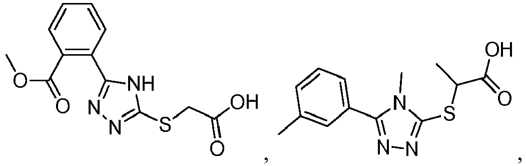 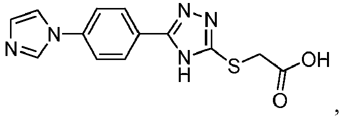 or a pharmaceutically acceptable salt thereof.

Non-limiting examples of an inhibitor as disclosed herein include the compounds of Table 1, or a pharmaceutically acceptable salt thereof.

**Table 1.**

| **#** | **Compound Structure** |
|---|---|
| **1** | 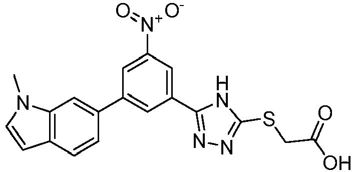 |
| **2** | 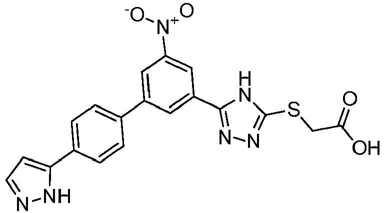 |
| **3** | 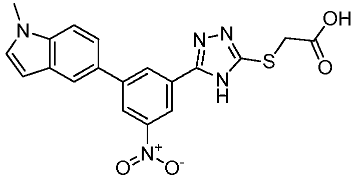 |
| **4** | 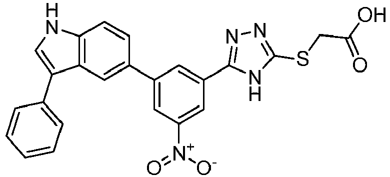 |
| **5** | 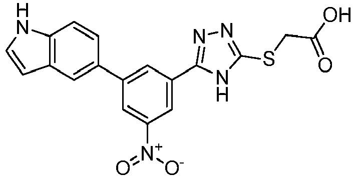 |
| **6** | 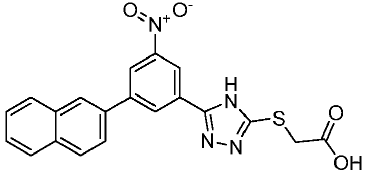 |
| **7** | 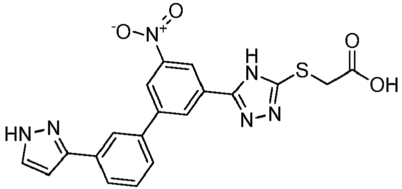 |
| **8** | 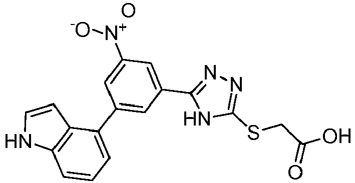 |
| **9** | 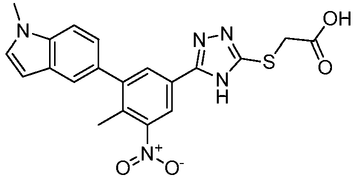 |
| **10** | 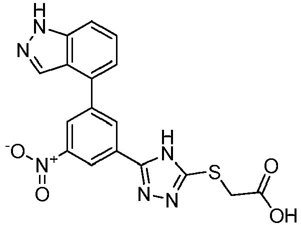 |
| **11** | 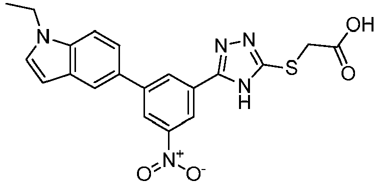 |
| **12** | 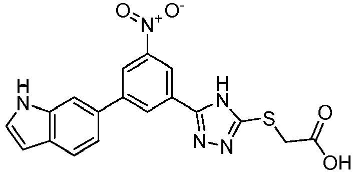 |
| **13** | 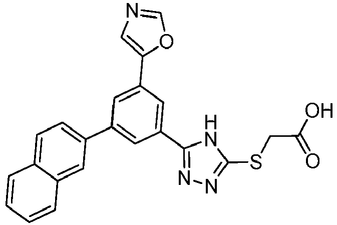 |
| **14** | 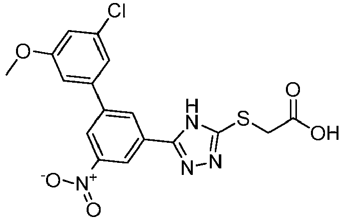 |
| **15** | 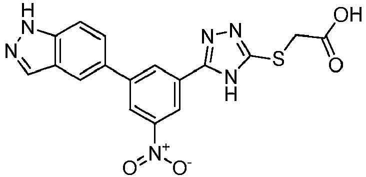 |
| **16** | 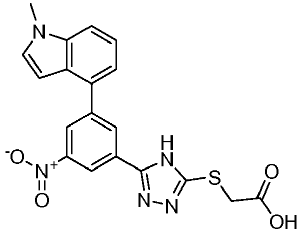 |
| **17** | 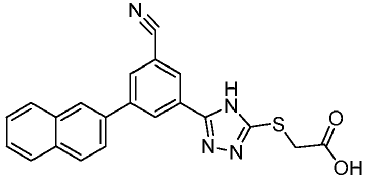 |
| **18** | 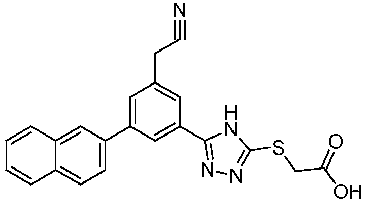 |
| **19** | 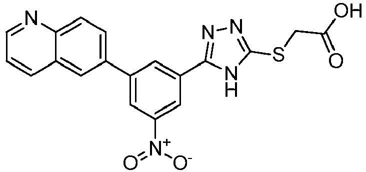 |
| **20** | 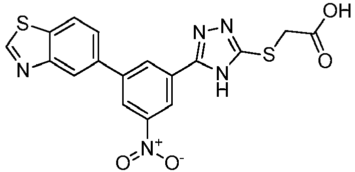 |
| **21** | 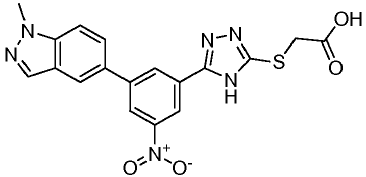 |
| **22** | 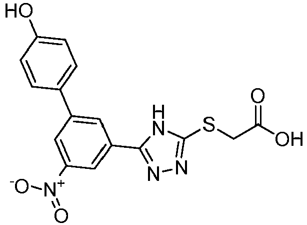 |
| **23** | 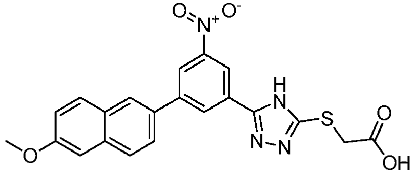 |
| **24** | 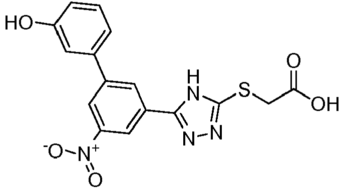 |
| **25** | 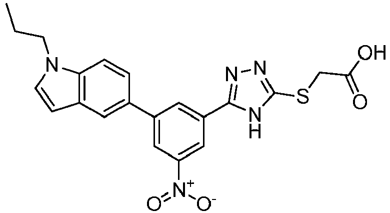 |
| **26** | 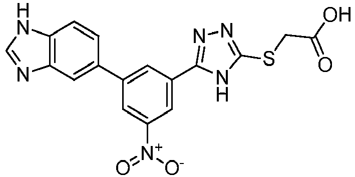 |
| **27** | 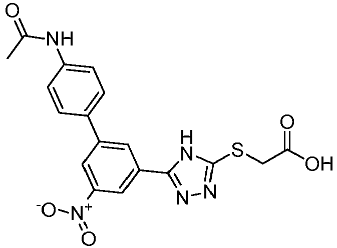 |
| **28** | 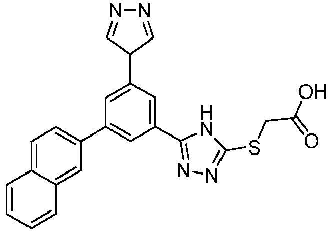 |
| **29** | 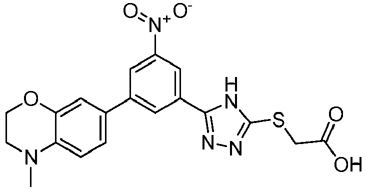 |
| **30** | 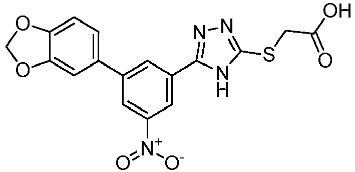 |
| **31** | 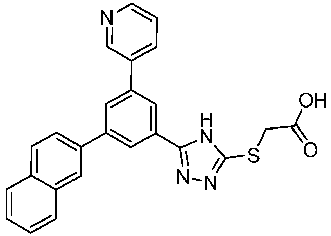 |
| **32** | 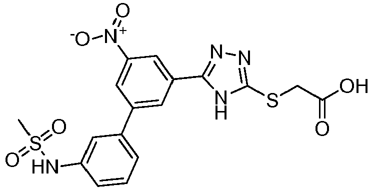 |
| **33** | 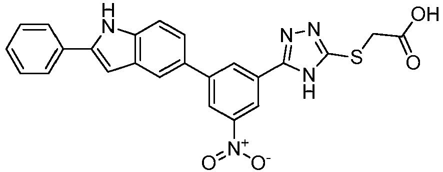 |
| **34** | 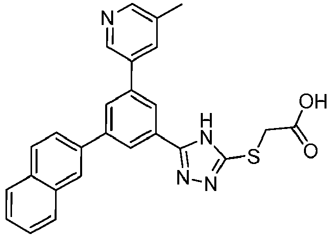 |
| **35** | 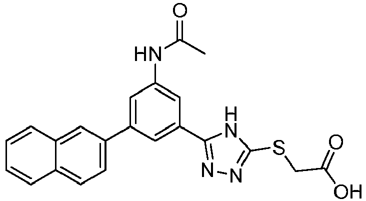 |
| **36** | 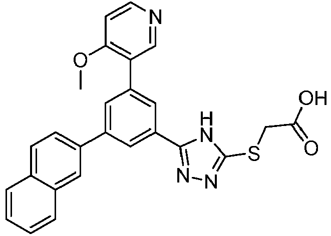 |
| **37** | 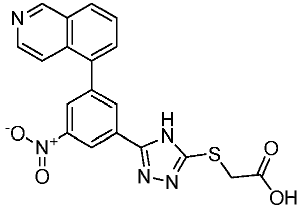 |
| **38** | 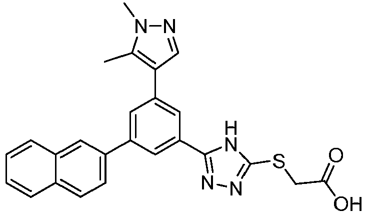 |
| **39** | 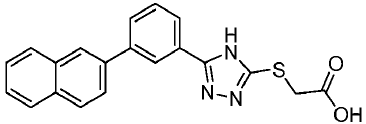 |
| **40** | 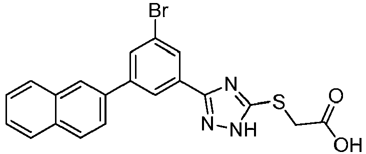 |
| **41** | 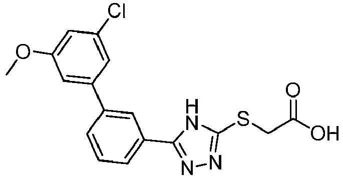 |
| **42** | 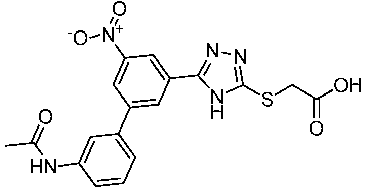 |
| **43** | 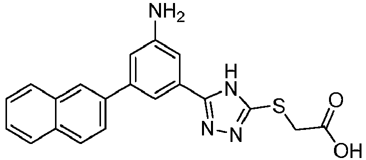 |
| **44** | 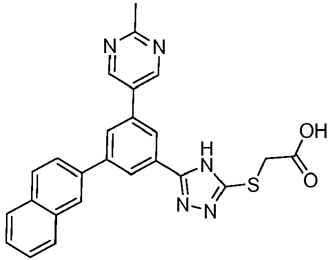 |
| **45** | 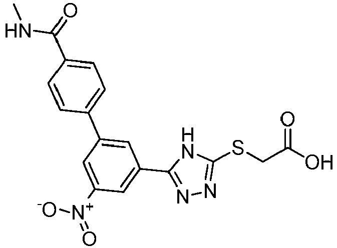 |
| **46** | 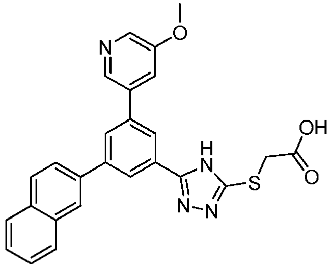 |
| **47** | 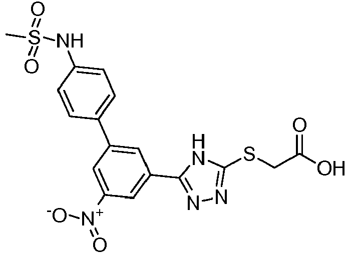 |
| **48** | 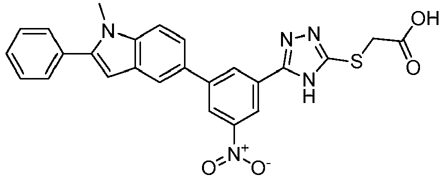 |
| **49** | 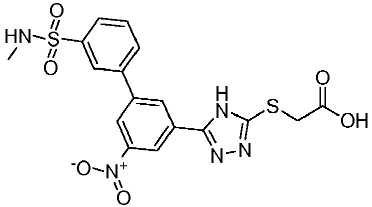 |
| **50** | 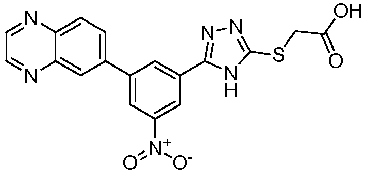 |
| **51** | 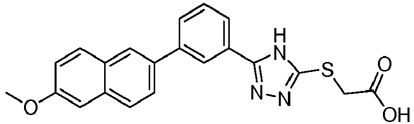 |
| **52** | 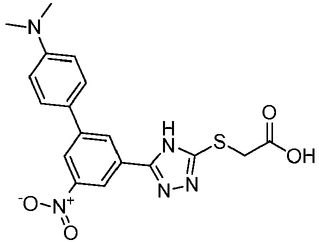 |
| **53** | 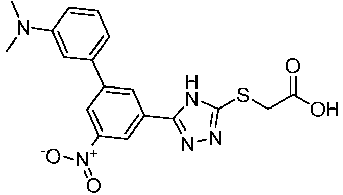 |
| **54** | 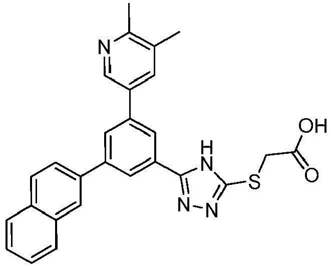 |
| **55** | 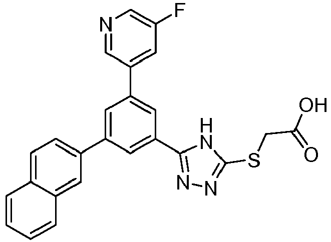 |
| **56** | 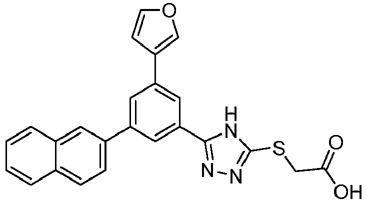 |
| **57** | 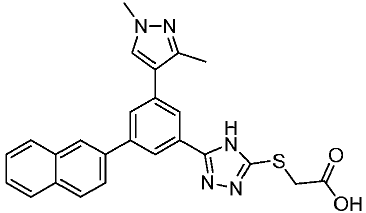 |
| **58** | 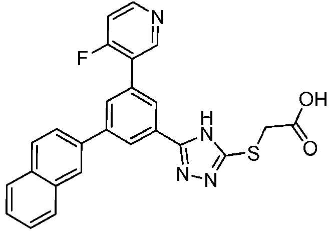 |
| **59** | 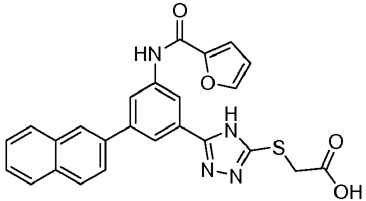 |
| **60** | 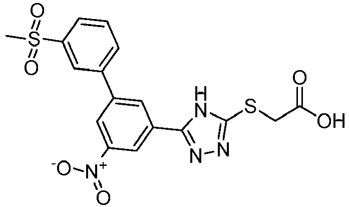 |
| **61** | 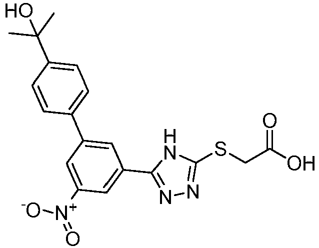 |
| **62** | 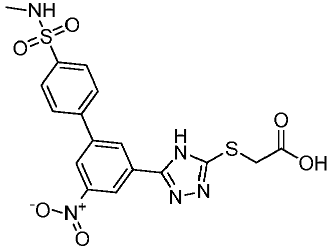 |
| **63** | 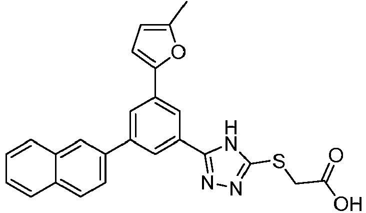 |
| **64** | 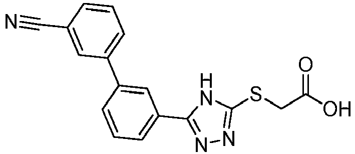 |
| **65** | 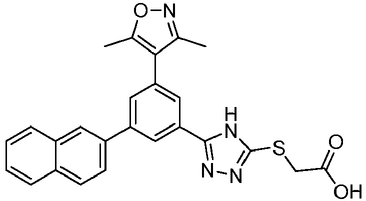 |
| **66** | 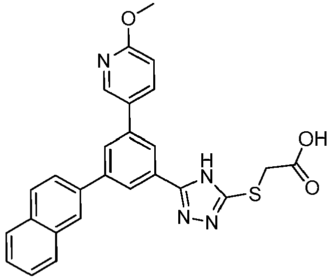 |
| **67** | 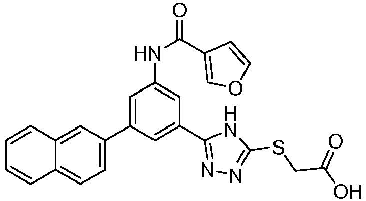 |
| **68** | 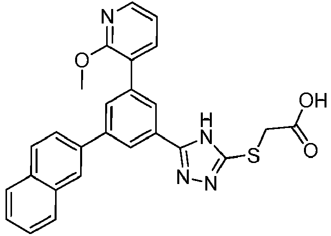 |
| **69** | 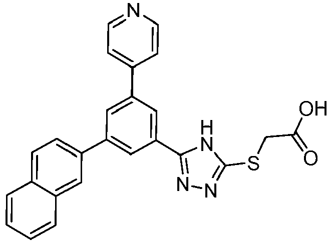 |
| **70** | 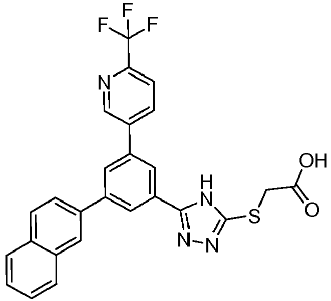 |
| **71** | 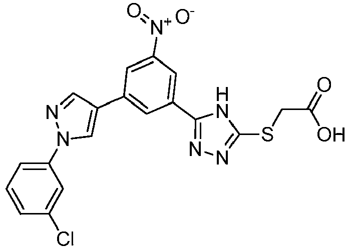 |
| **72** | 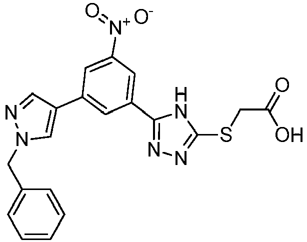 |
| **73** | 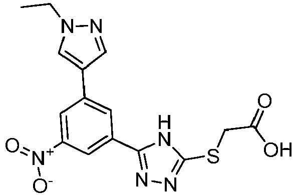 |
| **74** | 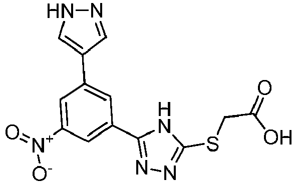 |
| **75** | 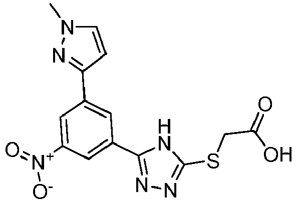 |
| **76** | 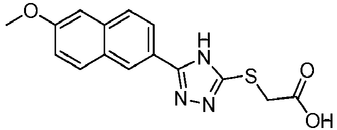 |
| **77** | 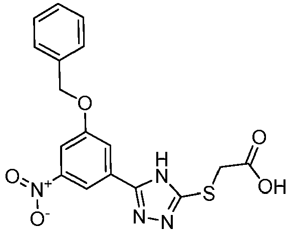 |
| **78** | 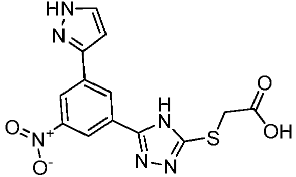 |
| **79** | 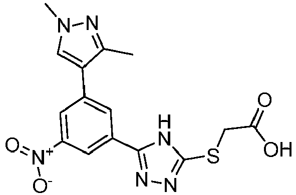 |
| **80** | 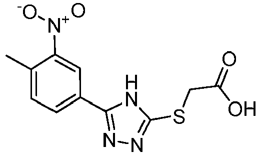 |
| **81** | 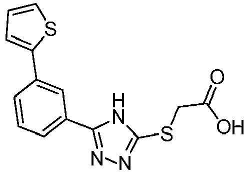 |
| **82** | 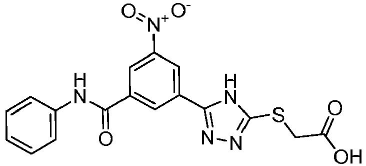 |
| **83** | 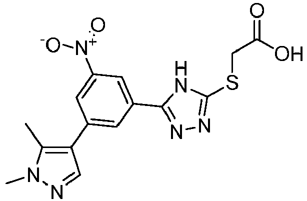 |
| **84** | 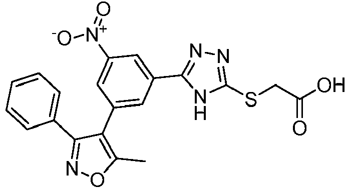 |
| **85** | 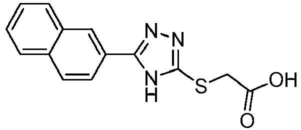 |
| **86** | 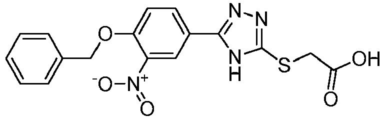 |
| **87** | 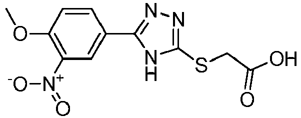 |
| **88** | 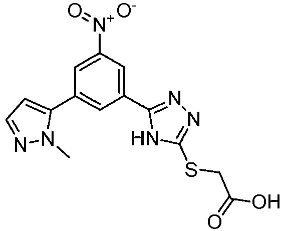 |
| **89** | 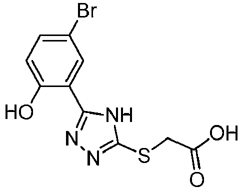 |
| **90** | 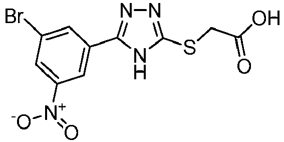 |
| **91** | 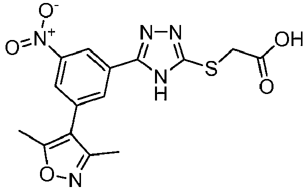 |
| **92** | 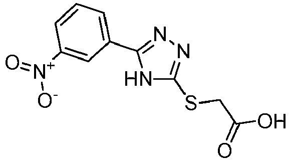 |
| **93** | 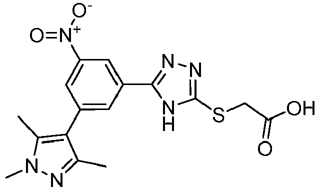 |
| **94** | 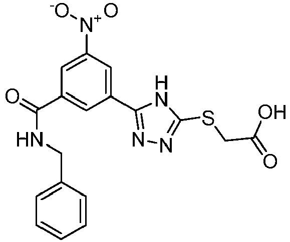 |
| **95** | 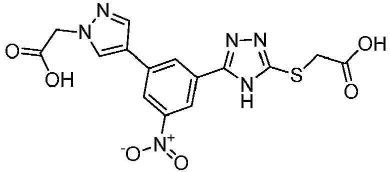 |
| **96** | 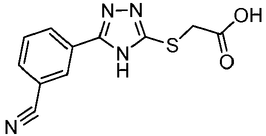 |
| **97** | 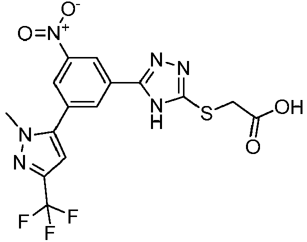 |
| **98** | 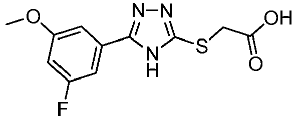 |
| **99** | 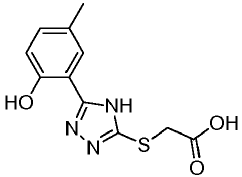 |
| **100** | 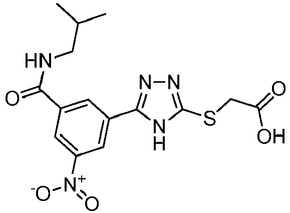 |
| **101** | 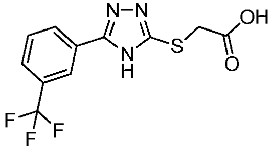 |
| **102** | 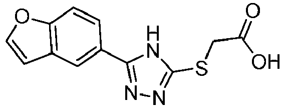 |
| **103** | 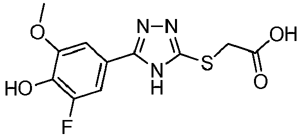 |
| **104** | 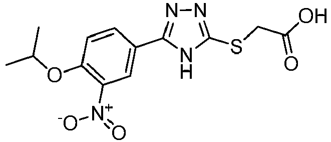 |
| **105** | 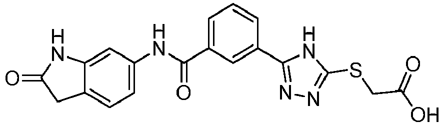 |
| **106** | 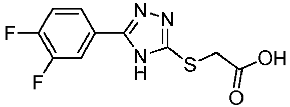 |
| **107** | 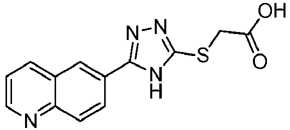 |
| **108** | 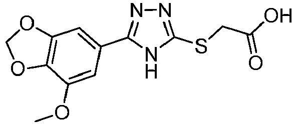 |
| **109** | 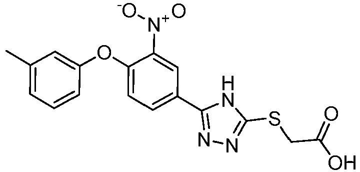 |
| **110** | 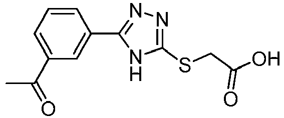 |
| **111** | 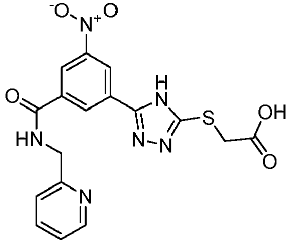 |
| **112** | 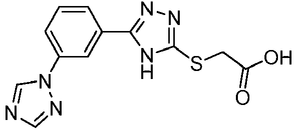 |
| **113** | 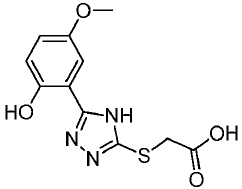 |
| **114** | 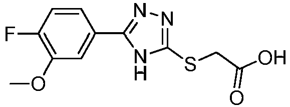 |
| **115** | 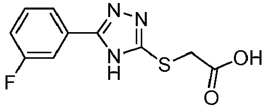 |
| **116** | 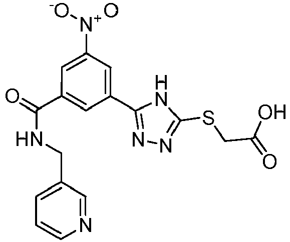 |
| **117** | 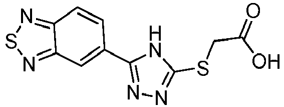 |
| **118** | 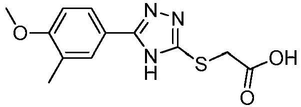 |
| **119** | 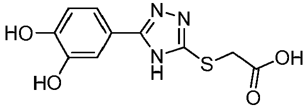 |
| **120** | 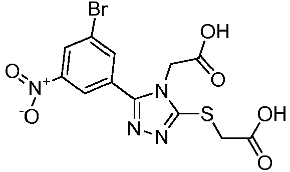 |
| **121** | 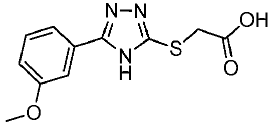 |
| **122** | 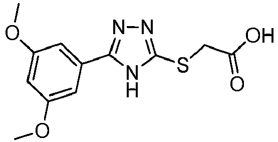 |
| **123** | 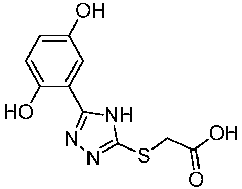 |
| **124** | 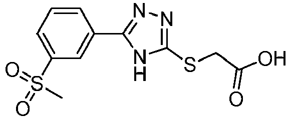 |
| **125** | 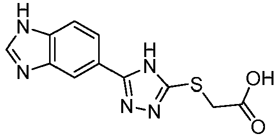 |
| **126** | 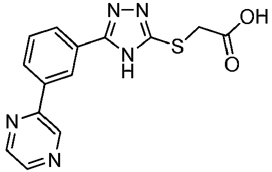 |
| **127** | 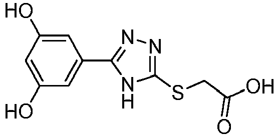 |
| **128** | 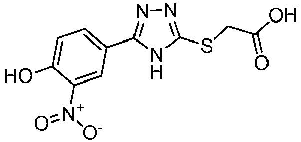 |
| **129** | 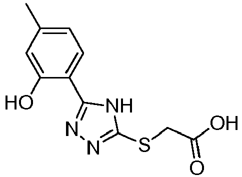 |
| **130** | 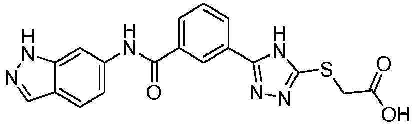 |
| **131** | 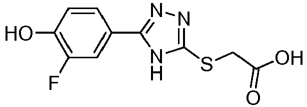 |
| **132** | 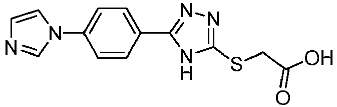 |
| **133** | 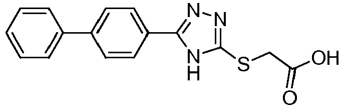 |
| **134** | 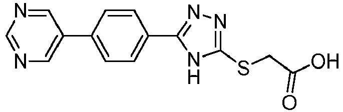 |
| **135** | 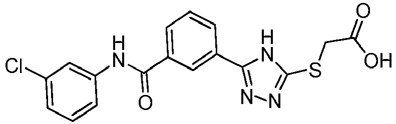 |
| **136** | 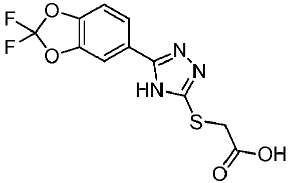 |
| **137** | 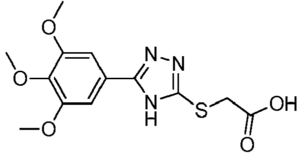 |
| **138** | 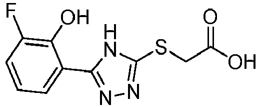 |
| **139** | 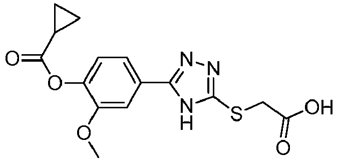 |
| **140** | 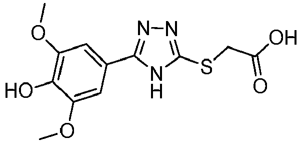 |
| **141** | 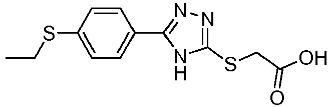 |
| **142** | 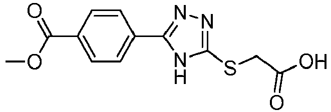 |
| **143** | 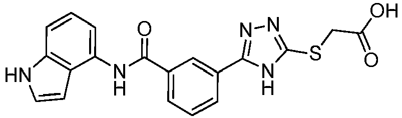 |
| **144** | 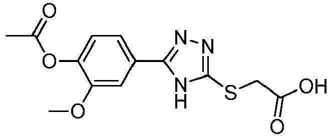 |
| **145** | 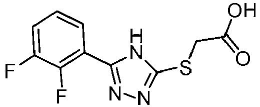 |
| **146** | 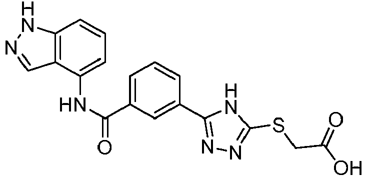 |
| **147** | 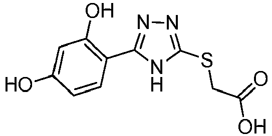 |
| **148** | 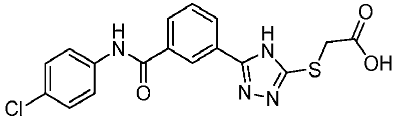 |
| **149** | 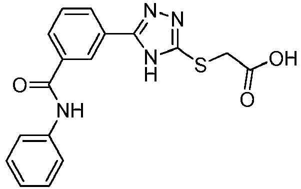 |
| **150** | 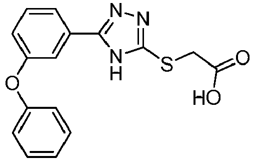 |
| **151** | 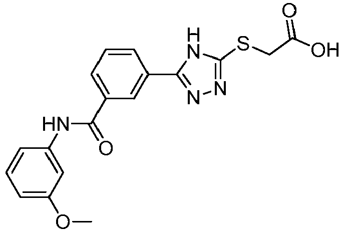 |
| **152** | 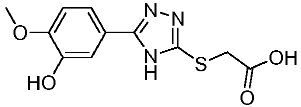 |
| **153** | 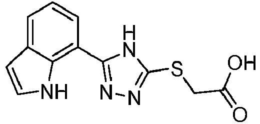 |
| **154** | 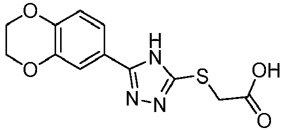 |
| **155** | 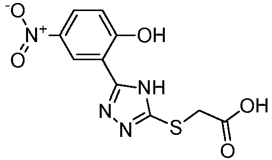 |
| **156** | 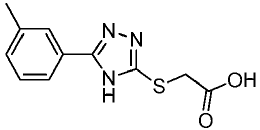 |
| **157** | 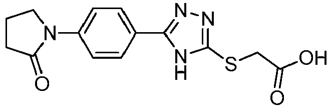 |
| **158** | 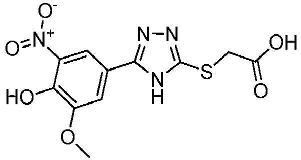 |
| **159** | 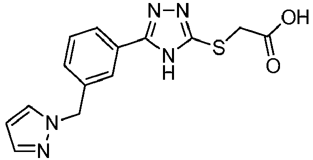 |
| **160** | 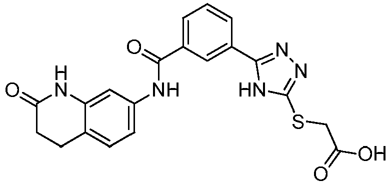 |
| **161** | 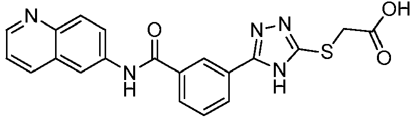 |
| **162** | 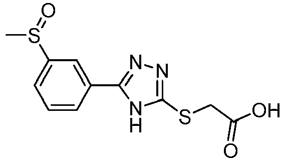 |
| **163** | 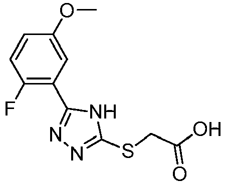 |
| **164** | 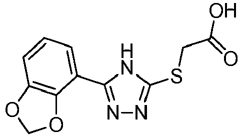 |
| **165** | 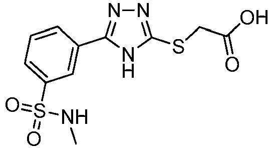 |
| **166** | 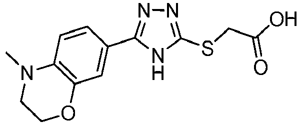 |
| **167** | 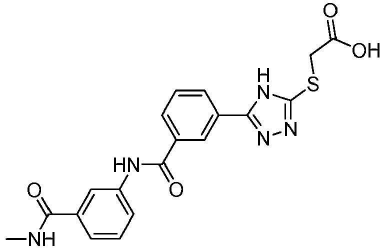 |
| **168** | 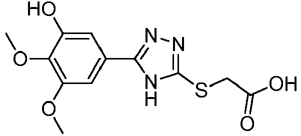 |
| **169** | 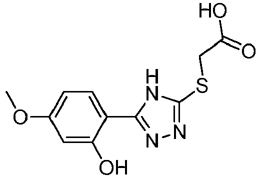 |
| **170** | 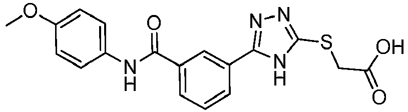 |
| **171** | 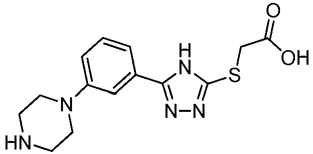 |
| **172** | 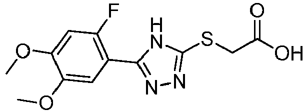 |
| **173** | 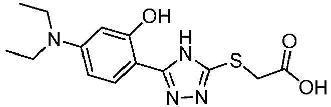 |
| **174** | 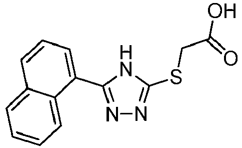 |
| **175** | 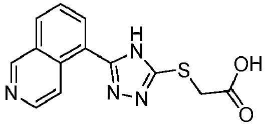 |
| **176** | 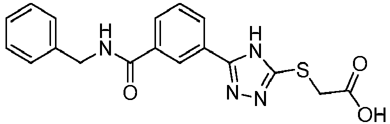 |
| **177** | 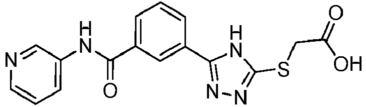 |
| **178** | 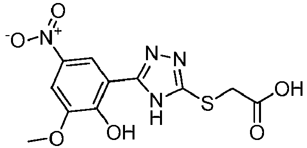 |
| **179** | 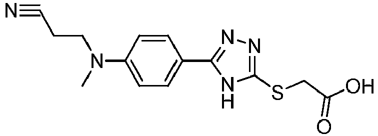 |
| **180** | 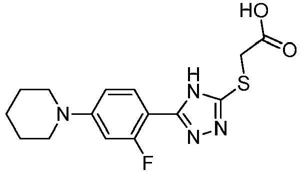 |
| **181** | 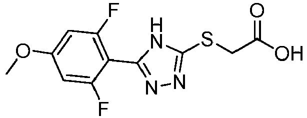 |
| **182** | 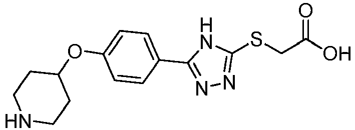 |
| **183** | 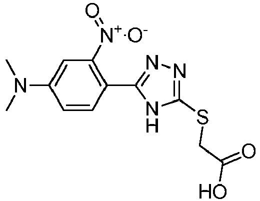 |
| **184** | 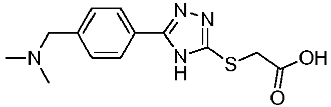 |
| **185** | 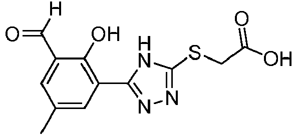 |
| **186** | 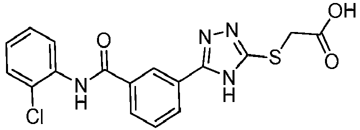 |
| **187** | 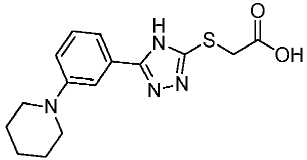 |
| **188** | 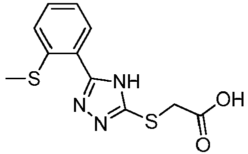 |
| **189** | 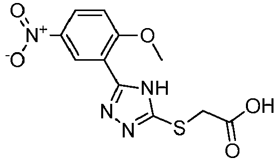 |
| **190** | 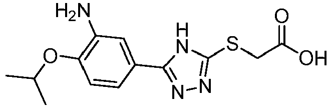 |
| **191** | 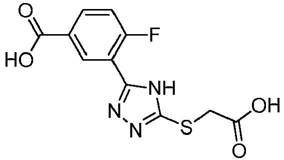 |
| **192** | 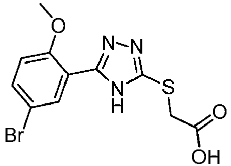 |
| **193** | 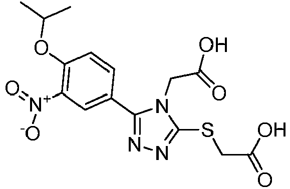 |
| **194** | 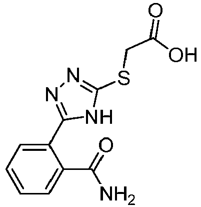 |
| **195** | 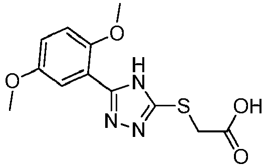 |
| **196** | 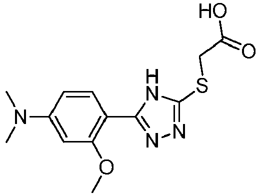 |
| **197** | 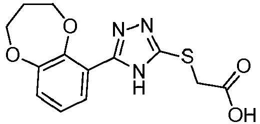 |
| **198** | 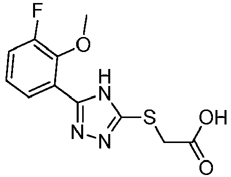 |
| **199** | 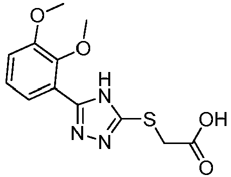 |
| **200** | 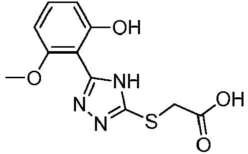 |
| **201** | 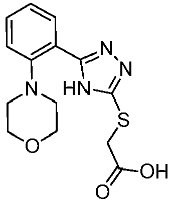 |
| **202** | 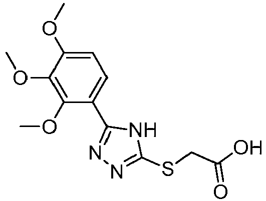 |
| **203** | 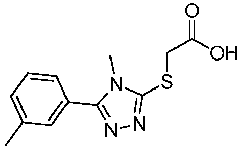 |
| **204** | 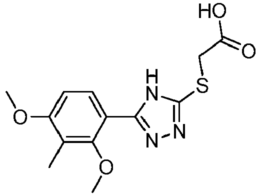 |
| **205** | 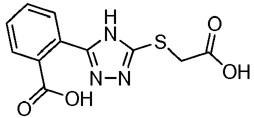 |
| **206** | 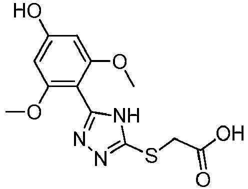 |
| **207** | 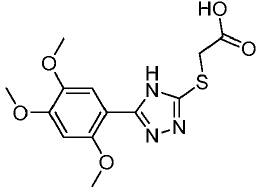 |
| **208** | 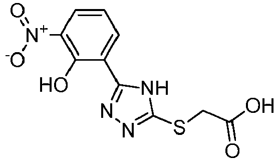 |
| **209** | 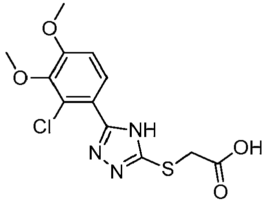 |
| **210** | 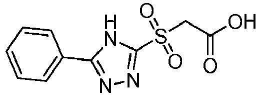 |
| **211** | 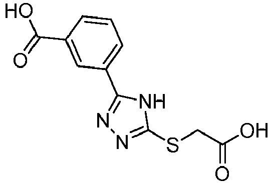 |
| **212** | 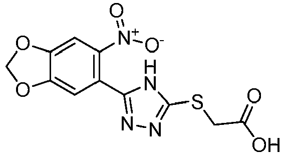 |
| **213** | 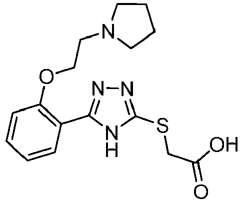 |
| **214** | 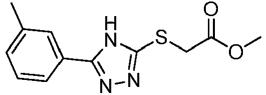 |
| **215** | 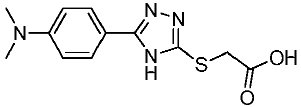 |
| **216** | 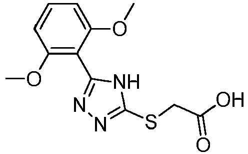 |
| **217** | 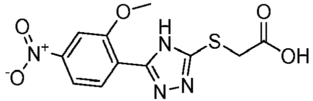 |
| **218** | 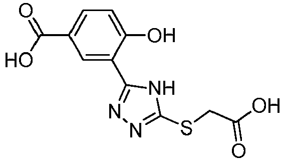 |
| **219** | 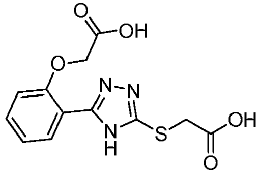 |
| **220** | 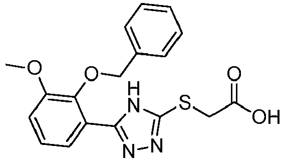 |
| **221** | 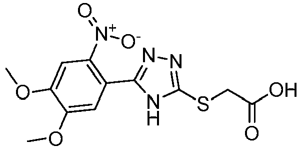 |
| **222** | |
| **223** | |
| **224** | |
| **225** | |
| **226** | |
| **227** | 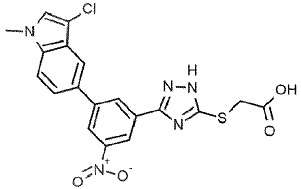 |
| **228** | 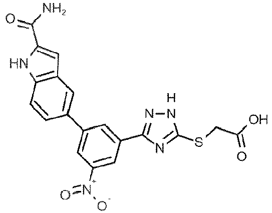 |
| **229** | 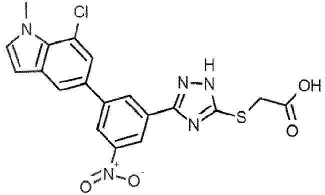 |
| **230** | 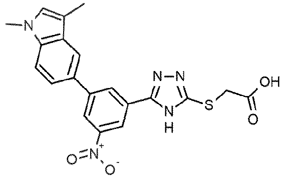 |
| **231** | 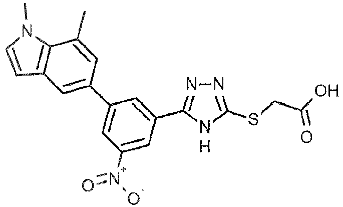 |
| **232** | 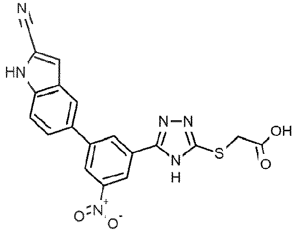 |
| **233** | 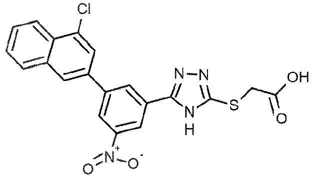 |
| **234** | 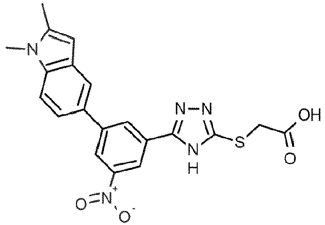 |
| **235** | 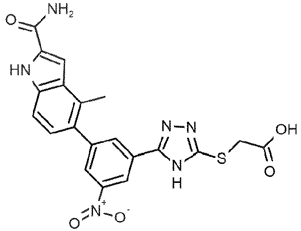 |
| **236** | 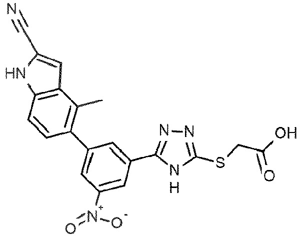 |
| **237** | 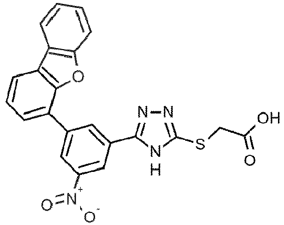 |
| **238** | 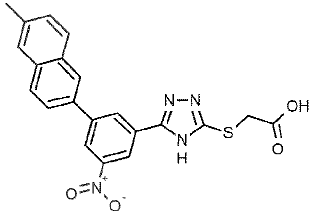 |
| **239** | 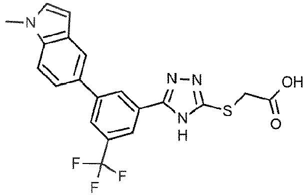 |
| **240** | 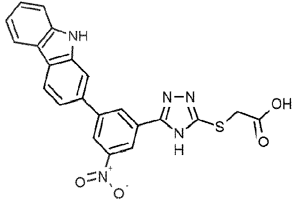 |
| **241** | 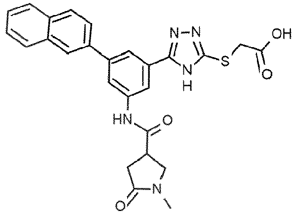 |
| **242** | 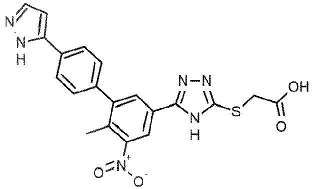 |
| **243** | 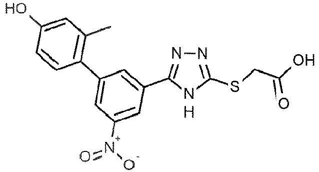 |
| **244** | 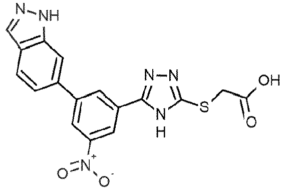 |
| **245** | 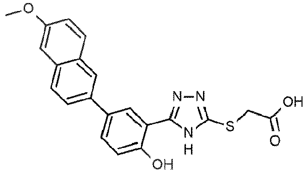 |

An inhibitor provided herein, including a pharmaceutically acceptable salt thereof, can be purchased commercially or prepared using known organic synthesis techniques. See, for example, Examples 1 and 2 and WO 2013/063458.

A reaction for preparing an inhibitor provided herein can be carried out in suitable solvents that can be readily selected by one of skill in the art of organic synthesis. Suitable solvents can be substantially non-reactive with the starting materials (reactants), the intermediates, or products at the temperatures at which the reactions are carried out, e.g., temperatures which can range from the solvent's freezing temperature to the solvent's boiling temperature. A given reaction can be carried out in one solvent or a mixture of more than one solvent. Depending on the particular reaction step, suitable solvents for a particular reaction step can be selected by the skilled artisan.

Preparation of an inhibitor can involve the protection and deprotection of various chemical groups. The need for protection and deprotection, and the selection of appropriate protecting groups, can be readily determined by one skilled in the art. The chemistry of protecting groups can be found, for example, in Protecting Group Chemistry, 1st Ed., Oxford University Press, 2000; and March's Advanced Organic chemistry: Reactions, Mechanisms, and Structure, 5th Ed., Wiley-Interscience Publication, 2001.

### Pharmaceutically Acceptable Salts and Compositions

This document also provides pharmaceutically acceptable salts of the inhibitors provided herein. Examples of pharmaceutically acceptable salts of the inhibitors provided herein include acid addition salts and base salts of the inhibitors.

Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, hydrogen phosphate, isethionate, D- and L-lactate, malate, maleate, malonate, mesylate, methylsulphate, 2-napsylate, nicotinate, nitrate, orotate, oxalate, palmitate, pamoate, phosphate/hydrogen, phosphate/phosphate dihydrogen, pyroglutamate, saccharate, stearate, succinate, tannate, D- and L-tartrate, 1-hydroxy-2-naphthoate tosylate, and xinafoate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine, and zinc salts.

Hemisalts of acids and bases may also be formed, for example, hemisulphate and hemicalcium salts.

An inhibitor provided herein intended for pharmaceutical use may be administered as a crystalline or amorphous product. In some cases, such a product may be obtained, for example, as a solid plug, powder, or film by methods such as precipitation, crystallization, freeze drying, spray drying, or evaporative drying. Microwave or radio frequency drying may be used for this purpose.

An inhibitor may be administered by any route, including oral, rectal, sublingual, and parenteral administration. Parenteral administration includes, for example, intravenous, intramuscular, intraarterial, intraperitoneal, intranasal, intravaginal, intravesical (e.g., to the bladder), intradermal, transdermal, topical or subcutaneous administration. Also contemplated is the installation of an inhibitor in the body of the patient in a controlled formulation, with systemic or local release of an inhibitor to occur at a later time. For example, an inhibitor can be localized in a depot for controlled release to the circulation, or for release to a local site. Advantageously, an inhibitor can be administered in the form of a pharmaceutical composition.

An inhibitor may be administered alone or in combination with one or more other inhibitors provided herein or in combination with one or more other drugs (or as any combination thereof). Generally, an inhibitor will be administered as a formulation in association with one or more pharmaceutically acceptable excipients. The term "excipient" is used herein to describe any ingredient other than an inhibitor(s) provided herein. The choice of excipient will to a large extent depend on factors such as the particular mode of administration, the effect of the excipient on solubility and stability, and the nature of the dosage form.

Non-limiting examples of pharmaceutical excipients suitable for administration of the inhibitors provided herein include any such carriers known to those skilled in the art to be suitable for the particular mode of administration. Pharmaceutically acceptable excipients include, but are not limited to, ion exchangers, alumina, aluminum stearate, lecithin, self-emulsifying drug delivery systems (SEDDS) such as d-α-tocopherol polyethylene glycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium-chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, and wool fat. Cyclodextrins such as α-, β, and γ-cyclodextrin, or chemically modified derivatives such as hydroxyalkylcyclodextrins, including 2- and 3-hydroxypropyl-b-cyclodextrins, or other solubilized derivatives can also be advantageously used to enhance delivery of an inhibitor provided herein. In some embodiments, the excipient is a physiologically acceptable saline solution.

A pharmaceutical composition can be, in one embodiment, formulated into suitable pharmaceutical preparations such as solutions, suspensions, tablets, dispersible tablets, pills, capsules, powders, sustained release formulations or elixirs, for oral administration or in sterile solutions or suspensions for parenteral administration, as well as transdermal ointments, creams, gels, and patch preparations and dry powder inhalers (see, e.g., Ansel Introduction to Pharmaceutical Dosage Forms, Fourth Edition 1985, 126).

The concentration of an inhibitor in a pharmaceutical composition will depend on absorption, inactivation, and excretion rates of the inhibitor, the physicochemical characteristics of the inhibitor, the dosage schedule, and amount administered as well as other factors known to those of skill in the art.

The pharmaceutical composition may be administered at once, or may be divided into a number of smaller doses to be administered at intervals of time. It is understood that the precise dosage and duration of treatment is a function of the disease being treated and may be determined empirically using known testing protocols or by extrapolation from *in vivo* or *in vitro* test data. It is to be noted that concentrations and dosage values may also vary with the severity of the condition to be alleviated. It is to be further understood that for any particular patient, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

The pharmaceutical compositions are provided for administration to humans and animals in unit dosage forms, such as tablets, capsules, pills, powders, granules, sterile parenteral solutions or suspensions, and oral solutions or suspensions, and oil-water emulsions containing suitable quantities of the inhibitors. The pharmaceutically therapeutically active inhibitors are, in one embodiment, formulated and administered in unit-dosage forms or multiple-dosage forms. Unit-dose forms as used herein refers to physically discrete units suitable for human and animal patients and packaged individually as is known in the art. Each unit-dose contains a predetermined quantity of the therapeutically active inhibitor sufficient to produce the desired therapeutic effect, in association with the required pharmaceutical carrier, vehicle or diluent. Examples of unit-dose forms include ampoules and syringes and individually packaged tablets or capsules. Unit-dose forms may be administered in fractions or multiples thereof. A multiple-dose form is a plurality of identical unit-dosage forms packaged in a single container to be administered in segregated unit-dose form. Examples of multiple-dose forms include vials, bottles of tablets or capsules or bottles of pints or gallons. Hence, multiple dose form is a multiple of unit-doses which are not segregated in packaging.

Liquid pharmaceutically administrable compositions can, for example, be prepared by dissolving, dispersing, or otherwise mixing an inhibitor as provided herein and optional pharmaceutical adjuvants in a carrier, such as, for example, water, saline, aqueous dextrose, glycerol, glycols, ethanol, and the like, to thereby form a solution or suspension. If desired, a pharmaceutical composition to be administered may also contain minor amounts of nontoxic auxiliary substances such as wetting agents, emulsifying agents, solubilizing agents, pH buffering agents and the like, for example, acetate, sodium citrate, cyclodextrine derivatives, sorbitan monolaurate, triethanolamine sodium acetate, triethanolamine oleate, and other such agents.

Dosage forms or compositions containing an inhibitor provided herein in the range of 0.005% to 100% with the balance made up from non-toxic carrier may be prepared. Methods for preparation of these compositions are known to those skilled in the art. The contemplated compositions may contain 0.001%-100% active ingredient, in one embodiment 0.1-95%, in another embodiment 75-85%.

Pharmaceutical compositions suitable for the delivery of inhibitor provided herein and methods for their preparation will be readily apparent to those skilled in the art. Such compositions and methods for their preparation may be found, for example, in Remington's Pharmaceutical Sciences, 19th Edition (Mack Publishing Company, 1995).

### Methods of Use

This document also provides methods and materials for using inhibitors of G protein couple receptor 6 kinase (GRK6) polypeptides. In some cases, an inhibitor provided herein may be used to treat any disease or disorder which involves the inhibition of a GRK6 polypeptide or a GRK6 polypeptide pathway. For example, a GRK6 polypeptidecan be inhibited in a patient by administering a therapeutically effective amount of an inhibitor provided herein. In addition, a GRK6 polypeptide can be inhibited in a cell by contacting the cell with an effective amount of an inhibitor provided herein.

An inhibitor provided herein can have an IC₅₀ value in a GRK6 polypeptide inhibition assay ranging from about 0.1 µM to greater than about 20 µM. For example, see Example 3.

Diseases and disorders which involve overexpression or over-activation of a GRK6 polypeptide can include, for example, hematological malignancies, inflammation diseases, and autoimmune disorders.

Hematological malignancies that may be treated by the inhibitors, compositions and methods described herein include, but are not limited to, cancers of the bone marrow, blood, and lymph nodes. For example, hematological malignancies can include, for example, malignant lymphoma (reticulum cell sarcoma), multiple myeloma, acute myeloid leukemia, chronic myeloid leukemia, acute lymphoblastic leukemia, chronic lymphocytic leukemia, myeloproliferative diseases, multiple myeloma, myelodysplastic syndrome, Hodgkin's lymphoma, non-Hodgkin's lymphoma (malignant lymphoma), harry cell leukemia, and Waldenström's macroglobulinemia.

Hematological malignancies may be solid tumors that may or may not be metastatic. Cancers may also occur, as in leukemia, as a diffuse tissue. Thus, the term "tumor cell", as provided herein, includes a cell afflicted by any one of the above identified disorders.

In some embodiments, the hematological malignancy is a B cell cancer. For example, the B cell cancer is a B cell Non-Hodgkin Lymphoma. B cell Non-Hodgkin's Lymphomas can include mediastinal large B-cell lymphoma, lymphoblastic B cell lymphoma, Waldenstrom's macroglobulinaemia, and follicular lymphoma. Thus, in some embodiments, the B cell Non-Hodgkin's Lymphoma is small lymphocytic lymphoma (SLL), a mantle cell lymphoma, a Burkitt's lymphoma, a follicle centre cell lymphoma, a follicular lymphoma, a Burkitt-like lymphoma, a marginal zone B-cell lymphoma (MZBCL), a nodal marginal zone B cell lymphoma, an extra-nodal marginal zone B cell lymphoma, a splenic marginal zone B cell lymphoma, a lymphoplasmacytic lymphoma, or a diffuse large B cell lymphoma. In some embodiments, the B cell cancer is myeloma.

In some embodiments, the B cell cancer is a B cell acute lymphocytic leukemia (B-ALL), a precursor B cell acute lymphocytic leukemia (B-ALL), a B cell chronic lymphocytic leukemia (B-CLL), a precursor B-lymphoblastic leukaemia, a precursor B-lymphoblastic lymphoma, a small lymphocytic lymphoma, a B cell prolymphocytic leukemia, an undifferentiated B cell lymphoma, a hairy cell leukemia, a mediastinal large B-cell lymphoma, a plasma cell myeloma, a plasmacytoma, a primary effusive lymphoma, a Burkitt's cell leukemia, or a B cell diffuse mixed lymphoma.

An inhibitor provided herein can also be administered in combination with existing methods of treating hematological malignancies, for example by chemotherapy, irradiation, or surgery. Thus, there is further provided a composition for use in a method of treating hematological malignancies comprising administering an effective amount of an inhibitor described herein, or a pharmaceutically acceptable salt form thereof, to a patient, wherein a therapeutically effective amount of one or more additional cancer chemotherapeutic agents are administered to the patient.

The inhibitors provided herein are also useful in treating an inflammatory disease in a patient. Examples of inflammatory diseases treated by an inhibitor provided herein include, but are not limited to, general inflammatory diseases such as encephalitis, inflammatory eye disease, otitis, pharyngitis, pneumonia, gastritis, enteritis, hepatitis, pancreatitis, nephritis, cystitis, urethritis, endometritis, vaginitis, arthritis, and peripheral neuritis, and further include inflammatory diseases that secondarily cause inflammation, such as malignant tumor, infectious diseases, allergic diseases, autoimmune diseases (such as rheumatism, systemic lupus erythematosus, and sarcoidosis), ischemic diseases (such as myocardial infarction and cerebral infarction), metabolic diseases (such as diabetes and gout), injury, scald, chemical corrosion, and neurodegenerative diseases (such as Alzheimer's disease).

For example, an inhibitor provided herein can be used to treat an autoimmune disease or disorder. The term "autoimmune" refers to the process by which immune system components such as antibodies or lymphocytes attack or harm molecules, cells, or tissues of the organism producing them. The term "autoimmune disorders" refers to diseases where damage, such as tissue damage, or pathogenesis is, at least partially, a result of an autoimmune process.

In some embodiments, suppression of the immune response is useful in the treatment of patients suffering from autoimmune diseases as well as adverse immune reactions associated with organ transplantations.

Autoimmune diseases include allograft rejection, autoimmune thyroid diseases (such as Graves' disease and Hashimoto's thyroiditis), autoimmune uveoretinitis, giant cell arteritis, inflammatory bowel diseases (including Crohn's disease, ulcerative colitis, regional enteritis, granulomatous enteritis, distal ileitis, regional ileitis, and terminal ileitis), insulin-dependent diabetes mellitus, multiple sclerosis, pernicious anemia, psoriasis, rheumatoid arthritis, sarcoidosis, scleroderma, and systemic lupus erythematosus.

Inhibitors provided herein are effective to inhibit a GRK6 polypeptide in a cell, for example, in a cancer cell (e.g., in a cell from a hematological malignancy). Therefore there is also provided a method of inhibiting a GRK6 polypeptide in a cell comprising contacting the cell with an effective amount of an inhibitor provided herein, or a pharmaceutically acceptable salt form thereof. The method may be performed by contacting the cell with an inhibitor as described herein, or a pharmaceutically acceptable salt form thereof, *in vitro,* thereby inhibiting a GRK6 polypeptide *in vitro.* Uses of such an *in vitro* method of inhibiting a GRK6 polypeptide include, but are not limited to use in a screening assay (for example, wherein an inhibitor described herein is used as a positive control or standard compared to compounds of unknown activity or potency in inhibiting a GRK6 polypeptide).

### EXAMPLES

### Example 1: Preparation of 2-((3-(3-nitro-5-(2-phenyl-1H-indol-5-yl)phenyl)-1H-1,2,4-triazol-5-yl)thio)acetic acid (33)

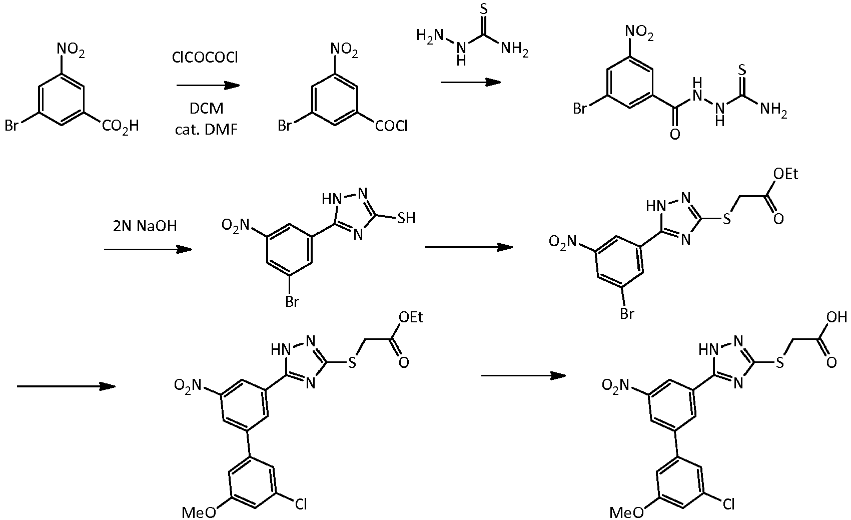

Step 1: To a stirred suspension of 3-bromo-5-nitrobenzoic acid (15.46 g) in dry dichloromethane (100 mL), oxalyl chloride (11.0 mL) was added in portions over 10 min at 0 °C. Upon completion, DMF (20 µL) was added and the ice bath was removed. When the solution became homogeneous and bubbling ceased, stirring was stopped and the solvent and excess reagent were removed under reduced pressure. The crude acid chloride was used without purification in the next reaction.

Step 2: Thiosemicarbazide (7.4 g) was suspended in dry pyridine (60 mL). The reaction mixture was cooled over ice and the crude acid chloride (Step 1) dissolved in THF / pyridine was slowly added and the reaction was set to stir overnight. The solvent was then removed and the crude residue was washed with water and dried.

Step 3: The crude aroylthiosemicarbazide (Step 2) was treated with 2 M sodium hydroxide at 85 °C overnight. After cooling, the solution was acidified with hydrochloric acid and the precipitate was filtered, washed with water, and dried. Step 4: The crude product of Step 3 was dissolved in dry DMF (100 mL) and treated with triethylamine (2 eq.) and ethyl bromoacetate (1 eq.). The mixture was stirred at room temperature for 4 h and then briefly heated to 50 °C. The reaction mixture was poured over ice and extracted with ethyl acetate. The organic layer was subsequently washed with water and evaporated to dryness to yield ethyl 2-((5-(3-bromo-5-nitrophenyl)-1H-1,2,4-triazol-3-yl)thio)acetate.

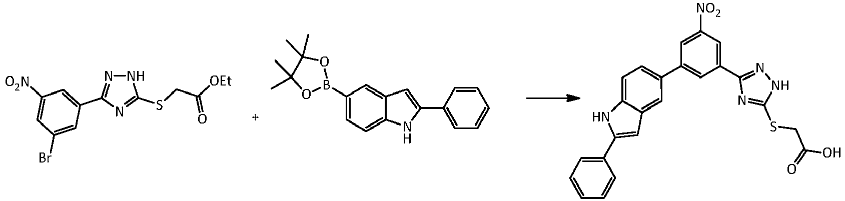

Step 5: Ethyl 2-((5-(3-bromo-5-nitrophenyl)-1H-1,2,4-triazol-3-yl)thio)acetate (19 mg), 2-phenyl-5-(4,4,5,5-tetramethyl-1,3,2-dioxaborolan-2-yl)-1H-indole (2 eq), and 1,1'-bis(diphenylphosphino) ferrocene (10 mole %) were added to a mixture of acetonitrile / water (3:1). The reaction mixture was heated at 120 °C in a microwave reactor for 30 minutes. After heating, the reaction mixture was poured into ethyl acetate and washed with water. The organic phase was collected and evaporated to dryness. The residue was purified by HPLC to afford a yellow solid. The solid was treated with a 1:1 mixture of dichloromethane and trifluoroacetic acid to give the title compound.

### Example 2: Preparation of additional compounds

The following examples of 2-((5-biaryl-1H-1,2,4-triazol-3-yl)thio)acetic acids (Table 2) can be prepared as described above for 2-((3-(3-nitro-5-(2-phenyl-1H-indol-5-yl)phenyl)-1H-1,2,4-triazol-5-yl)thio)acetic acid (Example 1) by substituting the appropriate starting materials. Only the compounds whose formula is in accordance with Formula I as claimed in claim 1 are part of the invention.

**Table 2.**

| **#** | **Compound Structure** | **MW** |
|---|---|---|
| **1** | 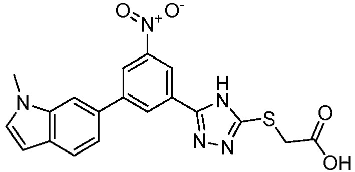 | 409.42 |
| **2** | 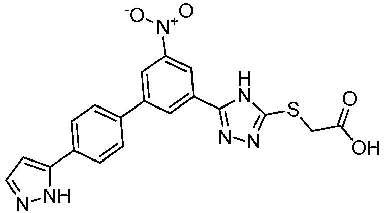 | 422.42 |
| **3** | 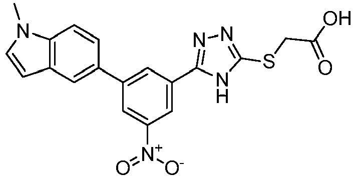 | 409.42 |
| **4** | 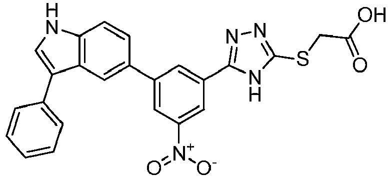 | 471.49 |
| **5** | 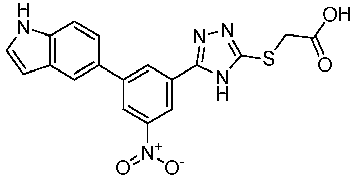 | 395.39 |
| **6** | 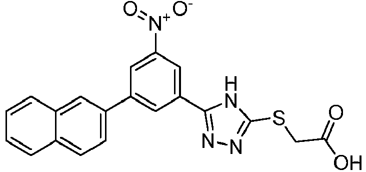 | 406.42 |
| **7** | 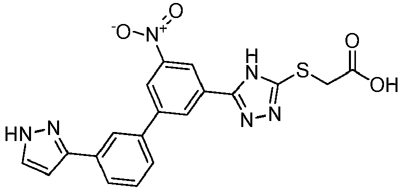 | 422.42 |
| **8** | 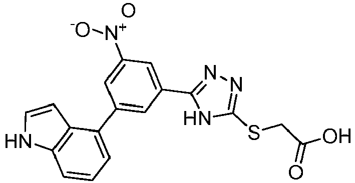 | 395.39 |
| **9** | 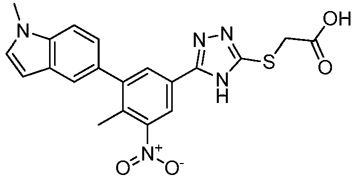 | 423.45 |
| **10** | 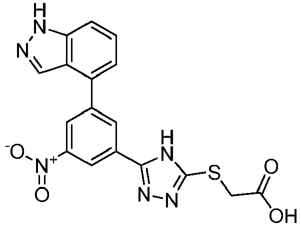 | 396.38 |
| **11** | 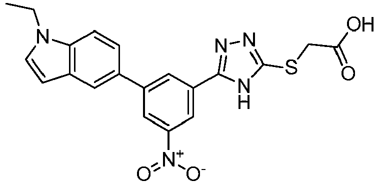 | 423.45 |
| **12** | 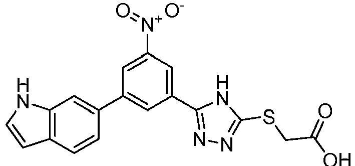 | 395.39 |
| **13** | 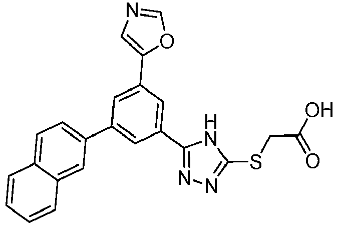 | 428.47 |
| **14** | 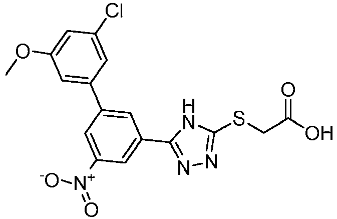 | 420.84 |
| **15** | 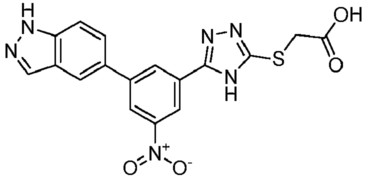 | 396.38 |
| **16** | 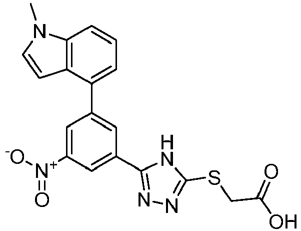 | 409.42 |
| **17** | 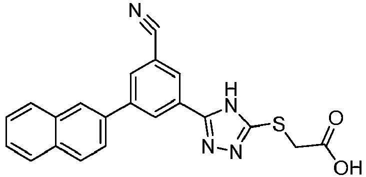 | 386.43 |
| **18** | 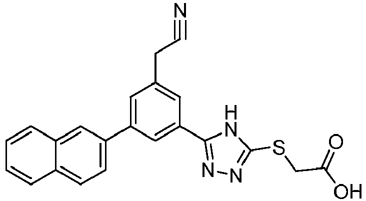 | 400.46 |
| **19** | 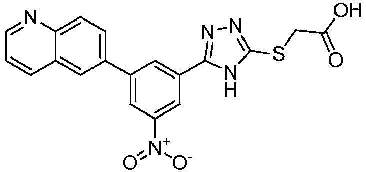 | 407.40 |
| **20** | 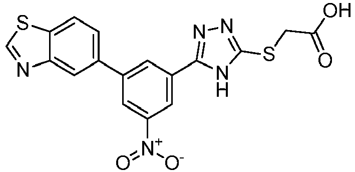 | 413.43 |
| **21** | 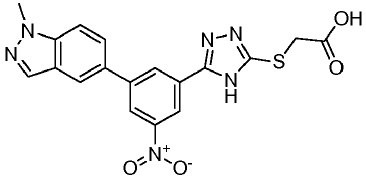 | 410.41 |
| **22** | 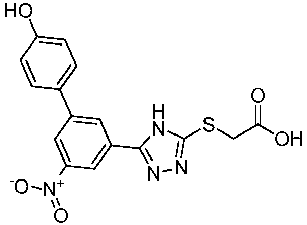 | 372.36 |
| **23** | 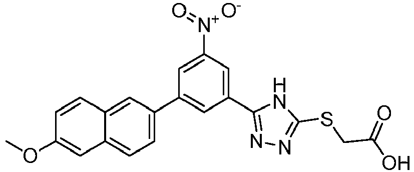 | 436.44 |
| **24** | 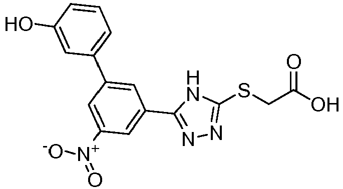 | 372.36 |
| **25** | 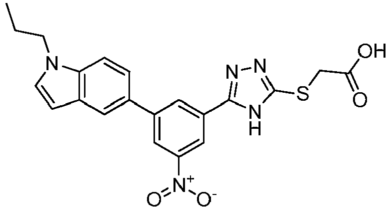 | 437.47 |
| **26** | 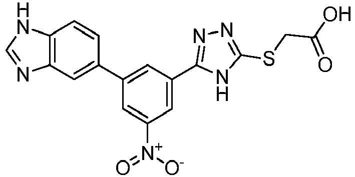 | 396.38 |
| **27** | 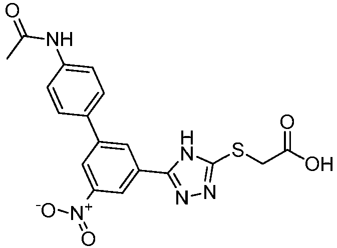 | 413.41 |
| **28** | 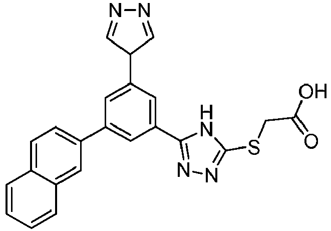 | 427.48 |
| **29** | 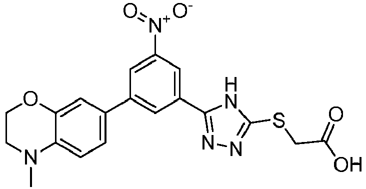 | 427.44 |
| **30** | 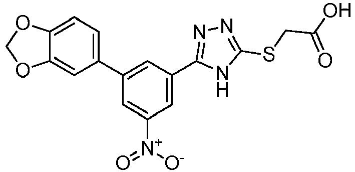 | 400.37 |
| **31** | 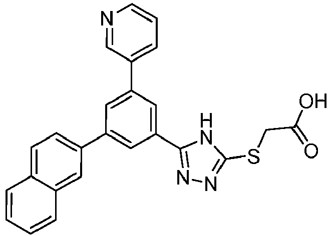 | 438.50 |
| **32** | 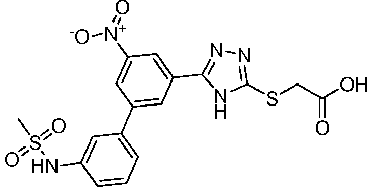 | 449.46 |
| **33** | 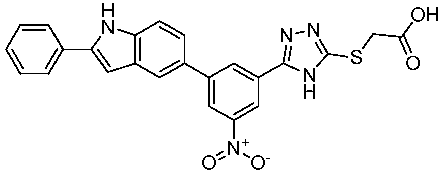 | 471.49 |
| **34** | 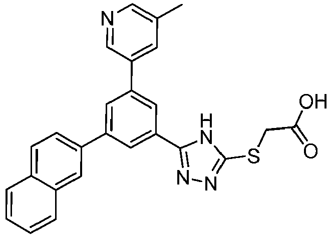 | 452.53 |
| **35** | 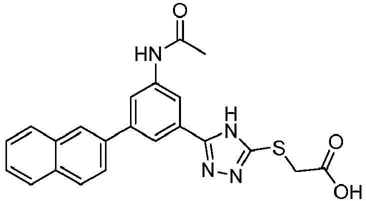 | 418.47 |
| **36** | 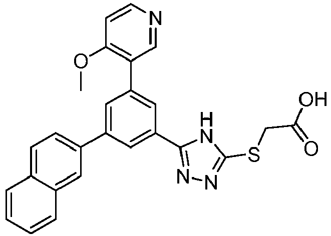 | 468.53 |
| **37** | 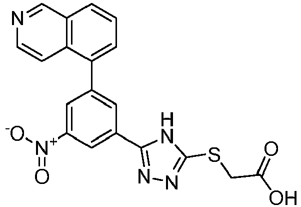 | 407.40 |
| **38** | 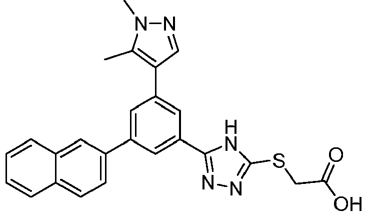 | 455.53 |
| **39** | 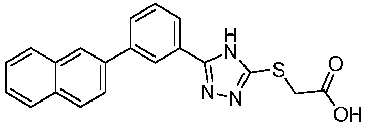 | 361.42 |
| **40** | 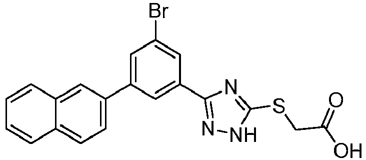 | 440.32 |
| **41** | 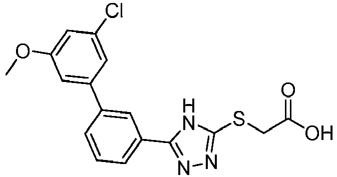 | 375.84 |
| **42** | 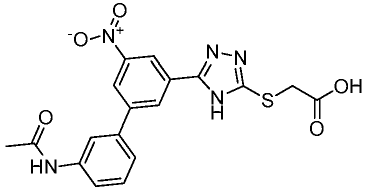 | 413.41 |
| **43** | 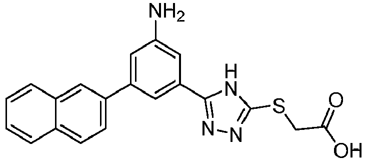 | 376.43 |
| **44** | 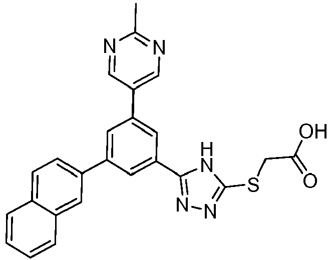 | 453.52 |
| **45** | 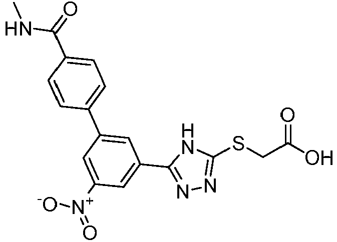 | 413.41 |
| **46** | 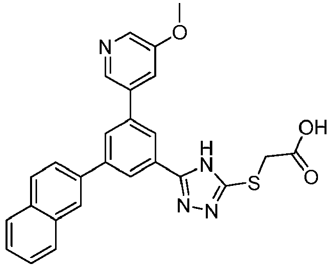 | 468.53 |
| **47** | 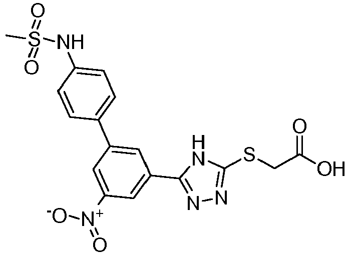 | 449.46 |
| **48** | 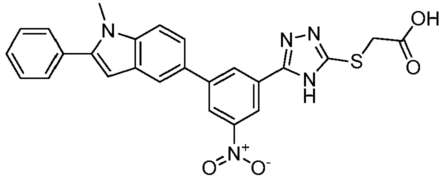 | 485.52 |
| **49** | 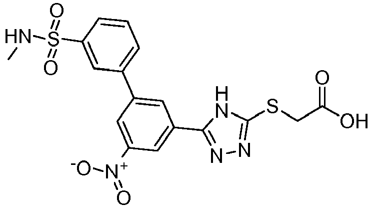 | 449.46 |
| **50** | 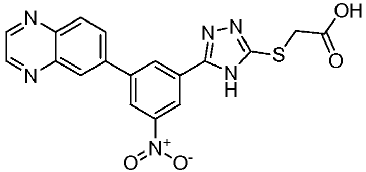 | 408.39 |
| **51** | 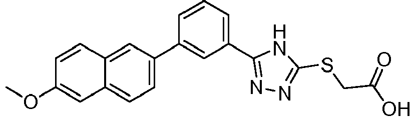 | 391.45 |
| **52** | 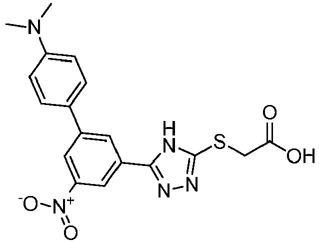 | 399.43 |
| **53** | 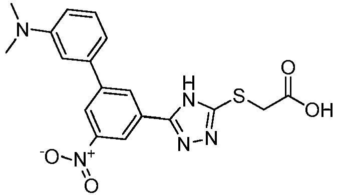 | 399.43 |
| **54** | 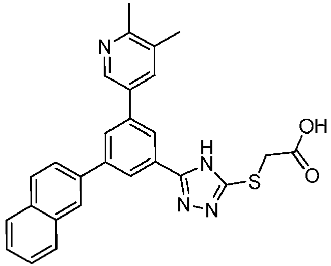 | 466.56 |
| **55** | 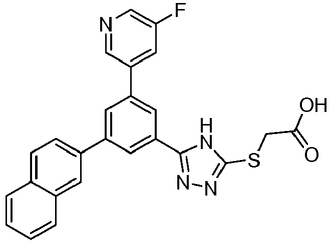 | 456.49 |
| **56** | 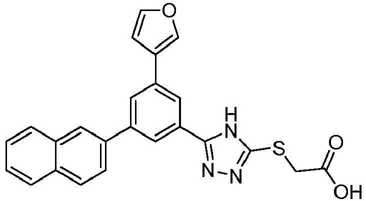 | 427.48 |
| **57** | 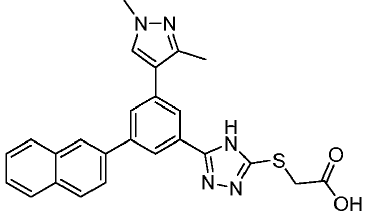 | 455.53 |
| **58** | 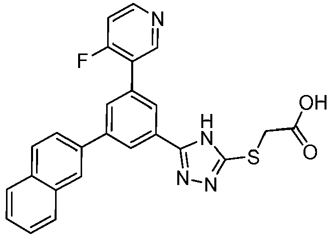 | 456.49 |
| **59** | 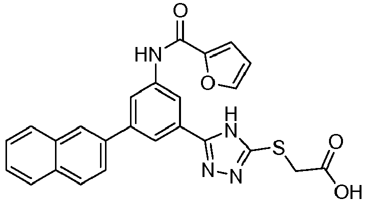 | 470.50 |
| **60** | 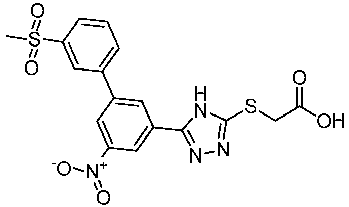 | 434.45 |
| **61** | 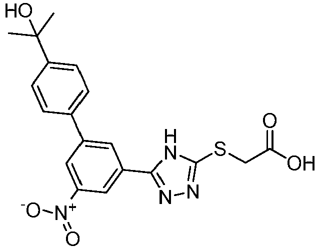 | 414.44 |
| **62** | 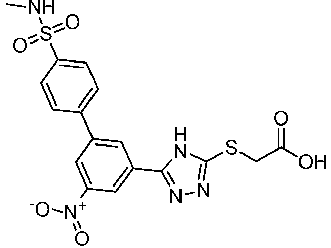 | 449.46 |
| **63** | 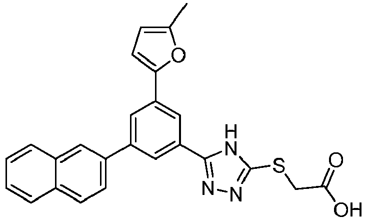 | 441.50 |
| **64** | 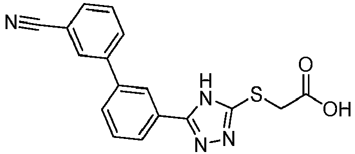 | 336.37 |
| **65** | 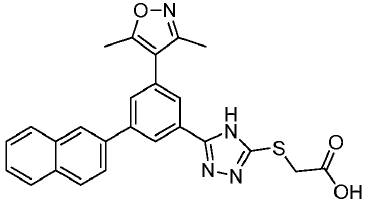 | 456.52 |
| **66** | 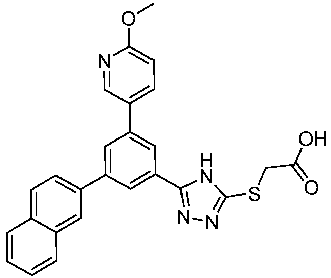 | 468.53 |
| **67** | 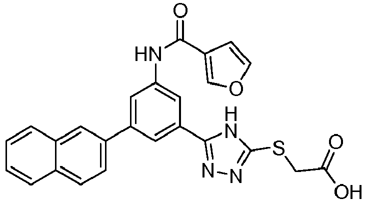 | 470.50 |
| **68** | 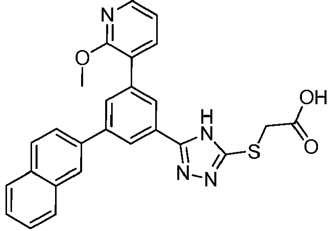 | 468.53 |
| **69** | 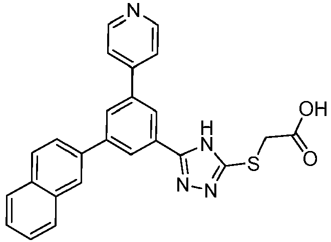 | 438.50 |
| **70** | 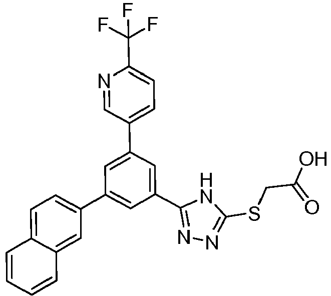 | 506.50 |

### Example 3: IC₅₀ Determination

### General Assay Conditions

The following assay conditions were used.
Buffer: 50 mM TRIS-HCl, pH 7.5, 5 mM MgCl₂, 2 mM DTT, 0.01% Triton X-100, 10 µM Na₃VO₄, 10 µM b-GP, 1%DMSO
Enzyme: 20 nM GRK6, recombinant full-length GST-tagged human protein ATP: 12 µM (Km)
Peptide substrate (Peptide 216): 1 µM Incubation Time: 7 h

Two hundred forty compounds were further characterized by determining IC₅₀ values in a GRK6 assay. This screen has been previously described in WO 2013/063458. Briefly, the following activities were performed: 8-point concentration-response determinations in singlicate wells (top concentration = 60 µM, 3-fold dilution steps); Repeat tests for QC failed or inconclusive compounds; and Repeat tests (titrate down) for compounds with >50% inhibition at all tested concentrations.

Results of the testing are shown in Table 2.

**Table 2.**

| **#** | **IC₅₀** | **#** | **IC₅₀** | **#** | **IC₅₀** | **#** | **IC₅₀** | **#** | **IC₅₀** |
|---|---|---|---|---|---|---|---|---|---|
| **1** | 0.05 | **51** | 0.51 | **101** | 3.02 | **151** | 12.9 | **201** | > 100 |
| **2** | 0.0588 | **52** | 0.518 | **102** | 3.08 | **152** | 13.3 | **202** | > 100 |
| **3** | 0.06 | **53** | 0.538 | **103** | 3.3 | **153** | 13.4 | **203** | > 100 |
| **4** | 0.0674 | **54** | 0.598 | **104** | 3.3 | **154** | 13.4 | **204** | > 100 |
| **5** | 0.0739 | **55** | 0.631 | **105** | 3.33 | **155** | 14.1 | **205** | > 100 |
| **6** | 0.0784 | **56** | 0.686 | **106** | 3.36 | **156** | 14.4 | **206** | > 100 |
| **7** | 0.0923 | **57** | 0.748 | **107** | 3.39 | **157** | 15.4 | **207** | > 100 |
| **8** | 0.101 | **58** | 0.751 | **108** | 3.47 | **158** | 15.9 | **208** | > 70 |
| **9** | 0.103 | **59** | 0.768 | **109** | 3.58 | **159** | 16.2 | **209** | > 100 |
| **10** | 0.114 | **60** | 0.779 | **110** | 4.11 | **160** | 17.3 | **210** | > 100 |
| **11** | 0.118 | **61** | 0.786 | **111** | 4.41 | **161** | 17.5 | **211** | > 74 |
| **12** | 0.122 | **62** | 0.824 | **112** | 4.47 | **162** | 17.5 | **212** | > 58 |
| **13** | 0.122 | **63** | 0.824 | **113** | 4.55 | **163** | 18.3 | **213** | > 100 |
| **14** | 0.136 | **64** | 0.919 | **114** | 4.62 | **164** | 18.5 | **214** | > 100 |
| **15** | 0.14 | **65** | 1.02 | **115** | 4.7 | **165** | 18.9 | **215** | > 100 |
| **16** | 0.14 | **66** | 1.08 | **116** | 4.78 | **166** | 20.2 | **216** | > 100 |
| **17** | 0.168 | **67** | 1.31 | **117** | 4.85 | **167** | 20.4 | **217** | > 100 |
| **18** | 0.178 | **68** | 1.58 | **118** | 4.99 | **168** | 20.7 | **218** | > 100 |
| **19** | 0.18 | **69** | 1.86 | **119** | 5.02 | **169** | 20.9 | **219** | > 100 |
| **20** | 0.184 | **70** | 2.73 | **120** | 5.06 | **170** | 21.3 | **220** | > 100 |
| **21** | 0.184 | **71** | 0.131 | **121** | 5.85 | **171** | 21.9 | **221** | > 100 |
| **22** | 0.187 | **72** | 0.141 | **122** | 6.1 | **172** | 23.7 | **222** | > 100 |
| **23** | 0.199 | **73** | 0.342 | **123** | 6.16 | **173** | 25.8 | **223** | > 100 |
| **24** | 0.212 | **74** | 0.379 | **124** | 6.36 | **174** | 28.1 | **224** | > 80 |
| **25** | 0.227 | **75** | 0.431 | **125** | 6.45 | **175** | 29.6 | **225** | > 100 |
| **26** | 0.249 | **76** | 0.653 | **126** | 6.5 | **176** | 33.3 | **226** | > 100 |
| **27** | 0.262 | **77** | 0.695 | **127** | 6.54 | **177** | 34 | **227** | 0.060 |
| **28** | 0.262 | **78** | 0.742 | **128** | 6.8 | **178** | 34.8 | **228** | 0.101 |
| **29** | 0.269 | **79** | 0.75 | **129** | 6.88 | **179** | 35.3 | **229** | 0.114 |
| **30** | 0.275 | **80** | 0.751 | **130** | 6.93 | **180** | 35.7 | **230** | 0.150 |
| **31** | 0.283 | **81** | 0.757 | **131** | 7.3 | **181** | 38.5 | **231** | 0.150 |
| **32** | 0.3 | **82** | 0.761 | **132** | 7.36 | **182** | 40 | **232** | 0.184 |
| **33** | 0.302 | **83** | 0.797 | **133** | 7.37 | **183** | 40 | **233** | 0.209 |
| **34** | 0.349 | **84** | 0.879 | **134** | 7.45 | **184** | 40 | **234** | 0.267 |
| **35** | 0.351 | **85** | 0.886 | **135** | 7.92 | **185** | 45.3 | **235** | 0.358 |
| **36** | 0.352 | **86** | 0.935 | **136** | 7.93 | **186** | 47.8 | **236** | 0.402 |
| **37** | 0.359 | **87** | 1.05 | **137** | 8.23 | **187** | 51.8 | **237** | 0.438 |
| **38** | 0.376 | **88** | 1.05 | **138** | 8.75 | **188** | 74.1 | **238** | 0.442 |
| **39** | 0.417 | **89** | 1.05 | **139** | 8.83 | **189** | 77.5 | **239** | 0.729 |
| **40** | 0.421 | **90** | 1.35 | **140** | 8.83 | **190** | 80.6 | **240** | 0.930 |
| **41** | 0.426 | **91** | 1.38 | **141** | 8.84 | **191** | 81.7 | | |
| **42** | 0.431 | **92** | 1.4 | **142** | 9.56 | **192** | 87 | | |
| **43** | 0.433 | **93** | 1.57 | **143** | 9.82 | **193** | >200 | | |
| **44** | 0.437 | **94** | 1.57 | **144** | 9.9 | **194** | > 100 | | |
| **45** | 0.44 | **95** | 2.02 | **145** | 10.7 | **195** | > 100 | | |
| **46** | 0.461 | **96** | 2.31 | **146** | 11.4 | **196** | > 100 | | |
| **47** | 0.473 | **97** | 2.49 | **147** | 11.6 | **197** | > 100 | | |
| **48** | 0.499 | **98** | 2.61 | **148** | 11.7 | **198** | > 100 | | |
| **49** | 0.507 | **99** | 2.71 | **149** | 11.8 | **199** | > 100 | | |
| **50** | 0.509 | **100** | 2.75 | **150** | 12.4 | **200** | > 100 | | |

## Claims

1. A compound of Formula I: 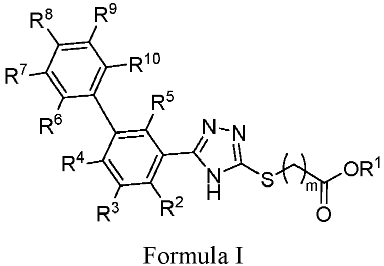 or a pharmaceutically acceptable salt thereof,
wherein:
R¹ is selected from the group consisting of H and (C₁-C₆)alkyl;
each of R², R⁴, and R⁵ are independently selected from the group consisting of: H, halo, substituted or unsubstituted (C₁-C₆)alkyl, (C₁-C₆)haloalkyl, OR^{A}, CN, NO₂, C(O)R^{A}, NR^{A}R^{B}, -S(O)₂R^{A}, -S(O)₂NR^{A}R^{B}, -S(O)NR^{A}R^{B}, -NR^{A}S(O)₂R^{B}, - C(NR^{A})R^{B}, -C(O)NR^{A}R^{B}, a substituted or unsubstituted aryl, a substituted or unsubstituted heteroaryl, a substituted or unsubstituted cycloalkyl, and a substituted or unsubstituted heterocycloalkyl;
R³ is NO₂;
each of R⁶, R⁷, R⁸, R⁹, and R¹⁰ are independently selected from the group consisting of: H, substituted or unsubstituted (C₁-C₆)alkyl, (C₁-C₆)haloalkyl, OR^{A},NO₂, C(O)R^{A}, NR^{A}R^{B}, -S(O)₂NR^{A}R^{B}, -S(O)NR^{A}R^{B}, -C(NR^{A})R^{B}, -C(O)NR^{A}R^{B},-NR^{A}S(O)₂R^{B}, -NR^{A}C(O)R^{B}, -S(O)₂R^{A}, a substituted or unsubstituted aryl, a substituted or unsubstituted heteroaryl, a substituted or unsubstituted cycloalkyl, and a substituted or unsubstituted heterocycloalkyl; or
R⁶ and R⁷, R⁷ and R⁸, R⁸ and R⁹, or R⁹ and R¹⁰ can come together to form a substituted or unsubstituted fused cycloalkyl ring, a substituted or unsubstituted fused heterocycloalkyl ring, or a substituted or unsubstituted fused heteroaryl ring;
each R^{A} and R^{B} is independently selected from the group consisting of H and (C₁-C₆)alkyl; and
m is an integer from 1 to 2.

2. The compound of claim 1, wherein R¹ is H or/and m is 1.

3. The compound of claim 2, wherein each of R², R⁴, and R⁵ are independently selected from the group consisting of: H, NO₂, and a substituted or unsubstituted (C₁-C₆)alkyl, wherein optionally one of R⁶ and R⁷, R⁷ and R⁸, R⁸ and R⁹, or R⁹ and R¹⁰ come together to form a substituted or unsubstituted fused cycloalkyl ring, or
come together to form a substituted or unsubstituted fused heterocycloalkyl ring, or come together to form a substituted or unsubstituted fused heteroaryl ring, or/and wherein each of R⁶, R⁷, R⁸, R⁹, and R¹⁰ are independently selected from the group consisting of: H, substituted or unsubstituted (C₁-C₆)alkyl, OR^{A}, C(O)R^{A}, NR^{A}R^{B}, - S(O)₂NR^{A}R^{B}, -S(O)NR^{A}R^{B}, -C(NR^{A})R^{B}, -C(O)NR^{A}R^{B}, -NR^{A}S(O)₂R^{B}, -NR^{A}C(O)R^{B}, -S(O)₂R^{A}, and a substituted or unsubstituted heteroaryl.

4. The compound of claim 1, wherein the compound of Formula I is selected from the group consisting of: 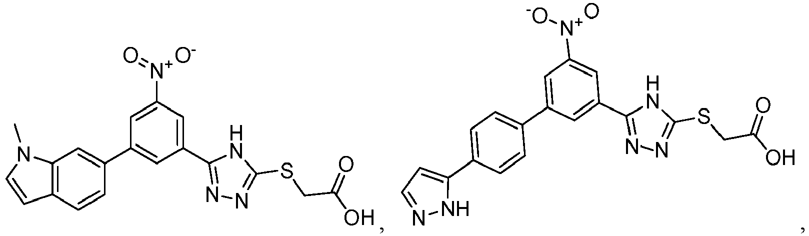 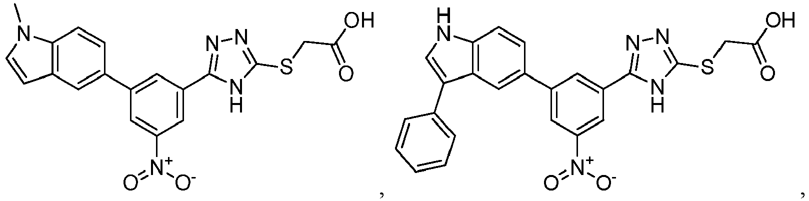 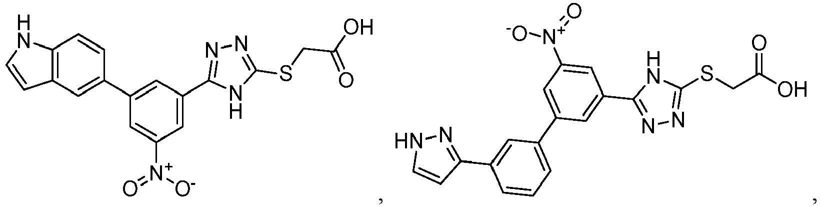 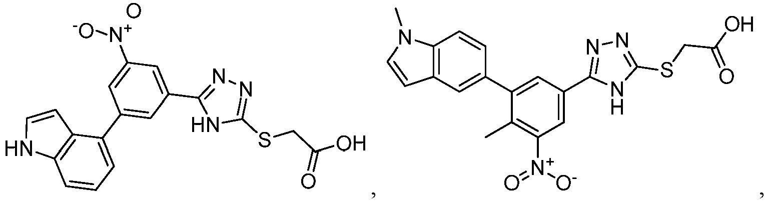 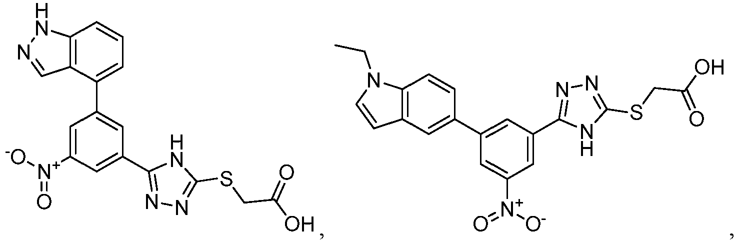 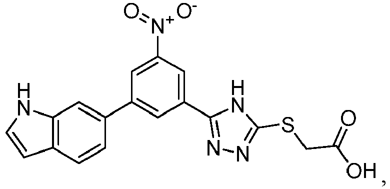 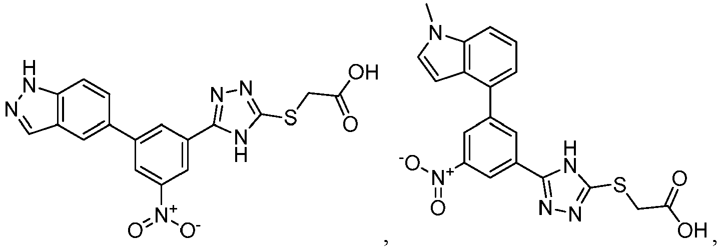 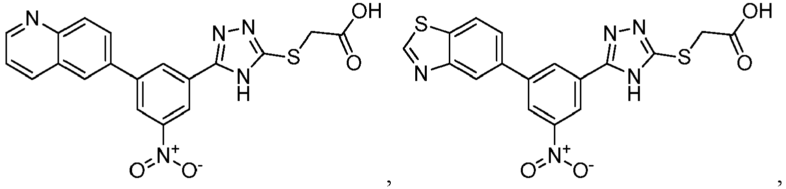 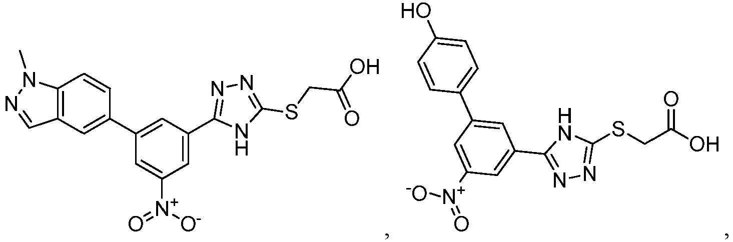 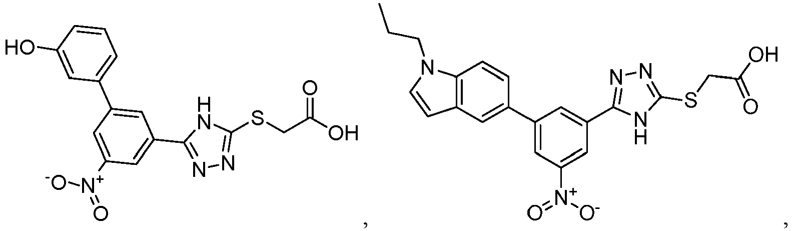 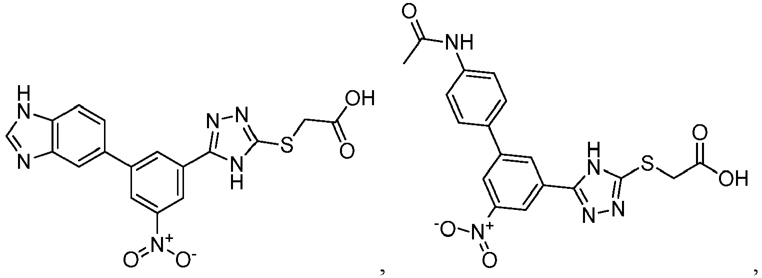 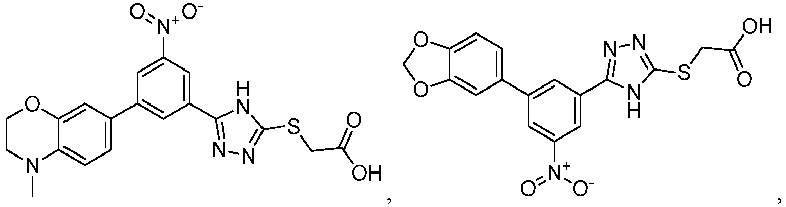 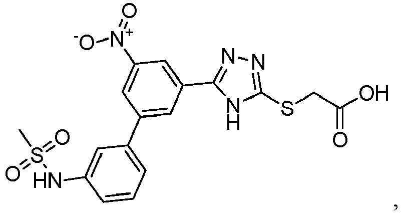 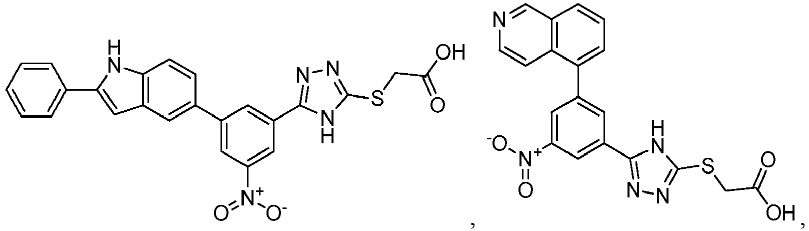 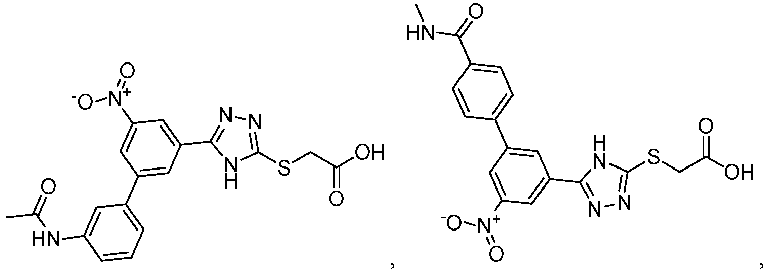 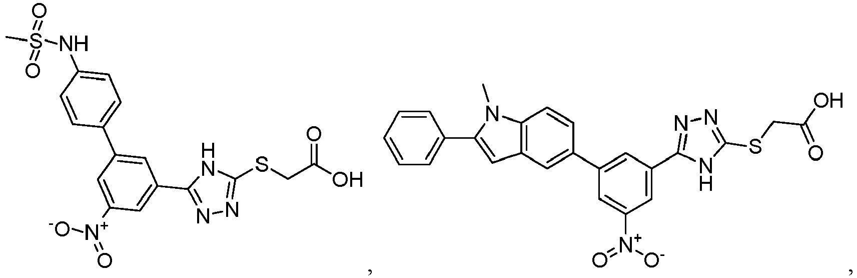 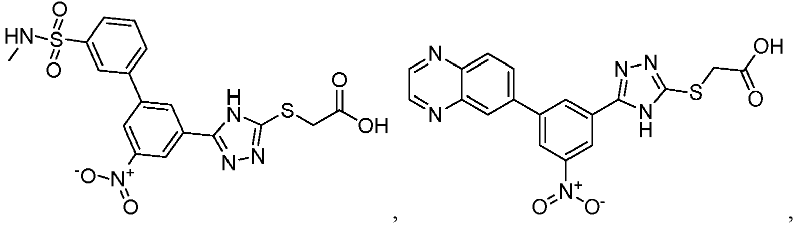 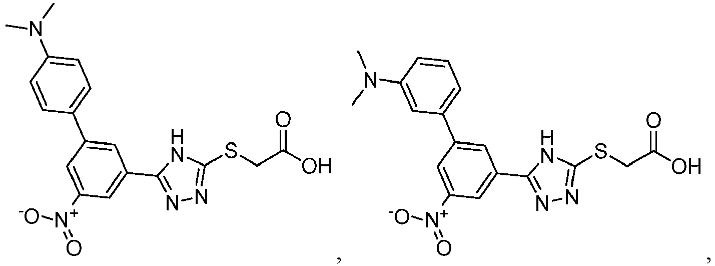 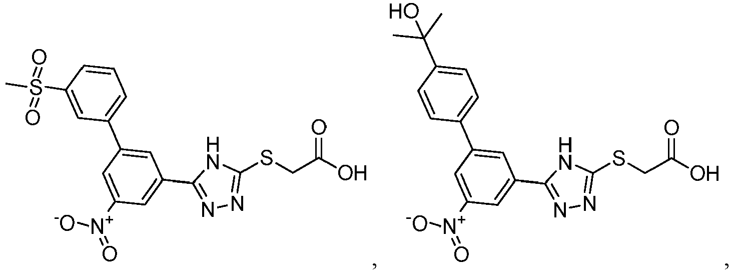 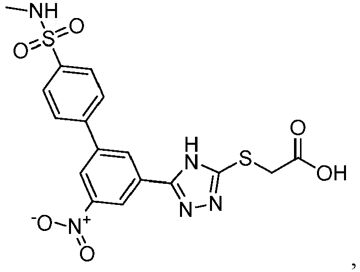 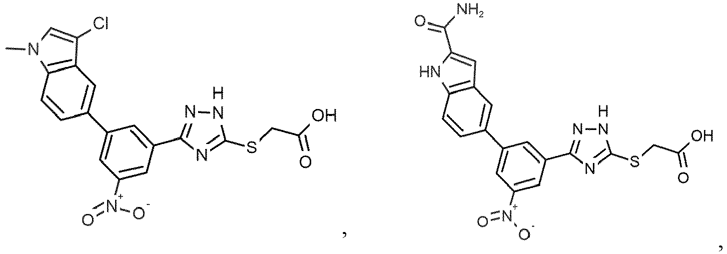 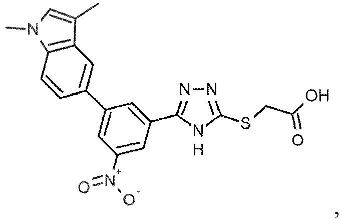 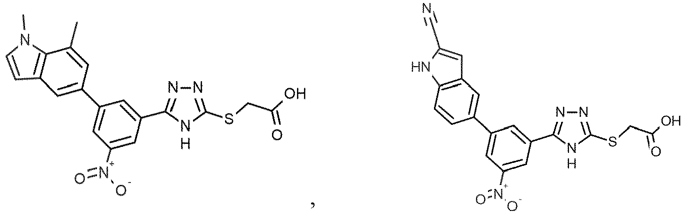 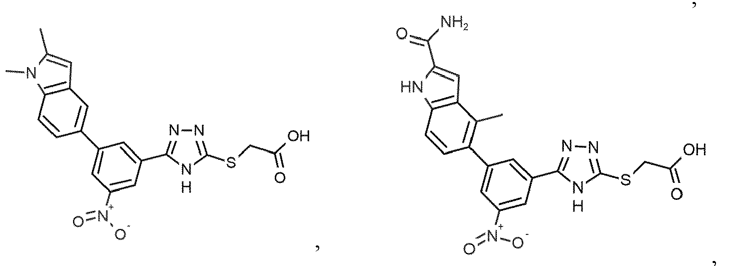 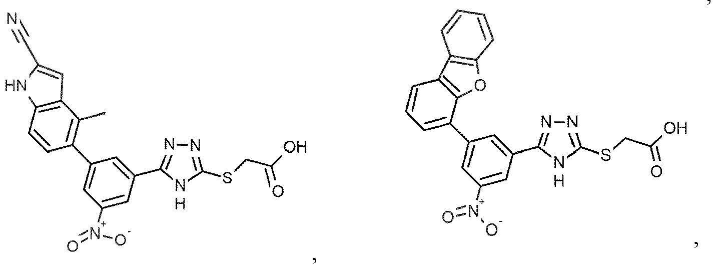 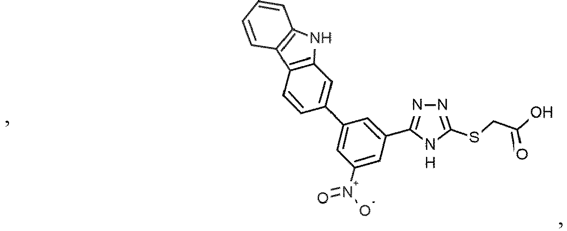 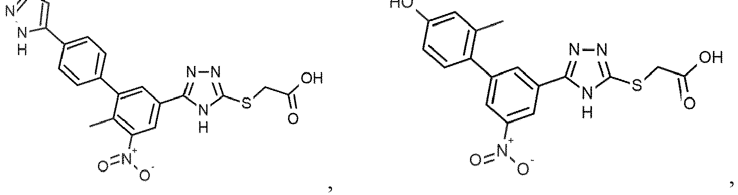 and 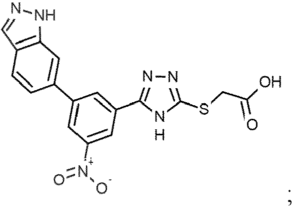 or a pharmaceutically acceptable salt thereof.

5. A pharmaceutical composition comprising a compound of any one of claims 1-4, or a pharmaceutically acceptable salt thereof, and a pharmaceutically acceptable carrier.

6. An *in vitro* method for inhibiting a G protein coupled receptor 6 kinase polypeptide in a cell, the method comprising contacting the cell with an effective amount of a compound of any one of claims 1-4, or a pharmaceutically acceptable salt thereof, wherein optionally the cell is a cancerous cell, wherein optionally the cancerous cell is a B cell cancerous cell.

7. Compound of any one of claims 1-4, or a pharmaceutically acceptable salt thereof, for use in treating a hematological malignancy or an inflammatory disease in a patient or suppressing an immune response in a patient.

8. The compound for the use of claim 7, wherein
(a) the hematological malignancy is a B cell cancer,
wherein optionally the B cell cancer is selected from the group consisting of: a small lymphocytic lymphoma (SLL), a mantle cell lymphoma, a Burkitt's lymphoma, a follicle centre cell lymphoma, a follicular lymphoma, a Burkitt-like lymphoma, a marginal zone B-cell lymphoma (MZBCL), a nodal marginal zone B cell lymphoma, an extra-nodal marginal zone B cell lymphoma, a splenic marginal zone B cell lymphoma, a lymphoplasmacytic lymphoma, a diffuse large B cell lymphoma, a B cell acute lymphocytic leukemia (B-ALL), a precursor B cell acute lymphocytic leukemia (B-ALL), a B cell chronic lymphocytic leukemia (B-CLL), a precursor B-lymphoblastic leukaemia, a precursor B-lymphoblastic lymphoma, a B cell prolymphocytic leukemia, an undifferentiated B cell lymphoma, a hairy cell leukemia, a mediastinal large B-cell lymphoma, a plasma cell myeloma, a plasmacytoma, a primary effusive lymphoma, a Burkitt's cell leukemia, and a B cell diffuse mixed lymphoma;
(b) the inflammatory disease is selected from the group consisting of: encephalitis, inflammatory eye disease, otitis, pharyngitis, pneumonia, gastritis, enteritis, hepatitis, pancreatitis, nephritis, cystitis, urethritis, endometritis, vaginitis, arthritis, peripheral neuritis, malignant tumor, infectious diseases, autoimmune diseases, ischemic diseases, metabolic diseases, injury, scald, chemical corrosion, and neurodegenerative diseases,
wherein optionally the autoimmune diseases are selected from the group consisting of: rheumatism, systemic lupus erythematosus, and sarcoidosis
wherein optionally the ischemic diseases are selected from the group consisting of: myocardial infarction and cerebral infarction,
wherein optionally the metabolic diseases are selected from the group consisting of: diabetes and gout, or
wherein optionally the neurodegenerative disease is Alzheimer's.

## Patentansprüche

1. Verbindung der Formel I: 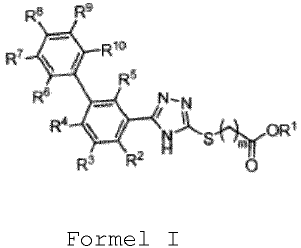 oder ein pharmazeutisch unbedenkliches Salz davon,
wobei:
R¹ ausgewählt ist aus der aus H und (C₁-C₆)Alkyl bestehenden Gruppe;
R², R⁴ und R⁵ jeweils unabhängig ausgewählt sind aus der folgenden Gruppe: H, Halogen, substituiertes oder unsubstituiertes (C₁-C₆) Alkyl, (C₁-C₆) Halogenalkyl, OR^{A}, CN, NO₂, C(O)R^{A}, NR^{A}R^{B}, -S(O)₂R^{A}, -S(O)₂NR^{A}R^{B}, -S(O)NR^{A}R^{B}, - NR^{A}S(O)₂R^{B}, -C(NR^{A})R^{B}, -C(O)NR^{A}R^{B}, ein substituiertes oder unsubstituiertes Aryl, ein substituiertes oder unsubstituiertes Heteroaryl, ein substituiertes oder unsubstituiertes Cycloalkyl und ein substituiertes oder unsubstituiertes Heterocycloalkyl;
R³ NO₂ ist;
R⁶, R⁷, R⁸, R⁹ und R¹⁰ jeweils unabhängig ausgewählt sind aus der folgenden Gruppe: H, substituiertes oder unsubstituiertes (C₁-C₆) Alkyl, (C₁-C₆) Halogenalkyl, OR^{A}, NO₂, C(O)R^{A}, NR^{A}R^{B}, -S(O)₂NR^{A}R^{B}, -S(O)NR^{A}R^{B}, -C(NR^{A})R^{B},-C(O)NR^{A}R^{B}, -NR^{A}S(O)₂R^{B}, -NR^{A}C(O)R^{B}, -S(O)₂R^{A}, ein substituiertes oder unsubstituiertes Aryl, ein substituiertes oder unsubstituiertes Heteroaryl, ein substituiertes oder unsubstituiertes Cycloalkyl und ein substituiertes oder unsubstituiertes Heterocycloalkyl; oder
R⁶ und R⁷, R⁷ und R⁸, R⁸ und R⁹ oder R⁹ und R¹⁰ zur Bildung eines substituierten oder unsubstituierten kondensierten Cycloalkyl-Rings, eines substituierten oder unsubstituierten kondensierten Heterocycloalkyl-Rings oder eines substituierten oder unsubstituierten kondensierten Heteroaryl-Rings zusammenkommen können;
jedes R^{A} und R⁸ unabhängig ausgewählt ist aus der aus H und (C₁-C₆)Alkyl bestehenden Gruppe; und
m eine Ganzzahl von 1 bis 2 ist.

2. Verbindung nach Anspruch 1, wobei R¹ H ist oder/und m 1 ist.

3. Verbindung nach Anspruch 2, wobei R², R⁴ und R⁵ jeweils unabhängig ausgewählt sind aus der folgenden Gruppe: H, NO₂ und ein substituiertes oder unsubstituiertes (C₁-C₆)Alkyl, wobei fakultativ einer von R⁶ und R⁷, R⁷ und R⁸, R⁸ und R⁹ oder R⁹ und R¹⁰ zur Bildung eines substituierten oder unsubstituierten kondensierten Cycloalkyl-Rings zusammenkommen, oder
zur Bildung eines substituierten oder unsubstituierten kondensierten Heterocycloalkyl-Rings zusammenkommen, oder zur Bildung oder eines substituierten oder unsubstituierten kondensierten Heteroaryl-Rings zusammenkommen, oder/und wobei R⁶, R⁷, R⁸, R⁹ und R¹⁰ jeweils unabhängig ausgewählt sind aus der folgenden Gruppe: H, substituiertes oder unsubstituiertes (C₁-C₆)Alkyl, OR^{A}, C(O)R^{A}, NR^{A}R^{B}, -S(O)₂NR^{A}R^{B}, -S(O)NR^{A}R^{B}, -C(NR^{A})R^{B}, -C(O)NR^{A}R^{B}, -NR^{A}S(O)²R^{B}, -NR^{A}C(O)R^{B}, -S(O)²R^{A}, und ein substituiertes oder unsubstituiertes Heteroaryl.

4. Verbindung nach Anspruch 1, wobei die Verbindung der Formel I aus der folgenden Gruppe ausgewählt ist: 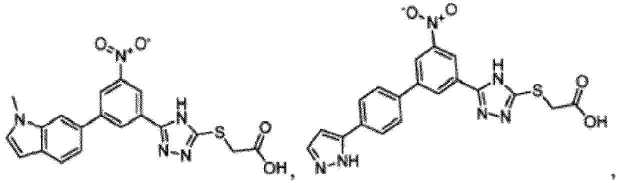 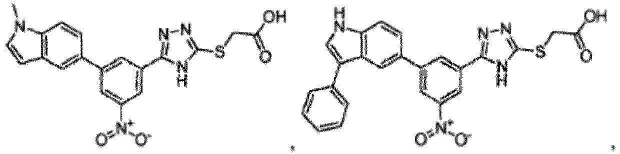 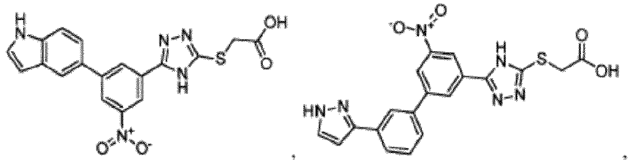 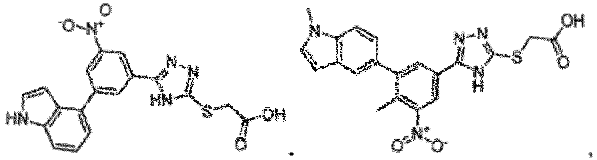 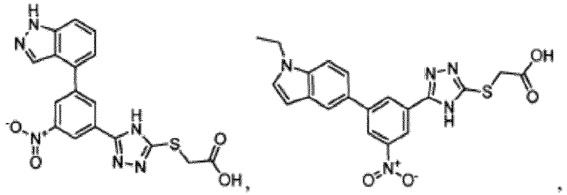 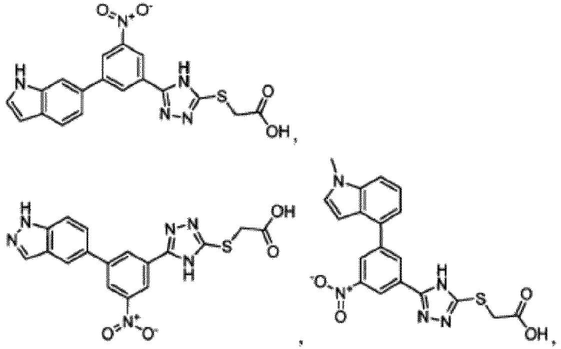 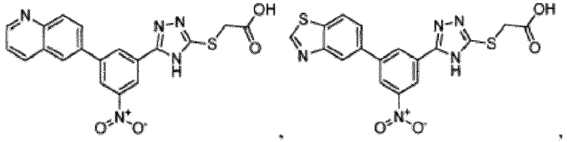 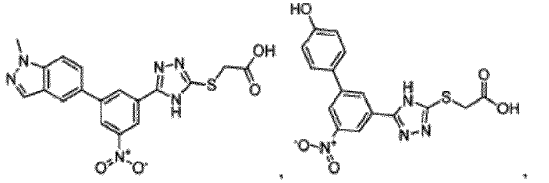 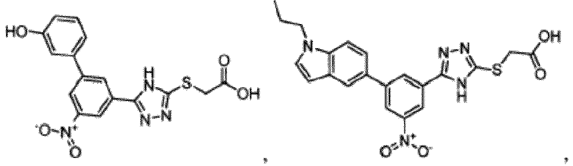 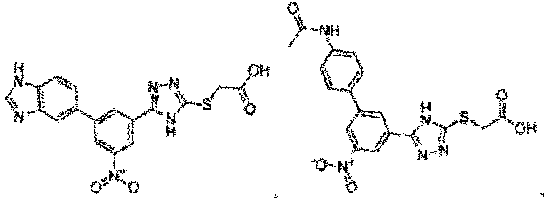 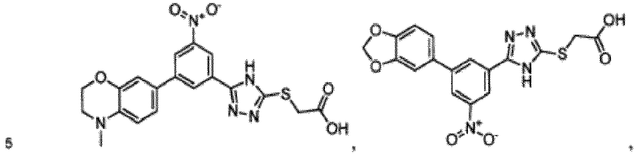 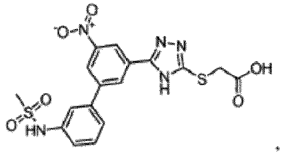 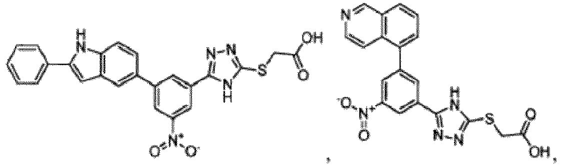 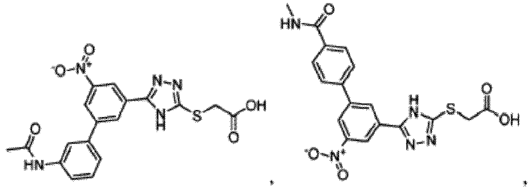 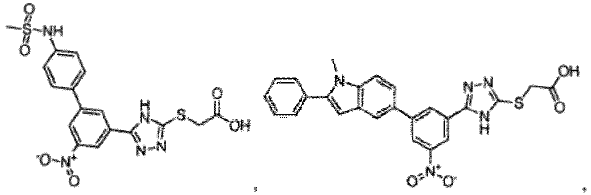 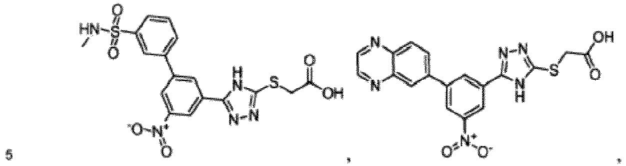 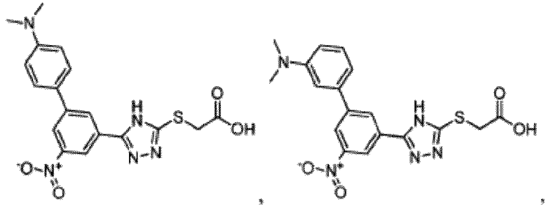 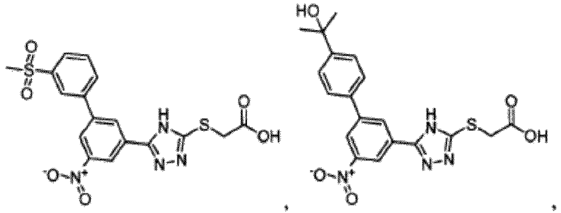 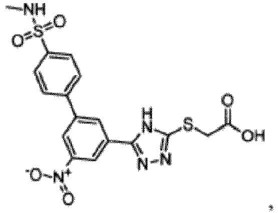 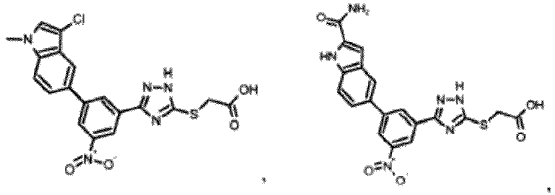 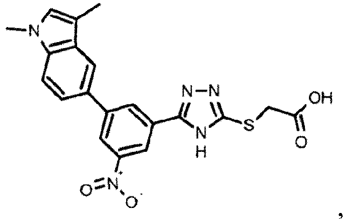 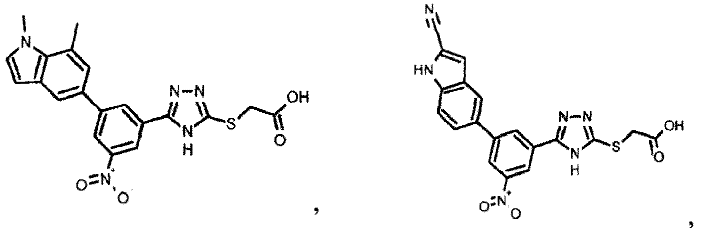 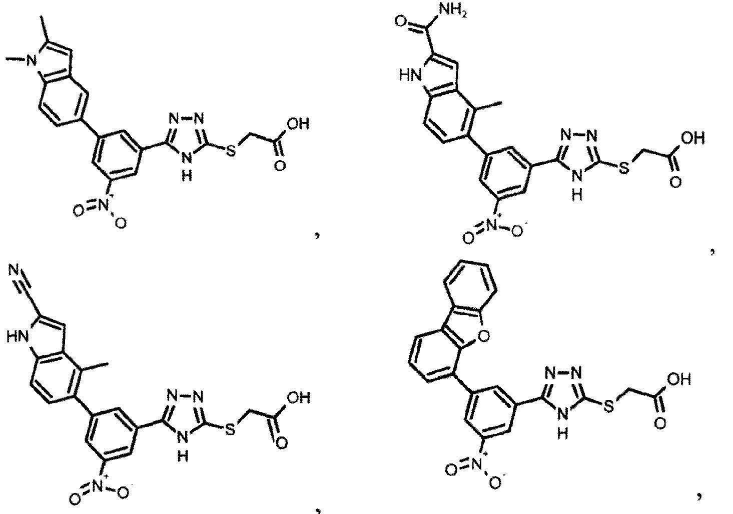 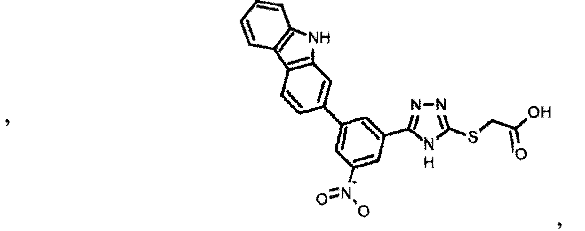 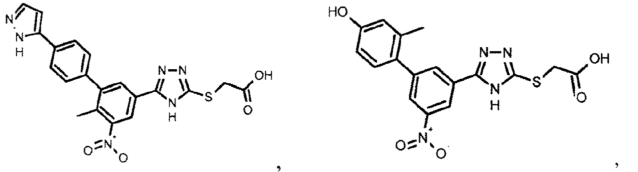 und 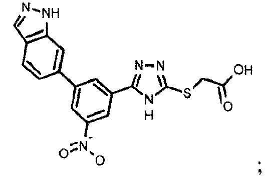 oder ein pharmazeutisch unbedenkliches Salz davon.

5. Pharmazeutische Zusammensetzung, umfassend eine Verbindung nach einem der Ansprüche 1-4 oder ein pharmazeutisch unbedenkliches Salz davon, und einen pharmazeutisch unbedenklichen Träger.

6. In-vitro-Verfahren zur Unterdrückung eines an G-Protein gekoppelten Rezeptor-6-Kinase-Polypeptids in einer Zelle, wobei das Verfahren das Kontaktieren der Zelle mit einer wirksamen Menge einer Verbindung nach einem der Ansprüche 1-4, oder eines pharmazeutisch unbedenklichen Salzes davon umfasst, wobei die Zelle fakultativ eine kanzeröse Zelle ist,
wobei die kanzeröse Zelle fakultativ eine B-Zell-Krebszelle ist.

7. Verbindung nach einem der Ansprüche 1-4, oder ein pharmazeutisch unbedenkliches Salz davon, zur Verwendung bei der Behandlung einer hämatologischen malignen Erkrankung oder einer entzündlichen Erkrankung in einem Patienten oder bei der Unterdrückung einer Immunantwort in einem Patienten.

8. Verbindung zur Verwendung nach Anspruch 7, wobei
(a) die hämatologische maligne Erkrankung ein B-Zell-Krebs ist,
wobei fakultativ der B-Zellkrebs aus der folgenden Gruppe ausgewählt ist: ein kleinzelliges lymphozytisches Lymphom (SLL), ein Mantelzelllymphom, ein Burkitt-Lymphom, ein follikuläres Keimzentrumslymphom, ein follikuläres Lymphom, ein Burkitt-like Lymphom, ein Marginalzonen-B-Zell-Lymphom (MZBCL), ein nodales Marginalzonen-B-Zell-Lymphom, ein extranodales Marginalzonen-B-Zell-Lymphom, ein Milz- Marginalzonen-B-Zell-Lymphom, ein lymphoplasmozytisches Lymphom, ein diffuses großzelliges B-Zell-Lymphom, eine akute lymphozytische B-Zell-Leukämie (B-ALL), eine akute lymphozytische Vorläufer-B-Zell-Leukämie (B-ALL), eine chronische lymphozytische B-Zell-Leukämie (B-CLL), eine Vorläufer-B-lymphoblastische Leukämie, ein Vorläufer-B-lymphoblastisches Lymphom, eine prolymphozytische B-Zell-Leukämie, ein undifferenziertes B-Zell-Lymphom, eine Haarzellenleukämie, ein mediastinales großzelliges B-Zell-Lymphom, ein Plasmazellmyelom, ein Plasmazytom, ein primäres Effusionslymphom, eine Burkitt-Zell-Leukämie und ein diffuses gemischtes B-Zell-Lymphom;
(b) die entzündliche Erkrankung aus der folgenden Gruppe ausgewählt ist: Enzephalitis, entzündliche Augenerkrankung, Otitis, Pharyngitis, Pneumonie, Gastritis, Enteritis, Hepatitis, Pankreatitis, Nephritis, Zystitis, Urethritis, Endometritis, Vaginitis, Arthritis, periphere Neuritis, maligner Tumor, Infektionskrankheiten, Autoimmunkrankheiten, ischämische Erkrankungen, Stoffwechselerkrankungen, Verletzung, Verbrühung, chemische Korrosion und neurodegenerative Erkrankungen,
wobei die Autoimmunkrankheiten fakultativ aus der folgenden Gruppe ausgewählt sind: Rheuma, systemischer Lupus erythematodes und Sarkoidose,
wobei die ischämischen Erkrankungen fakultativ aus der folgenden Gruppe ausgewählt sind: Myokardinfarkt und Zerebralinfarkt,
wobei die Stoffwechselerkrankungen fakultativ aus der folgenden Gruppe ausgewählt sind: Diabetes und Gicht, oder
wobei die neurodegenerative Erkrankung fakultativ Alzheimer-Krankheit ist.

## Revendications

1. Composé ayant la formule (I) : 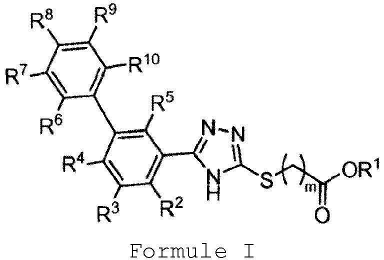 ou sel pharmaceutiquement acceptable de celui-ci,
dans lequel :
R¹ est sélectionné dans le groupe constitué d'un atome d'hydrogène et d'un groupe alkyle C₁-C₆ ;
chacun de R², R⁴ et R⁵ est sélectionné indépendamment dans le groupe constitué d'un atome d'hydrogène et de groupes halo, alkyle C₁-C₆ substitué ou non substitué, haloalkyle C₁-C₆, OR^{A}, CN, NO₂, C(O)R^{A}, NR^{A}R^{B}, -S(O)₂R^{A}, -S(O)₂NR^{A}R^{B}, -S(O)NR^{A}R^{B}, -NR^{A}S(O)₂R^{B}, -C(NR^{A})R^{B}, -C(O)NR^{A}R^{B}, aryle substitué ou non substitué, hétéroaryle substitué ou non substitué, cycloalkyle substitué ou non substitué, et hétérocycloalkyle substitué ou non substitué ;
R³ est un groupe NO₂ ;
chacun de R⁶, R⁷, R⁸, R⁹ et R¹⁰ est sélectionné indépendamment dans le groupe constitué d'un atome d'hydrogène et de groupes alkyle C₁-C₆ substitué ou non substitué, haloalkyle C₁-C₆, OR^{A}, NO₂, C(O)R^{A}, NR^{A}R^{B}, -S(O)₂NR^{A}R^{B}, -S(O)NR^{A}R^{B}, -C(NR^{A})R^{B}, -C(O)NR^{A}R^{B}, -NR^{A}S(O)₂R^{B}, -NR^{A}C(O)R^{B}, -S(O)₂R^{A}, aryle substitué ou non substitué, hétéroaryle substitué ou non substitué, cycloalkyle substitué ou non substitué, et hétérocycloalkyle substitué ou non substitué ; ou
R⁶ et R⁷, R⁷ et R⁸, R⁸ et R⁹, ou R⁹ et R¹⁰ peuvent se réunir pour former un cycle cycloalkyle condensé substitué ou non substitué, un cycle hétérocycloalkyle condensé substitué ou non substitué, ou un cycle hétéroaryle condensé substitué ou non substitué ;
chaque groupe R^{A} et R^{B} est sélectionné indépendamment dans le groupe constitué d'un atome d'hydrogène et d'un groupe alkyle C₁-C₆ ; et
m est un nombre entier de 1 à 2.

2. Composé selon la revendication 1, dans lequel R¹ est un atome d'hydrogène ou/et m vaut 1.

3. Composé selon la revendication 2, dans lequel chacun de R², R⁴ et R⁵ est sélectionné indépendamment dans le groupe constitué d'un atome d'hydrogène, d'un groupe NO₂, et d'un groupe alkyle C₁-C₆ substitué ou non substitué, dans lequel optionnellement R⁶ et R⁷, ou R⁷ et R⁸, ou R⁸ et R⁹, ou R⁹ et R¹⁰, se réunissent pour former un cycle cycloalkyle condensé substitué ou non substitué, ou se réunissent pour former un cycle hétérocycloalkyle condensé substitué ou non substitué, ou se réunissent pour former un cycle hétéroaryle condensé substitué ou non substitué, ou/et dans lequel chacun de R⁶, R⁷, R⁸, R⁹ et R¹⁰ est sélectionné indépendamment dans le groupe constitué d'un atome d'hydrogène et de groupes alkyle C₁-C₆ substitué ou non substitué, OR^{A}, C(O)R^{A}, NR^{A}R^{B}, - S(O)₂NR^{A}R^{B}, -S(O)NR^{A}R^{B}, -C(NR^{A})R^{B}, -C(O)NR^{A}R^{B}, -NR^{A}S(O)₂R^{B},-NR^{A}C(O)R^{B}, -S(O)₂R^{A}, et hétéroaryle substitué ou non substitué.

4. Composé selon la revendication 1, le composé ayant la Formule I étant sélectionné dans le groupe constitué de : 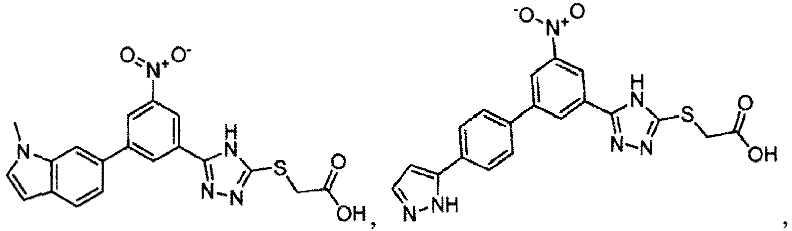 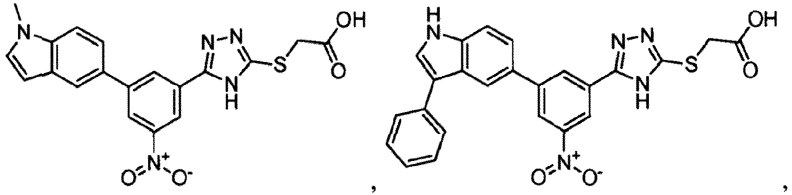 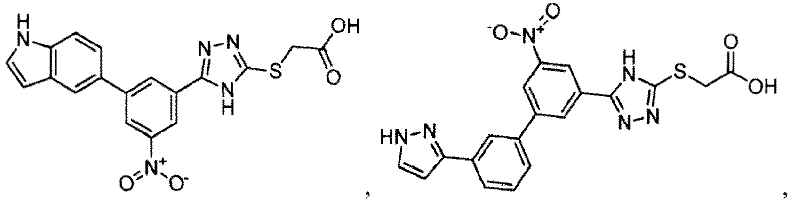 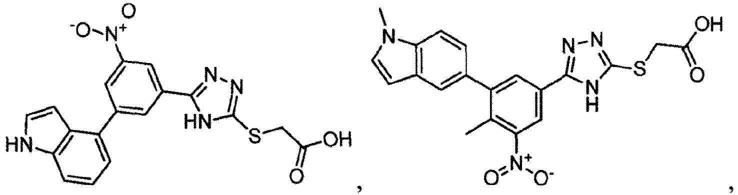 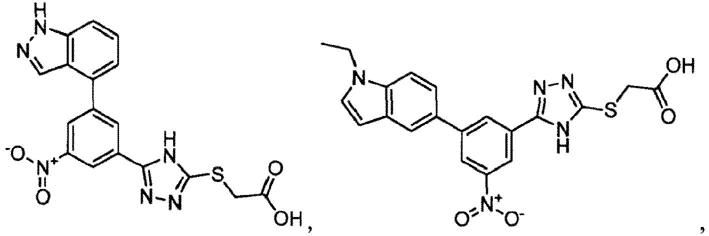 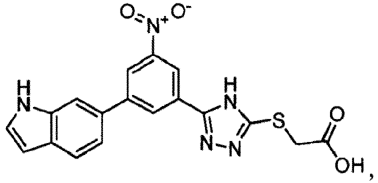 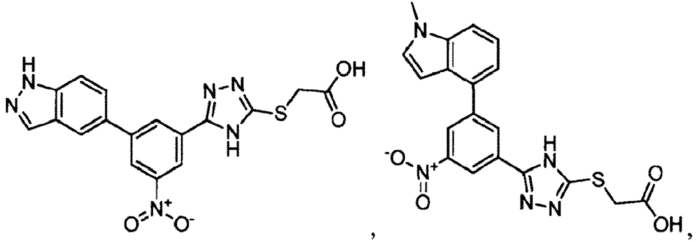 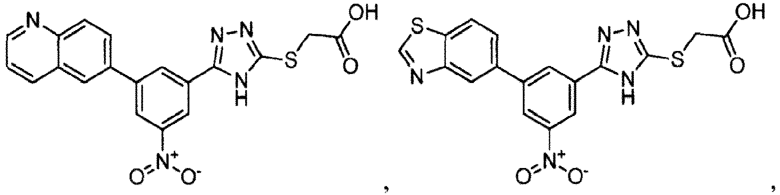 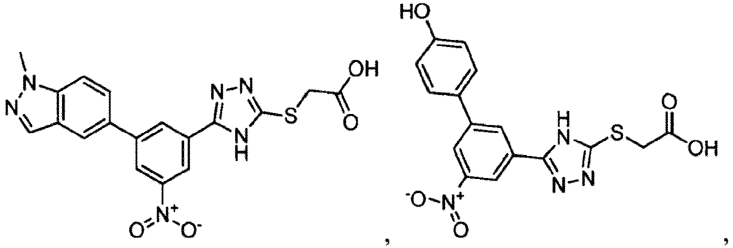 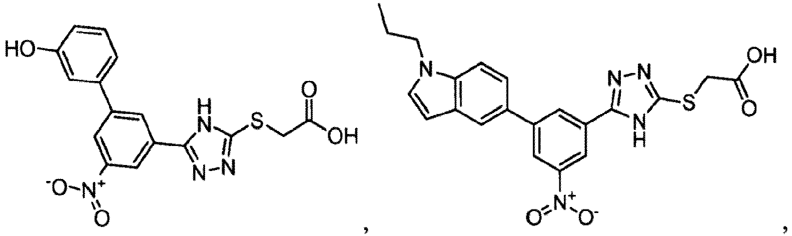 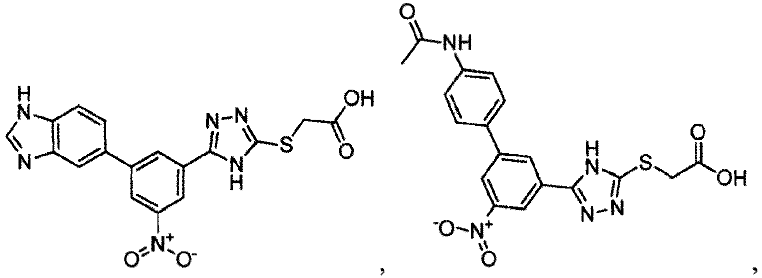 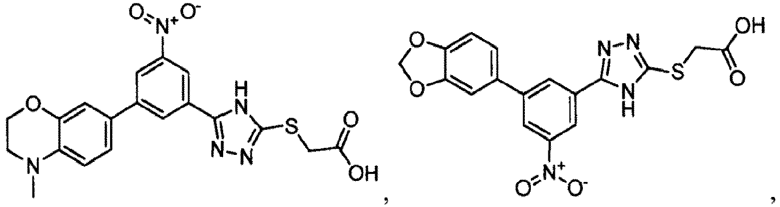 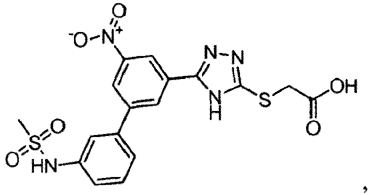 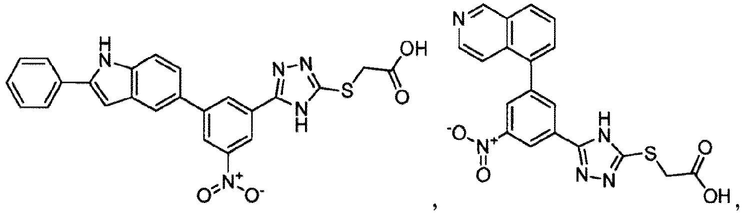 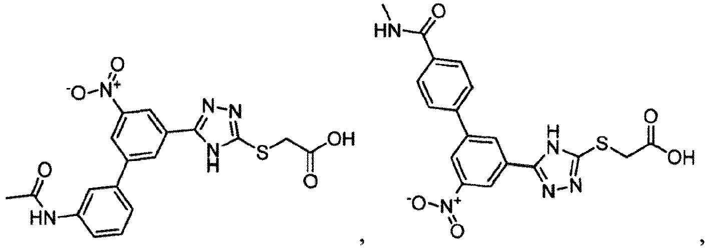 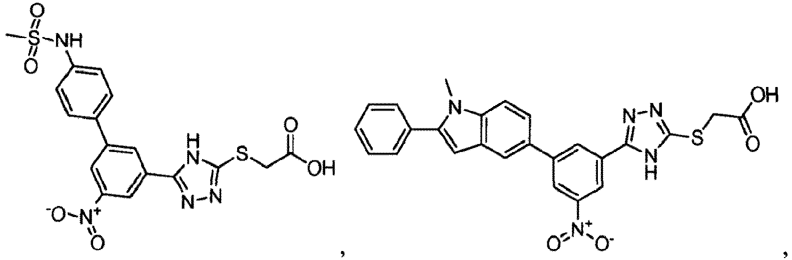 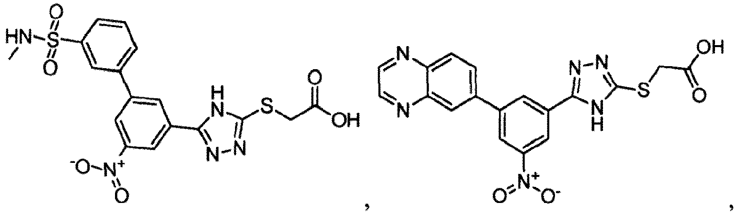 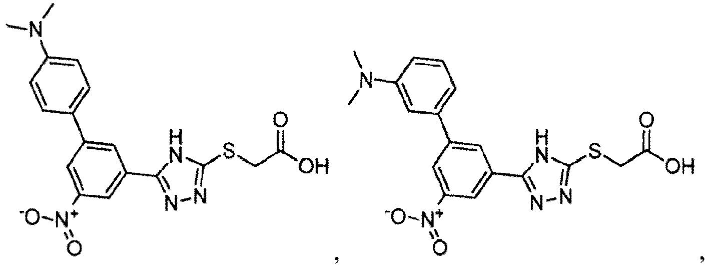 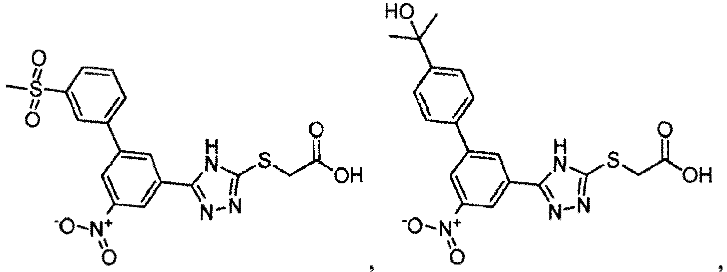 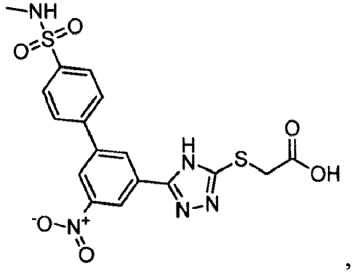 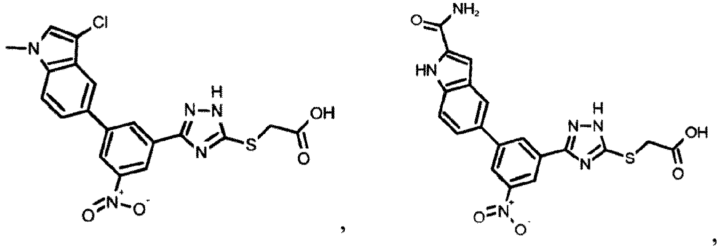 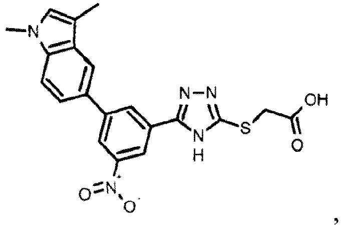 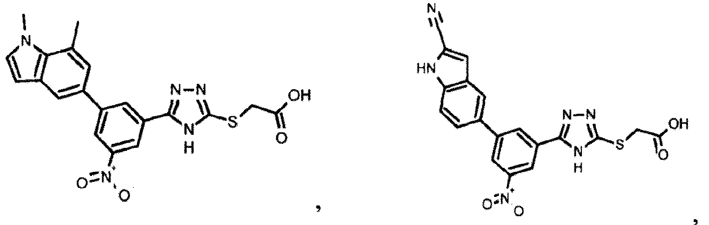 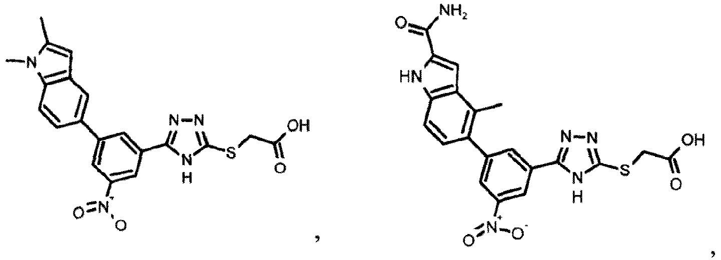 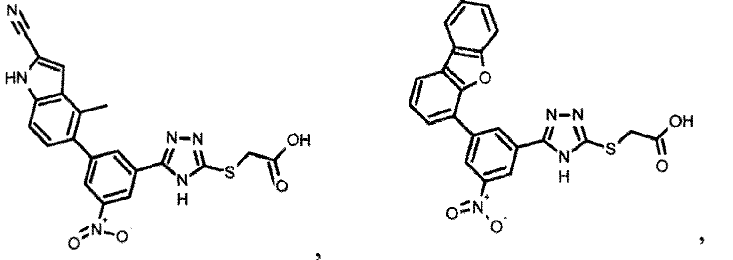 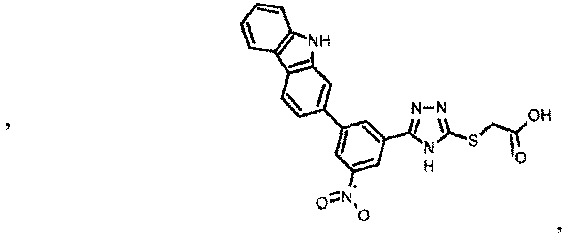 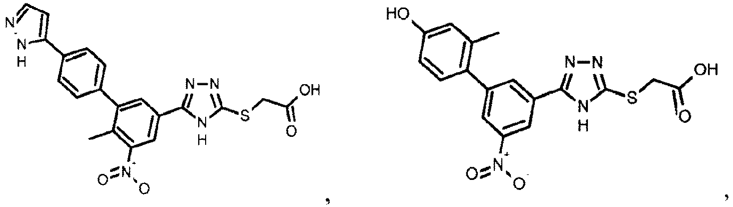 et 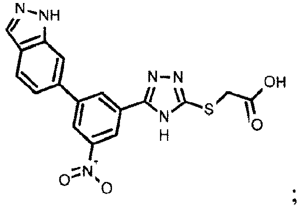 ou sel pharmaceutiquement acceptable de celui-ci.

5. Composition pharmaceutique comprenant un composé selon l'une quelconque des revendications 1 à 4, ou un sel pharmaceutiquement acceptable de celui-ci, et un support pharmaceutiquement acceptable.

6. Procédé *in vitro* d'inhibition d'un polypeptide de GRK6 (G protein coupled receptor kinase 6) dans une cellule, le procédé comprenant la mise en contact de la cellule avec une quantité efficace d'un composé selon l'une quelconque des revendications 1 à 4, ou d'un sel pharmaceutiquement acceptable de celui-ci,
dans lequel, optionnellement, la cellule est une cellule cancéreuse,
dans lequel, optionnellement, la cellule cancéreuse est une cellule cancéreuse de type cellule B.

7. Composé selon l'une quelconque des revendications 1 à 4, ou sel pharmaceutiquement acceptable de celui-ci, destiné à une utilisation dans le traitement d'une malignité hématologique ou d'une maladie inflammatoire chez un patient ou la suppression d'une réponse immunitaire chez un patient.

8. Composé destiné à l'utilisation selon la revendication 7,
(a) la malignité hématologique étant un cancer des cellules B,
le cancer des cellules B étant optionnellement sélectionné dans le groupe constitué d'un lymphome lymphocytaire à petites cellules (LLPC), d'un lymphome à cellules du manteau, d'un lymphome de Burkitt, d'un lymphome centro-folliculaire, d'un lymphome folliculaire, d'un lymphome Burkitt-like, d'un lymphome de la zone marginale (LZM) à cellules B, d'un lymphome B ganglionnaire de la zone marginale, d'un lymphome B extraganglionnaire de la zone marginale, d'un lymphome B splénique de la zone marginale, d'un lymphome lymphoplasmacytique, d'un lymphome diffus à grandes cellules B, d'une leucémie lymphoïde aiguë à cellules B (LLA-B), d'une leucémie lymphoïde aiguë à précurseurs B (LLA-B), d'une leucémie lymphoïde chronique à cellules B (LLC-B), d'une leucémie lymphoblastique à précurseurs B, d'un lymphome lymphoblastique à précurseurs B, d'une leucémie prolymphocytaire à cellules B, d'un lymphome indifférencié à cellules B, d'une leucémie à tricholeucocytes, d'un lymphome médiastinal à grandes cellules B, d'un myélome à plasmocytes, d'un plasmacytome, d'un lymphome primitif des séreuses, d'une leucémie à cellules de Burkitt, et d'un lymphome mixte diffus à cellules B ;
(b) la maladie inflammatoire étant sélectionnée dans le groupe constitué d'une encéphalite, d'une maladie oculaire inflammatoire, d'une otite, d'une pharyngite, d'une pneumonie, d'une gastrite, d'une entérite, d'une hépatite, d'une pancréatite, d'une néphrite, d'une cystite, d'une urétrite, d'une endométrite, d'une vaginite, d'une arthrite, d'une névrite périphérique, d'une tumeur maligne, de maladies infectieuses, de maladies auto-immunes, de maladies ischémiques, de maladies métaboliques, d'une blessure, d'une brûlure, d'une corrosion par une substance chimique, et de maladies neurodégénératives,
les maladies auto-immunes étant optionnellement sélectionnées dans le groupe constitué de rhumatismes, d'un lupus érythémateux disséminé, et d'une sarcoïdose, les maladies ischémiques étant optionnellement sélectionnées dans le groupe constitué d'un infarctus du myocarde et d'un infarctus cérébral,
les maladies métaboliques étant optionnellement sélectionnées dans le groupe constitué du diabète et de la goutte, ou
la maladie neurodégénérative étant optionnellement la maladie d'Alzheimer.
